(19) Europäisches Patentamt
European Patent Office
Office européen des brevets

(11) EP 0 657 440 B1

(12) **EUROPÄISCHE PATENTSCHRIFT**

(45) Veröffentlichungstag und Bekanntmachung des Hinweises auf die Patenterteilung:
**05.04.2000 Patentblatt 2000/14**

(51) Int. Cl.[7]: **C07D 263/24**, A61K 31/42, C07D 413/04, C07D 413/10

(21) Anmeldenummer: **94119100.9**

(22) Anmeldetag: **03.12.1994**

(54) **Oxazolidin-2-onderivate als MAO-Inhibitoren**
Oxazolidine-2-one derivatives as MAO inhibitors
Dérivés d'oxazolidine-2-one comme inhibiteurs de MAO

(84) Benannte Vertragsstaaten:
**AT BE CH DE DK ES FR GB GR IE IT LI LU MC NL PT SE**

(30) Priorität: **13.12.1993 CH 370193**
**27.09.1994 CH 292794**

(43) Veröffentlichungstag der Anmeldung:
**14.06.1995 Patentblatt 1995/24**

(73) Patentinhaber:
**F. HOFFMANN-LA ROCHE AG**
**4002 Basel (CH)**

(72) Erfinder:
- **Borgulya, Janos**
  **CH-4058 Basel (DE)**
- **Bruderer, Hans**
  **CH-4105 Biel-Benken (DE)**
- **Jakob-Roetne, Roland**
  **D-79594 Inzlingen (DE)**
- **Röver, Stephan**
  **D-79639 Grenzach-Wyhlen (DE)**

(74) Vertreter: **Poppe, Regina et al**
**F.Hoffmann-La Roche AG**
**Patent Department(PLP),**
**124 Grenzacherstrasse**
**4070 Basel (CH)**

(56) Entgegenhaltungen:
**FR-A- 2 381 037**
**GB-A- 2 003 151**
**GB-A- 2 076 813**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.16.7 (HRS)/3.6

## Beschreibung

Die vorliegende Erfindung betrifft Oxazolidinon-Derivate. Im Speziellen betrifft sie Oxazolidin-2-on-Derivate der allgemeinen Formel

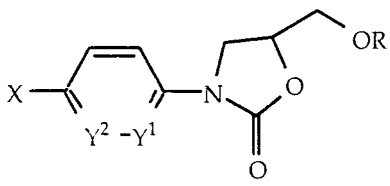

I

worin

- R niederes Alkyl;
- X Cycloalkenyl; Bicyclo[2.2.1]hept-2-yl, ggf. substituiert durch Phenyl-2-oxo-5-methoxymethyl-oxazolidinyl; Bicyclo[2.2.1]hept-5-en-2-yl; Adamantyl; oder Cycloalkyl oder Piperidin, gegebenenfalls einfach oder mehrfach substituiert durch Halogen, Amino, niederes Alkyl, Nitril, eine Oxo-Gruppe, Hydroxyimino, Ethylendioxy oder durch $-OR^1$, worin
- $R^1$ $-CH(C_6H_5)_2$, $-(CH_2)_nC_6H_5$, niederes Alkyl, Wasserstoff, $-(CH_2)_nNHCOCH_3$, $-(CH_2)_nNH_2$, $-(CH_2)_nSOCH_3$, $-(CH_2)_nCN$, $-(CH_2)_nSCH_3$, $-(CH_2)_nSO_2CH_3$, -CO-niederes-Alkyl, $-COC_6H_5$, ggf. substituiert durch eine Oxazolidinyl-Gruppe: oder durch $=CR^2R^3$, worin
- $R^2$ Wasserstoff oder niederes Alkyl;
- $R^3$ Wasserstoff, Nitril, niederes Alkyl, Phenyl oder COO-niederes-Alkyl; oder durch $-(CH_2)_nR^4$, worin
- $R^4$ Nitril, Amino, $-NHCOCH_3$, $-COC_6H_5Hal$, Phenyl oder Hydroxy; oder durch $-COR^5$, worin
- $R^5$ niederes Alkyl, $-CH=CH-C_6H_5$, $-C_6H_5CF_3$, $-O-C(CH_3)_3$ oder -O-niederes-Alkyl; oder durch $-NR^6R^7$, worin
- $R^6$ Wasserstoff oder $-COCH_3$;
- $R^7$ $-COCH_3$, Benzyl oder$-(CH_2)_nNHCOC_6H_4Hal$;
- n 1-3;
- $Y^1$ -CH=;
- $Y^2$ -CH=, -C(OH)=, $-C(NO_2)=$, $-C(NH_2)=$, **oder** -C(Hal)= bedeuten,

sowie pharmazeutisch annehmbare Salze von basischen Verbindungen der Formel I mit Säuren.

Diese Verbindungen und Salze sind neu und zeichnen sich durch wertvolle pharmakologische Eigenschaften aus, denn sie besitzen Monoaminooxidase (MAO) hemmende Eigenschaften. Auf Grund dieser Aktivität können die Verbindungen der Formel I und deren pharmazeutisch anwendbare Salze zur Behandlung von, Panik- und Angstzuständen, kognitiven Erkrankungen und neurodegenerativen Erkrankungen wie der Parkinsonschen Krankheit und der Alzheimerschen Krankheit verwendet werden.

**Oxazolidin-2-on Verbindungen sind in der Literatur beschrieben. So beschreibt beispielsweise die französische Patentschrift Nr. 2 381 037 5-Hydroxymethyl-oxazolidin-2-on Verbindungen mit psychotropen Eigenschaften. Weiterhin sind aus den englischen Patentschriften GB 2 076 813 und GB 2 003 151 N-Aryl-2-oxazolidinone zur Behandlung von exogenen und endogenen depressiven Zuständen bekannt.**

Gegenstand der vorliegenden Erfindung sind Verbindungen der allgemeinen Formel I und pharmazeutisch anwendbare Salze davon als solche und als pharmazeutische Wirkstoffe, Verfahren zur Herstellung dieser Verbindungen und Salze, ferner diese enthaltende Arzneimittel und die Herstellung solcher Arzneimittel, sowie die Verwendung von Verbindungen der allgemeinen Formel I und von pharmazeutisch anwendbaren Salzen davon bei der Bekämpfung bzw. Verhütung von Krankheiten bzw. bei der Verbesserung der Gesundheit und die Verwendung von Verbindungen der allgemeinen Formel I und von pharmazeutisch anwendbaren Salzen davon zur Herstellung von Arzneimitteln für die Bekämpfung bzw. Verhütung von, Panik- und Angstzuständen, kognitiven Erkrankungen und neurodegenerativen Erkrankungen wie der Parkinsonschen Krankheit und der Alzheimerschen Krankheit.

Der in der vorliegenden Beschreibung verwendete Ausdruck "niederes Alkyl" bezeichnet geradkettige oder verzweigte gesättigte Kohlenwasserstoffreste, wie Methyl, Ethyl, n-Propyl, Isopropyl, n-Butyl, Isobutyl, s-Butyl, t-Butyl und dergleichen.

Der Ausdruck "Cycloalkyl" bezeichnet gesättigte cyclische Kohlenwasserstoffe mit vorzugsweise 3 bis 7 Ringkohlenstoffatomen, wie z.B. Cyclopropan, Cyclobutan, Cyclopentan, Cyclohexan oder Cycloheptan.

Halogen bedeutet Fluor, Chlor, Brom oder Jod.

Der Ausdruck "Abgangsgruppe" bezeichnet im Rahmen der vorliegenden Erfindung bekannte Gruppen wie Halogen, vorzugsweise Brom, Arylsulfonyloxy, Alkylsulfonyloxy und dergleichen.

Reaktive Carbonyl-liefernde Mittel können im Sinne dieser Beschreibung beispielsweise Diethylcarbonat, Phosgen oder dergleichen bedeuten.

Der Ausdruck "pharmazeutisch anwendbare Salze" umfasst Salze mit anorganischen und organischen Säuren, wie Salzsäure, Bromwasserstoffsäure, Schwefelsäure, Phosphorsäure, Citronensäure, Ameisensäure, Maleinsäure, Essigsäure, Bernsteinsäure, Weinsäure, Methansulfonsäure, p-Toluolsulfonsäure und dergleichen.

Bevorzugte Verbindungen der Formel I sind solche, worin X Cyclohexyl oder substituiertes Cyclohexyl, R Methyl und $Y^1$ und $Y^2$ CH bedeuten. Bevorzugt sind solche Cyclohexyl-Substituenten wie die Oxo-Gruppe, Hydroxy, Hydroxyimino, Methoxy sowie $-O-(CH_2)_2-R^8$, worin $R^8$ -CN oder $-CH_2NH_2$ bedeutet. Das sind z.B. die folgenden Verbindungen:

(RS)-3-(4-Cyclohexyl-phenyl)-5-methoxymethyl-oxazolidin-2-on;
(R)-3-(4-Cyclohexyl-phenyl)-5-methoxymethyl-oxazolidin-2-on;
(RS)-3-[4-(4-Oxocyclohexyl)-phenyl]-5-methoxymethyl-oxazolidin-2-on;
(RS)-3-[4-(trans-4-Hydroxy-cyclohexyl)-phenyl]-5-methoxymethyl-oxazolidin-2-on;
(RS)-3-[4-(4-Hydroxy-imino-cyclohexyl)-phenyl]-5-methoxymethyl-oxazolidin-2-on;
(R)-3[4-trans-4-Hydroxy-cyclohexyl)-phenyl]-5-methoxymethyl-oxazolidin-2-on;
(RS)-3-[4-(trans-4-Methoxy-cyclohexyl)-phenyl]-5-methoxymethyl-oxazolidin-2-on;
(R)-3-[4-(4-Oxo-cyclohexyl)-phenyl]-5-methoxymethyl-oxazolidin-2-on;
(RS)-3-[trans-4-[4-(5-Methoxymethyl-2-oxo-oxazolidin-3-yl)-phenyl]-cyclohexyloxy]-propionitril;
Essigsäure (RS)-trans-4-[4-(5-Methoxymethyl-2-oxo-oxazolidin-3-yl)-phenyl]-cyclohexylester;
(RS)-3-[4-(cis- und trans-4-Hydroxymethyl-cyclohexyl)-phenyl]-5-methoxymethyl-oxazolidin-2-on; und
(RS)-3-(4-(cis- oder trans-4-Hydroxy-4-methyl-cyclohexyl)-phenyl]-5-methoxymethyl-oxazolidin-2-on.

Weiterhin sind auch solche Verbindungen bevorzugt, worin X Bicyclo[2.2.1]hept-2-yl oder Bicyclo[2.2.1]hept-5-en-2-yl bedeutet, beispielsweise die Verbindungen

3-[(1RS,2RS,4SR)-4-Bicyclo[2.2.1]hept-2-yl-phenyl]-5-methoxymethyl-oxazolidin-2-on und
3-[(1RS,2SR,4RS)-4-Bicyclo[2.2.1]-hept-5-en-2-yl-phenyl]-5-methoxymethyl-oxazolidin-2-on.

Ebenso bevorzugt sind solche Verbindungen, worin X Cyclohexyl oder Piperidin, substituiert durch $-CH_2CN$, $-COCH=CH-C_6H_5$, $-O(CH_2)NH_2$ oder $-OCH_2CN$, R Methyl und $Y^1$ und $Y^2$ eine CH-Gruppe bedeuten, beispielsweise die folgenden Verbindungen

(RS)-[trans-4-[4-(5-Methoxymethyl-2-oxo-oxazolidin-3-yl)-phenyl]-cyclohexyl]-acetonitril;
(R)-[trans-4-[4-(5-Methoxymethyl-2-oxo-oxazolidin-3-yl)-phenyl]-cyclohexyl]-acetonitril;
(RS)-4-[4-(5-Methoxymethyl-2-oxo-oxazolidin-3-yl)-phenyl]-1-(1-oxo-3-phenyl-2-(E)-propenyl)-piperidin;
(RS)-3-(trans-4-[4-(5-Methoxymethyl-2-oxo-oxazolidin-3-yl)-phenyl]-cyclohexyloxy]-ethanamin;
(R)-[trans-4-[4-(5-Methoxymethyl-2-oxo-oxazolidin-3-yl)-phenyl]-cyclohexyloxy]-acetonitril;
(RS)-trans-4-[4-(5-Methoxymethyl-2-oxo-oxazolidin-3-yl)-phenyl]-cyclohexyloxy]-acetonitril und
(R)-3-[4-[trans-4-(3-Amino-propoxy)-cyclohexyl]-phenyl]-5-methoxymethyl-oxazolidin-2-on.

Verbindungen der allgemeinen Formel I und pharmazeutisch annehmbare Salze davon können erfindungsgemäss hergestellt werden, indem man

a) eine Verbindung der Formel

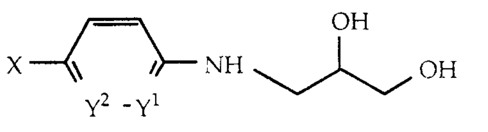

II

worin X, $Y^1$ und $Y^2$ die oben angegebenen Bedeutungen haben,
mit **Diethylcarbonat oder Phosgen** cyclisiert und alkyliert, oder
**b)** eine Verbindung der Formel

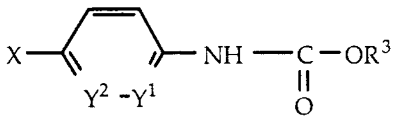

III

worin X, $Y^1$ und $Y^2$ die oben genannten Bedeutungen haben und $R^3$ niederes Alkyl oder Benzyl bedeutet, mit einer Verbindung der Formel

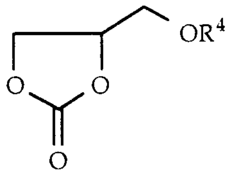

IV

umsetzt,

worin $R^4$ niederes Alkyl oder Benzyl bedeutet,

oder

**c)** eine Verbindung der allgemeinen Formel

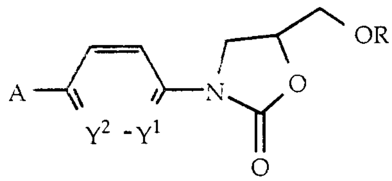

V

worin R, $Y^1$ und $Y^2$ die oben angegebenen Bedeutungen haben und A eine Abgangsgruppe bedeutet,

mit einem reaktiven, den Substituenten X liefernden Mittel behandelt, oder

**d)** eine Verbindung der Formel

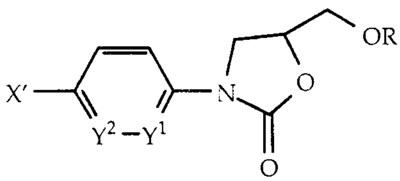

Ia

worin X' ein ($C_5$-$C_7$)-Cycloalkenyl-Rest bedeutet, der wie in Formel I für Cycloalkyl oder Piperidin beschrieben, substituiert sein kann,

und $Y^1$, $Y^2$ und R die obigen Bedeutungen haben, in die entsprechende gesättigte Cycloalkyl-Verbindung katalytisch hydriert, oder

**e)** eine Verbindung der Formel I, worin X ein durch Ethylendioxy substituiertes Cycloalkyl bedeutet, hydrolysiert, oder

**f)** eine Verbindung der Formel I, worin X ein durch $-O-(CH_2)_2-CN$, $=CH-CN$ oder $-CH_2CN$ substituierter Cycloalkyl- oder Piperidin-Rest bedeutet, zur Aminoverbindung hydriert, oder

**g)** eine Verbindung der Formel I, worin X ein durch eine Oxo-Gruppe substituierter Cycloalkyl- oder Piperidin-Rest bedeutet,

- diese in eine Hydroxyimino-Gruppe überführt, oder
- diese zu einer Hydroxy-Gruppe reduziert, oder
- mit entsprechend substituierten Aminen diese in eine - $NH(CH_2)_nNHCOC_6H_4R^3$-Gruppe überführt, worin $R^3$

und n die obigen Bedeutungen haben, oder

- diese in eine Methylen-Gruppe, eine Benzyliden-Gruppe, eine Dimethylmethylen-Gruppe, eine Methylen-Nitril-Gruppe oder in eine Methoxycarbonylmethylen-Gruppe überführt, oder
- diese in eine Ethylendioxy-Gruppe überführt, oder
- diese in eine Aminogruppe überführt, oder
- diese mit einem entsprechenden Phosphonat in eine =CH-CN-Gruppe überführt, oder

**h)** eine Verbindung der Formel I, worin X ein durch eine Hydroxy-Gruppe substituierter Cycloalkyl- oder Piperidin-Rest bedeutet,

- mit Acrylnitril in eine $-O(CH_2)_2CN$-Gruppe überführt, oder
- mit entsprechenden Alkyl-, Aryl- oder Acylhalogeniden alkyliert oder acyliert, oder
- mit geeigneten Halogenierungsmitteln halogeniert, oder
- zu den entsprechenden Sulfanyl-Verbindungen umsetzt und diese gegebenenfalls anschliessend zu den Sulfinyl- oder Sulfonyl-Verbindungen oxydiert, oder

**i)** eine Verbindung der Formel I, worin X ein durch eine Hydroxy-Gruppe substituierter Cycloalkylrest bedeutet,

- diesen zu einem Cycloalkenylrest dehydriert, oder

**j)** eine Verbindung der Formel I, worin X durch eine $-O(CH_2)_n-SOCH_3$-Gruppe substituierter Cycloalkyl- oder Piperidin-Rest bedeutet,

- diese in eine $-O(CH_2)_nCN$-Gruppe überführt, oder

**k)** eine Verbindung der Formel I, worin X ein Cycloalkyl-Rest, ein Piperidin- oder ein geschützter Piperidin-Rest bedeutet, der nicht über ein Stickstoff verknüpft ist,

- mit entsprechenden Acylierungsmitteln acyliert, oder

**l)** eine Verbindung der Formel I, worin X ein durch eine Methylen-Gruppe substituierter Cycloalkyl- oder Piperidin-Rest bedeutet,

- diese in eine Hydroxymethyl-Gruppe überführt, oder
- diese in eine 4-Hydroxy-4-methyl-Gruppe überführt, und

**m)** erwünschtenfalls eine basische Verbindung der Formel I mittels einer Säure in ein pharmazeutisch anwendbares Salz überführt.

Eine Verbindung der Formel II kann nach Variante a) des erfindungsgemässen Verfahrens zu einer Verbindung der Formel I cyclisiert werden, indem man mit einem reaktiven Carbonyl-liefernden Mittel behandelt. Zweckmässigerweise erfolgt die Cyclisierung nach an sich bekannten Methoden mit Diethylcarbonat, wobei man folgendermassen vorgeht: Die Verbindung der Formel II wird in einem Lösungsmittel, z.B. Toluol gelöst, mit Diethylcarbonat versetzt und nach Zugabe einer methanolischen Natriummethylatlösung unter Erwärmen mehrere Stunden gerührt. Die Reaktionsbedingungen, wie Temperatur, Dauer, Lösungsmittel usw. können je nach Natur des eingesetzten reaktiven Derivats variieren.

Nach der erfindungsgemässen Verfahrensvariante **b)** können Verbindungen der Formel I auch durch Umsetzung von Verbindungen der Formeln III mit IV hergestellt werden.

Zweckmässigerweise versetzt man einen Carbamidsäurealkylester der Formel III mit einem 4-(Alkoxymethyl)-1,3-dioxolan-2-on IV und rührt mehrere Stunden unter Erwärmen. Eine weitere Möglichkeit der Herstellung von Verbindungen der Formel I zeigt die Verfahrensvariate **c).** Hierbei werden Verbindungen der Formel V mit einem reaktiven, den Substituenten X liefernden Mittel behandelt. Bedeutet A in der Formel V als Abgangsgruppe ein Halogenatom, insbesondere Brom oder Jod, kann die Umsetzung zweckmässigerweise wie folgt vorgenommen werden: Ein Oxazolidinon der Formel V wird unter Zugabe von Triphenylphosphin, Bis(triphenylphosphin)palladium-II-dichlorid, Lithiumchlorid, 2,6-Di-tert-butyl-p-kresol und Tributyl-cycloalkenylstannan mehrere Stunden in DMF umgesetzt und anschliessend nach an sich bekannten Methoden aufgearbeitet.

Cycloalkyl-Derivate der Formel I können auch hergestellt werden, indem die entsprechenden Cycloalkenyl-Verbindungen der Formel VI katalytisch hydriert werden (Variante **d).** Geeignet sind dazu vor allem 5- bis 7-gliedrige

Ringsysteme, die zu den Cyclopentyl-, Cyclohexyl- oder Cycloheptyl-Verbindungen hydriert werden können. Die Hydrierung erfolgt nach an sich bekannten Methoden, zweckmässigerweise mit einem Palladium-Katalysator bei Raumtemperatur und Normaldruck.

Nach der Verfahrensvariante **e)** erhält man ein durch eine Oxo-Gruppe substituiertes Cyclohexyl-Derivat der Formel I, indem man Verbindungen der Formel I, worin X ein durch Ethylendioxy substituiertes Cyclohexyl bedeutet, nach an sich bekannten Methoden hydrolysiert. Zweckmässigerweise wird in Tetrahydrofuran gelöst, mit Salzsäure mehrere Stunden gerührt und mit Natronlauge versetzt.

Eine Verbindung der Formel I, worin X ein durch $-O-(CH_2)_2-CN$-substituiertes Cycloalkyl oder Piperidin bedeutet, kann nach an sich bekannten Methoden in eine Aminoverbindung hydriert werden (Variante **f**). Zweckmässigerweise versetzt man eine Suspension aus Natriumborhydrid, THF und Trifluoressigsäure bei Raumtemperatur mit der entsprechenden Verbindung der Formel I und rührt mehrere Stunden. Anschliessend erfolgt die Aufarbeitung nach üblichen Methoden.

Bedeutet X ein durch eine Acetonitril-Gruppe substituiertes Cycloalkyl, kann die Hydrierung zur Amino-Ethyl-Gruppe in methanolischem Ammoniak im Beisein von Raney-Nickel erfolgen. Eine weitere Variante, eine Amino-Verbindung der Formel I zu erhalten, besteht darin, ein durch eine =CH-CN-Gruppe substituiertes Cycloalkyl mit Pd-Kohle zu hydrieren und anschliessend wie oben beschrieben, zur Amino-Verbindung umzusetzen.

Bedeutet X in der Formel I ein durch eine Oxo-Gruppe substituierter Cycloalkyl- oder Piperidin-Rest, können diese Verbindungen als Ausgangsstoffe für die Herstellung weiterer Verbindungen der Formel I verwendet werden (Variante **g).**

Bedeutet X beispielsweise ein Cycloalkanon, kann die Oxo-Gruppe in eine Hydroxyimino-Gruppe überführt werden. Das geschieht zweckmässigerweise nach bekannten Methoden wie folgt: Zu einer entsprechenden Verbindung der Formel I gibt man unter Rühren und Kühlen eine wässrige Hydroxylaminlösung. Die entsprechende Hydroxyimino-Verbindung scheidet sich nach Zugabe einer wässrigen Natriumcarbonatlösung kristallin aus.

Weiterhin kann man OH-substituierte Derivate der Formel I erhalten, wenn man die Oxo-Gruppe reduziert. Das kann mittels eines komplexen Hydrids, wie beispielsweise Natrium- oder Lithiumborhydrid in einem unter den Reaktionsbedingungen inerten organischen Lösungsmittel, wie Methanol, Ethanol oder dergleichen, erfolgen. Die Reduktion erfolgt zweckmässigerweise bei Raumtemperatur.

Die durch eine Oxo-Gruppe substituierten Cycloalkyl- oder Piperidin-Derivate der Formel I können auch mit Aminen, beispielsweise mit N-(2-Aminoethyl)-4-chlorbenzamid, umgesetzt werden. Zweckmässigerweise werden beide Reaktionspartner mit p-Toluolsulfonsäuremonohydrat versetzt und in einem Gemisch aus Toluol/DMF gelöst. Anschliessend wird mehrere Stunden unter Erwärmen gerührt und nach Abkühlung mit Natriumborhydrid und Salzsäure versetzt.

Weiterhin können entsprechend Variante **g)** Verbindungen der Formel I, worin X ein durch eine Oxo-Gruppe substituierter Cycloalkyl- oder Piperidin-Rest bedeutet, in Methylen-, Benzyliden-, Dimethylmethylen-, Methylen-Nitril- oder Methoxycarbonylmethylen-substituierte Cycloalkyl- oder Piperidin-Derivate überführt werden.

Zweckmässigerweise geht man dabei folgendermassen vor: Unter Argon werden beispielsweise Methyltriphenylphosphoniumbromid/Natriumamid-Gemische oder Isopropyltriphenylphosphoniumbromid/Natriumamid-Gemische oder Benzyltriphenylphosphoniumbromid/Natriumamid-Gemische oder Methoxycarbonylmethylentriphenylphosphoniumbromid/Natriumamid-Gemische in THF suspendiert und gerührt. Dazu gibt man die entsprechende oxo-Cycloalkyl- oder oxo-Piperidin-Verbindung der Formel I und rührt mehrere Stunden unter Erwärmen. Die Aufarbeitung erfolgt nach üblichen Methoden. Die Methylengruppe kann gegebenenfalls anschliessend wieder hydriert werden.

Ebenfalls nach Verfahrensvariante **g)** können Verbindungen der Formel I hergestellt werden, worin X eine durch eine Ethylendioxy-Gruppe substituierter Cycloalkyl- oder Piperidin-Rest bedeutet. Zweckmässigerweise setzt man eine entsprechend substituierte oxo-Cycloalkyl- oder oxo-Piperidin-Verbindung der Formel I mit Ethylenglykol im Beisein von Toluolsulfonsäuremonohydrat in Toluol um. Die Zielverbindungen entstehen nach mehreren Stunden Rühren unter Erwärmen und üblicher Aufarbeitung.

Ein durch eine Oxo-Gruppe substituierter Cycloalkyl- oder Piperidin-Rest kann entsprechend Variante **g)** in eine Aminogruppe überführt werden, indem die entsprechende Oxo-Verbindung zweckmässigerweise mit Benzylamin und Toluolsulfonsäure umgesetzt und anschliessend mehrere Stunden mit-Natriumborhydrid bei Raumtemperatur hydriert wird.

Eine Oxo-Cyclohexyl-Gruppe kann nach Variante **g)** auch in eine Cyclohexyliden-Acetonitril-Gruppe überführt werden, indem man zweckmässigerweise Natrium in Ethanol löst und diese Lösung mit einer ethanolischen Diethylcyanomethylphosphonat-Lösung versetzt. Anschliessend erfolgt die Umsetzung mit einer entsprechenden Oxo-Cyclohexyl-Verbindung der Formel I zur Zielverbindung.

Bedeutet X in der Formel I eine durch eine Hydroxy-Gruppe substituierter Cycloalkyl- oder Piperidin-Rest, können auch diese Verbindungen als Ausgangsstoffe für die Herstellung weiterer Verbindungen der Formel I verwendet werden (Variante **h).** Nitril-substituierte Verbindungen können beispielsweise durch die Umsetzung von Hydroxy-Cycloalkyl- oder Piperidin-Derivaten der Formel I mit Acrylnitril in Anwesenheit von Kalium-tert-butylat hergestellt werden.

Die auf diese Weise entstandenen Nitril-substituierten Verbindungen der Formel I können gegebenenfalls in entsprechende Aminoverbindungen umgesetzt werden, indem man sie mit trockenem Ammoniak-Gas, gelöst in Methanol behandelt und in Gegenwart von Raney-Nickel hydriert.

Verbindungen der Formel I, die eine Hydroxy-Gruppe enthalten, können mit Säurechloriden in die entsprechenden Carbonyloxy-Verbindungen acyliert werden. Zweckmässigerweise löst man die durch eine Hydroxy-Gruppe substituierte Cycloalkyl- oder Piperidin-Verbindung der Formel I in einem Lösungsmittel, z.B. Methylenchlorid, versetzt diese Lösung mit Pyridin und einem Säurechlorid, beispielsweise Acetylchlorid oder Benzoylchlorid und rührt mehrere Stunden bei Raumtemperatur. Die Aufarbeitung erfolgt nach üblichen Methoden.

Eine Alkylierung bzw. Acylierung von Verbindungen der Formel I, die eine Hydroxy-Gruppe enthalten, kann mit entsprechenden Alkyl- oder Acylhalogeniden durchgeführt werden, beispielsweise mit Methyljodid, Benzylbromid oder Chlordiphenylmethan. Diese Umsetzungen erfolgen nach an sich bekannten Methoden.

Eine Halogenierung von Verbindungen der Formel I, die eine Hydroxy-Gruppe enthalten, kann ebenfalls nach bekannten Methoden mit einem geeigneten Halogenierungsmittel erfolgen. Beispielsweise entsteht die Fluor-Cycloalkylverbindung der Formel I durch Umsetzung der Hydroxy-Cycloalkyl-Verbindung mit einem Gemisch aus Diethylamino-Schwefeltrifluorid und Methylenchlorid nach Rühren bei Raumtemperatur.

Ebenfalls nach Verfahrensvariante **h**) können Verbindungen, die eine Hydroxy-Gruppe enthalten, zu entsprechenden Sulfanyl-Verbindungen umgesetzt werden. Das kann zweckmässigerweise so erfolgen, dass man die Hydroxy-Verbindung in Dimethylsulfoxid löst und dann mit Eisessig und Essigsäureanhydrid versetzt und mehrere Stunden bei Raumtemperatur rührt. Weitere mögliche Verfahrensvarianten sind beschrieben in "Chemistry letters, pp. 1277-1278", veröffentlicht von der japanischen chemischen Gesellschaft.

Die entsprechende Sulfinylverbindung kann gegebenenfalls anschliessend an die oben beschriebene Variante durch Oxydation der Sulfanyl-Verbindung entstehen. Als Oxydationsmittel ist beispielsweise Natriumperjodat gut geeignet.

Die entsprechende Sulfonylverbindung kann durch Oxydation der Sulfanylverbindung entstehen, zweckmässigerweise verwendet man als Oxydationsmittel Chlorperbenzoesäure. Ein geeignetes Lösungsmittel ist beispielsweise Methylenchlorid.

Nach Verfahrensvariante **i)** werden Verbindungen der Formel I, worin X ein durch eine Hydroxy-Gruppe substituierter Cycloalkylrest bedeutet, zu einem Cycloalkenylrest dehydriert. Das erfolgt zweckmässigerweise durch Umsetzung der Hydroxy-Verbindung mit Triphenylphosphin und Benzoesäure in Tetrahydrofüran bei Raumtemperatur und anschliessendem Versetzen mit Azodicarbonsäurediethylester.

Nach Verfahrensvariante **j),** worin X durch eine-$O(CH_2)_n$-$SO$-$CH_3$-Gruppe substituierter Cycloalkyl- oder Piperidin-Rest bedeutet, wird diese Gruppe in eine Gruppe -$O(CH_2)_nCN$ überführt, indem die entsprechende Sulfinylverbindung in einem Lösungsmittel, beispielsweise Tetrahydrofüran gelöst und in Gegenwart von Zinkjodid und Trimethylsilylcyanid umgesetzt wird. Diese Umsetzung verläuft über mehrere Stunden bei Raumtemperatur.

Eine Verbindung der Formel I, worin X ein Piperidin- oder geschützter Piperidin-Rest, der nicht über ein Stickstoffatom verknüpft ist, oder ein Cycloalkylrest bedeutet, kann nach Verfahrensvariante **k)** acyliert werden. Das erfolgt zweckmässigerweise mit geeigneten Acylierungsmitteln, beispielsweise mit Benzylbromid, w-Chlor-4-fluorbutyrophenon, Trifluormethylbenzoylchlorid, Essigsäureanhydrid oder Zimtsäurechlorid nach an sich bekannten Methoden. Als Lösungsmittel eignen sich besonders Dimethylformamid oder Methylenchlorid.

Verbindungen der Formel I, die eine Methylen-Gruppe als Cycloalkyl- oder Piperidin-Substituenten enthalten, können ebenso als Ausgangsstoffe für die Herstellung weiterer Verbindungen der Formel I verwendet werden (Variante **l)**.

Eine entsprechende Verbindung der Formel I, worin X ein durch eine Methylengruppe substituierter Cycloalkyl- oder Piperidin-Rest bedeutet, kann in eine Hydroxymethyl-substituierte Verbindung überführt werden, indem man diese Verbindung, gelöst in einem Lösungsmittel, beispielsweise THF, mit Natriumborhydrid in Anwesenheit von Dimethylsulfat hydroboriert und das Zwischenprodukt mit $H_2O_2$ oxidiert.

Die 4-Methylen-substituierten Cycloalkyl-Verbindungen der Formel I können auch in entsprechende 4-Hydroxy-4-methyl-substituierte Cycloalkyl- oder Piperidin-Derivate umgesetzt werden. Dazu wird Quecksilberacetat in THF gelöst und mit der 4-Methylen-substituierten Cycloalkyl-Verbindung der Formel I versetzt. Es wird bei Raumtemperatur gerührt, mit Natronlauge versetzt und mit Natriumborhydrid umgesetzt. Die Aufarbeitung erfolgt auf übliche Weise.

Eine Verbindung der Formel I, worin R Benzyl bedeutet, kann durch Umsetzung eines entsprechend substituierten Cycloalkyl- oder Piperidinphenyl-ethoxycarbaminesters mit Benzyloxymethyl-1,3-dioxolan-2-on nach an sich bekannten Methoden hergestellt werden.

Die pharmazeutisch verwendbaren Salze können im Hinblick auf den Stand der Technik und unter Berücksichtigung der Natur der in ein Salz zu überführenden Verbindung nach an sich bekannten Methoden ohne weiteres hergestellt werden.

Die in Verfahrensvariante a) als Ausgangsstoffe verwendeten Verbindungen der Formel II können folgendermassen hergestellt werden: 4-Cyclohexylanilin oder andere entsprechend substituierte Aniline und Methansulfonsäure (RS)-2,2-dimethyl-1,3-dioxolan-4-ylmethylester (beschrieben in J. Med. Chem 27, 1176 [1934]) werden in Triethylamin einige Stunden bei ca. 140°C in einem Bombenrohr gehalten. Anschliessend, nach Entfernen des Lösungsmittels, wird der Rückstand mit Salzsäure gerührt, alkalisch gestellt und nach üblichen Methoden aufgearbeitet.

Eine weitere Möglichkeit besteht in der Umsetzung von 4-Cyclohexylanilin oder anderen entsprechend substituierten Anilinen mit [(R)-2,2-Dimethyl-1,3-dioxolan-4-methanol-toluol-4-sulfönat] in Triethylamin bei Temperaturen um 140°C Es wird einige Stunden gerührt und das aufgearbeitete Produkt wird anschliessend mit HCl versetzt und nach ca. 1 Stunde Rühren mit Natronlauge alkalisch gestellt. Anschliessend wird nach bekannten und üblichen Methoden aufgearbeitet.

Die in Verfahrensvariante **b)** als Ausgangsstoffe benötigte Verbindung III ist bekannt und kann folgendermassen hergestellt werden.

4-Cycloalkylanilin oder andere entsprechend substituierte Aniline werden in einem Lösungsmittel, z.B. THF und Wasser, gelöst, mit Natriumbicarbonat versetzt und anschliessend mit Chlorameisensäureäthylester umgesetzt. Die Reaktionstemperatur sollte 20°C nicht übersteigen.

Die Verbindungen der Formel IV sind bekannt und können nach in der Literatur bekannten Verfahren hergestellt werden.

Die für die Verfahrensvariante **c)** benötigte Verbindung V kann zweckmässigerweise folgendermassen hergestellt werden, wobei im folgenden Beispiel $Y^2$ Stickstoff bedeutet:

Ein Gemisch aus Pyridin-3-yl-carbamidsäureethylester, Methoxymethyl-1,3-dioxolan-2-on und Kaliumcarbonat werden erhitzt und einige Stunden gerührt. Die entstandene Verbindung wird mit einem Oxydationsmittel, vorzugsweise 3-Chlorperbenzoesäure, behandelt, wobei die entstandene Verbindung 5-Methoxymethyl-3-(1-oxy-pyridin-3-yl)oxazolidin-2-on bromiert und anschliessend mit einem Reduktionsmittel, beispielsweise Phosphortribromid, in eine Verbindung der Formel V überführt wird. Diese Verbindung kann dann, wie beschrieben, mit einem reaktiven, den Substituenten X liefernden Mittel umgesetzt werden. Als geeignet erwiesen sich solche Mittel, die als Rest beispielsweise eine Tributyl-stannyl-Gruppe enthalten. Die Herstellung dieser Verbindungen erfolgt nach an sich bekannten Methoden.

Substituierte Cycloalkenyl-Verbindungen der Formel VI können hergestellt werden, indem man Verbindungen der Formel V, worin A eine Abgangsgruppe, vorzugsweise Halogen bedeutet, mit reaktiven, den Substituenten X' liefernden Mittel behandelt, worin X' ein ($C_5$-$C_7$)-Cycloalkenyl-Rest bedeutet, der wie in Formel I für Cycloalkyl beschrieben, substituiert sein kann. Die reaktive Gruppe kann beispielsweise die Trimethylstannyl-Gruppe sein. Zweckmässigerweise erfolgt die Umsetzung unter Argon in Anwesenheit von Bis(triphenylphosphin)palladium(II)dichlorid in THF nach an sich bekannten Methoden.

Die in den Varianten **d)**, **e)**, **f)**, **g)**, **h)**, **i)**, **j)**, **k)**, **l)** und **m)** als Ausgangsstoffe verwendeten Verbindungen fallen unter die Formel I und können demnach nach den für die Herstellung dieser Verbindungen beschriebenen Methoden a), **b)** und **c)** erhalten werden.

Die Verbindungen der Formel I und deren pharmazeutisch anwendbare Salze haben - wie bereits oben erwähnt - Monoaminooxydase (MAO) hemmende Aktivität. Auf Grund dieser Aktivität können die Verbindungen der Formel I und deren pharmazeutisch anwendbare Salze zur Behandlung von Panik- und Angstzuständen, kognitiven Erkrankungen und neurodegenerativen Erkrankungen verwendet werden. Beispiele solcher Erkrankungen sind Parkinsonsche altersbedingte Gedächtnisschwächen, primäre und sekundäre degenerative Demenz, z.B. Demenz vom Alzheimer-Typ oder multi-infarktbedingte Demenz und cerebrovaskuläre Erkrankungen und Folgen von Hirnschäden.

Die MAO hemmende Aktivität der erfindungsgemässen Verbindungen kann unter Verwendung von Standardmethoden bestimmt werden.

So wurden die zu prüfenden Präparate dem nachfolgend beschriebenen in vitro-Test unterzogen, reicher sich an die von R.J. Wurtmann und J.A. Axelrod publizierte [Biochem. Pharmacol. 12, 1439-1441 (1963)] Methode anlehnt.

Isolierte Rattengehirne werden im Verhältnis 1:9 (Gewicht-Volumen) in 0,1 molarem Kaliumphosphatpuffer (pH 7,4) homogenisiert, worauf die Homogenate im Verhältnis 1:4 (Volumen/Volumen) mit demselben Puffer verdünnt und bei -20°C aufbewahrt werden. Zur Inkubation verwendet man eine Mischung folgender Zusammensetzung:

- 100 µl 1M Phosphatpuffer (pH 7,4);
- 100 µl Solubilisat der zu prüfenden Substanz in Wasser oder wässrigem Dimethylsulfoxid;
- 50 µl Rattenhirnhomogenat; und als Substrat
- 50 µl $^{14}C$-Serotonin (5-HT) bzw. $^{14}C$-Phenyläthylamin (PEA), jeweils 100 000 Zerfälle pro Minute, entsprechend einer Endkonzentration von $2 \cdot 10^{-4}$ Mol/l bzw. $2 \cdot 10^{-5}$ Mol/l.

Vor Zugabe des Substrats erfolgt während 30 Minuten eine Vorinkubation bei 37°C. Die Inkubation (in Gegenwart des Substrats) erfolgt ebenfalls bei 37°C und dauert 10 Minuten. Die Reaktion wird durch Zugabe von 200 µl 2N Salzsäure gestoppt. Zur Extraktion der deaminierten Produkte wird, je nach Verwendung von 5-HT bzw. von PEA

als Substrat, während 10 Minuten mit 5 ml Diäthyläther bzw. mit 5 ml n-Heptan geschüttelt, worauf man zentrifügiert, die Wasserphase im Trockeneisbad ausfriert und die organische Phase in Zählgläser dekantiert.

Man bestimmt aufgrund der b-Zählerwerte die Aktivität der MAO im Vergleich zu Kontrollhomogenaten.

Unter unseren Versuchsbedingungen verläuft die Aktivität linear zur Zeit und zur Konzentration des Homogenisats. Die $IC_{50}$-Werte wurden graphisch bestimmt als log der Konzentrations-Aktivitäts-Kurve. Als $IC_{50}$ wurde diejenige Konzentration einer zu prüfenden Substanz definiert, welche die Aktivität der MAO beim Substrat 5-HT bzw. PEA auf 50% herabsetzt.

Die so ermittelte Aktivität einiger erfindungsgemässer Verbindungen wird aus den in der folgenden Tabelle 1 aufgeführten $IC$-$_{50}$-Werten ersichtlich.

Tabelle 1

| **Beispiel Nr.** | **MAO-Hemmung in vitro (Hirn) $IC_{50}$ in nMol/l** |
|---|---|
| Befloxaton | 1,9 |
| **1** | 2,0 |
| **3** | 0,1 |
| **4** | 10,0 |
| **5** | 8,4 |
| **6** | 6,0 |
| **9** | 1,0 |
| **10** | 8,5 |
| **11** | 6,0 |
| **13** | 9,0 |
| **19** | 5,0 |
| **22** | 10,0 |
| **27** | 3,0 |
| **28** | 4,0 |
| **38** | 0,9 |
| **39** | 1,0 |
| **48** | 10,0 |
| **52** | 1,6 |
| **55** | 2,0 |

Die Verbindungen der Formel I sowie deren pharmazeutisch verwendbare Säureadditionssalze können als Heilmittel, z.B. in Form pharmazeutischer Präparate, Verwendung finden. Die pharmazeutischen Präparate können oral, z.B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z.B. in Form von Suppositorien, oder parenteral, z.B. in Form von Injektionslösungen, erfolgen.

Zur Herstellung von Tabletten, Lacktabletten, Dragees und Hartgelatinekapseln können die Verbindungen der Formel I und deren pharmazeutisch verwendbaren Säureadditionssalze mit pharmazeutisch inerten, anorganischen oder organischen Excipientien verarbeitet werden. Als solche Excipientien kann man z.B. für Tabletten, Dragées und Hartgelatinekapseln, Lactose, Maisstärke oder Derivate davon, Talk, Stearinsäure oder deren Salze etc. verwenden.

Für Weichgelatinekapseln eignen sich als Excipientien z.B. vegetabile Oele, Wachse, Fette, halbfeste und flüssige Polyole etc.

Zur Herstellung von Lösungen und Sirupen eignen sich als Excipientien z.B. Wasser, Polyole, Saccharose, Invertzucker, Glukose etc.

Für Injektionslösungen eignen sich als Excipientien z.B. Wasser, Alkohole, Polyole, Glycerin, vegetabile

Oele etc.

Für Suppositorien eignen sich als Excipientien z.B. natürliche oder gehärtete Oele, Wachse, Fette, halbflüssige oder flüssige Polyole etc.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Ueberzugsmittel oder Antioxidantien enthalten. Sie können auch noch andere therapeutisch wertvolle Stoffe enthalten.

Erfindungsgemäss kann man Verbindungen der allgemeinen Formel I sowie deren pharmazeutisch verwendbare Säureadditionssalze bei der Bekämpfung bzw. Verhütung von kognitiven Erkrankungen und neurodegenerativen Erkrankungen wie der Parkinsonschen Krankheit und der Alzheimerschen Krankheit verwenden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10 bis 100 mg einer Verbindung der allgemeinen Formel I angemessen sein, wobei aber die soeben angegebene obere Grenze auch überschritten werden kann, wenn sich dies als angezeigt erweisen sollte.

Die folgenden Beispiele sollen die vorliegende Erfindung erläutern, sie jedoch in keiner Weise einschränken. Alle Temperaturen sind in Celsius Graden angegeben.

Beispiel **1**

(RS)-3-(4-Cyclohexyl-phenyl)-5-methoxymethyl-oxazolidin-2-on

1.5 g (5.45 mmol) (RS)-3-(4-Cyclohexyl-phenyl)-5-hydroxymethyl-oxazolidin-2-on wurden mit 15 ml Toluol, 1.6 ml Dimethylsulfat (16.3 mmol), 185 mg Tetrabutylammoniumhydrogensulfat und einer Lösung von 1.09 g (27.2 mmol) Natriumhydroxyd in 1.3 ml Wasser versetzt und während 30 Minuten intensiv gerührt. Das Reaktionsgemisch wurde auf Eiswasser gegossen und mit Essigester extrahiert, die organische Phase wurde mit Wasser gewaschen, über Magnesiumsulfat getrocknet und das Lösungsmittel abdestilliert. Erhalten wurden 1.6 g eines gelben Oels. Nach Versetzen mit Essigester/Hexan resultierten 0.7 g farblose Kristalle. Smp.: 69-70°.

Beispiel **2**

(S)-3-(4-Cyclohexyl-phenyl)-5-methoxymethyl-oxazolidin-2-on

Zu einer Lösung von 1.0 g (3.63 mmol) (S)-3-(4-Cyclohexyl-phenyl)-5-hydroxymethyl-oxazolidin-2-on in 10 ml Toluol wurden 250 mg Tetrabutylammoniumdihydrogensulfat, eine Lösung von 0.73 g Natriumhydroxid (18.2 mmol) in 1 ml Wasser und 1.1 ml Dimethylsulfat (10.9 mmol) zugegeben und während 1 Stunde bei 110° gerührt. Nach dem Abkühlen wurde mit 10 ml Wasser versetzt und mit Essigester extrahiert. Nach Einengen des Lösungsmittels resultierten 1.2 g eines kristallinen Gemisches, das an der 30-fachen Menge Kieselgel chromatographiert wurde. Die DC-einheitlichen Essigester-Hexan (1:1)-Fraktionen wurden zusammengefasst und das Lösungsmittel abdestilliert. Es resultierten 0.85 g (S)-3-(4-Cyclohexyl-phenyl)-5-methoxymethyl-oxazolidin-2-on in Form farbloser Kristalle. Smp.: 90-92°. $[a]_D$=+39.7° (c=0.7/$CHCl_3$).

Beispiel **3**

(R)-3-(4-Cyclohexyl-phenyl)-5-methoxymethyl-oxazolidin-2-on

2.0 g (7.26 mmol) des (R)-3-(4-Cyclohexyl-phenyl)-5-hydroxymethyl-oxazolidin-2-on wurden wie in Beispiel 4 angegeben umgesetzt und aufgearbeitet. Es entstanden 1.91 g (91%) (R)-3-(4-Cyclohexyl-phenyl)-5-methoxymethyl oxazolidin-2-on in Form farbloser Kristalle. Smp.: 86-88°. $[a]_D$ =-37.7° (c=0.3/$CHCl_3$)

Beispiel **4**

(RS)-3-[4-(4-oxo-Cyclohexyl)-phenyl]-5-methoxymethyl-oxazolidin-2-on

10.0 g (38.3 mmol) [4-(4-oxo-Cyclohexyl)-phenyl]-ethoxycarbaminester, 10.0 g (75 mmol) (RS)-4-(Methoxymethyl)-1,3-dioxolan-2-on und 0.5 g Kaliumcarbonat wurden während 4 Stunden bei einer Oelbadtemperatur von 160° intensiv gerührt. Nach dem Abkühlen wurde mit 50 ml Wasser versetzt und mit Essigester extrahiert. Das nach Abdestillieren des Lösungsmittels angefallene gelbe Oel (15,5 g) wurde an der 30-fachen Menge Kieselgel chromatographiert. Die im DC (Essigester/Hexan 7:3) einheitlichen Methylenchlorid/Essigester (15:1)-Fraktionen wurden

zusammengefasst und das Lösungsmittel abdestilliert. Es resultierten 5.54 g (48%) eines farblosen Produkts. Smp.: 114-116°.

Beispiel **5**

(RS)-3-[4(trans-4-Hydroxy-cyclohexyl)-phenyl]-5-methoxymethyl-oxazolidin-2-on

5.0 g (16.44 mmol) (RS)-5-Methoxymethyl-3-[4-(4-oxo-cyclohexyl)-phenyl]-oxazolidin-2-on wurden in 150 ml Ethanol in der Wärme gelöst und nach dem Abkühlen unter Rühren mit 620 mg (16,4 mmol) Natriumborhydrid versetzt, über Nacht gekühlt und das Lösungsmittel anschliessend abdestiliert. Der erhaltene ölige Rückstand wurde mit Wasser und 1 N Salzsäure versetzt und das Reaktionsprodukt in Essigester aufgenommen. Die organische Phase wurde mit Wasser gewaschen, über Magnesiumsulfat getrocknet und das Lösungsmittel abdestilliert. Das erhaltene farblose Oel (5.3 g) wurde in Essigester gelöst, mit Hexan bis zur Trübung versetzt und der ausgefallenen Niederschlag nach 1 Stunde abgesaugt. Es resultierten 3.3 g eines im NMR-Spektrum einheitlichen Alkohols. Smp. 109-110°.

Beispiel **6**

(RS)-3-[4-(4-Hydroxy-imino-cyclohexyl)-phenyl]-5-methoxymethyl-oxazolidin-2-on

Das aus dem Keton auf übliche Art bereitete Oxim wurde aus Essigester umkristallisiert. Smp. 146-147°.

Beispiel **7** (ZWISCHENPRODUKT)

(RS)-5-Benzyloxymethyl-3-[4-(4-oxo-Cyclohexyl)-phenyl]-oxazolidin-2-on

20 g (76.5 mmol) [4-(4-oxo-Cyclohexyl)-phenyl]-ethoxycarbaminester, 23.8 g (RS)-4-(Benzyloxymethyl)-1,3-dioxolan-2-on und 0.25 g Kaliumcarbonat wurden während 3 Stunden intensiv gerührt und wie in Beispiel 7 angegeben aufgearbeitet und chromatographiert. Der erhaltene Benzylether wurde aus t-Butylmethylether/Hexan umgelöst. Smp.: 126,5-128°.

Beispiel **8**

(RS)-3-(4-Cyclopropyl-phenyl)-5-methoxymethyl-oxazolidin-2-on

3.0 g (10.48 mmol) (RS)-5-Methoxymethyl-3(4-brom-phenyl)-oxazolidin-2-on, 6.29 g (6.29 mmol) Triphenylphosphin, 0.88 g (1.25 mmol) Bis(triphenylphosphin)palladium-II-dichlorid, 3.73 g Lithiumchlorid, 1 Spatelspitze 2,6-di-tert-butyl-p-kresol und 6.94 g Tributyl-cyclopropylstannan in 50 ml DMF wurden während 6 Stunden bei 120° gerührt. Das Reaktionsgemisch wurde mit Wasser und 1 N Natronlauge versetzt und mit Ether extrahiert. Das erhaltene Rohprodukt wurde an der 30-fachen Menge Kieselgel chromatographiert. Die im DC (KG, Essigester-Hexan 1:1) einheitlichen Extrakte wurden zusammengefasst und das Lösungsmittel abdestilliert. Aus tert-Butylmethylether/Hexan resultierten 0.3 g farblose Kristalle. Smp. 58-60°.

Beispiel **9**

(R)-3-[4-(4-Oxo-cyclohexyl)-phenyl]-5-methoxymethyl-oxazolidin-2-on

5.0 g (19.13 mmol) [4-(4-Oxo-cyclohexyl)-phenyl]-ethoxycarbaminester werden mit 3.0 g (S)-4-Methoxymethyl-1,3-dioxolan-2-on wie im Beispiel 7 angegeben umgesetzt und aufgearbeitet. Aus tert-Butylmethylether/Hexan resultierten 1.25 g Produkt in Form gelblicher Kristalle. Smp. 92-93°, $[a]_D$ =-38,1 (c=1, $CHCl_3$).

Beispiel **10**

(R)-3-[4(trans-4-Hydroxy-cyclohexyl)-phenyl]-5-methoxymethyl-oxazolidin-2-on

1.0 g Keton werden wie in Beispiel 8 angegeben mit Natriumborhydrid in Ethanol reduziert und aufgearbeitet. Aus Essigester/Hexan wurden 0.7 g farblose Kristalle isoliert. Smp. 133.5-134.5°. $[a]_D$ =-38,6° (c=0,7, $CHCl_3$).

Beispiel **11**

(RS)-3-[4-(trans-4-Methoxy-cyclohexyl)-phenyl]-5-methoxymethyl-oxazolidin-2-on

0.5 g (1.64 mmol) (RS)-3-[4-trans-4-Hydroxy-cyclohexyl)-phenyl]-5-methoxymethyl-oxazolidin-2-on wurden in 5 ml Dimethylformamid gelöst, mit 0.51 ml (8.2 mmol) Methyljodid und 107 mg (2.46 mmol) Natriumhydriddispersion (55%) versetzt und über Nacht bei 40° gerührt. Das Reaktionsgemisch wurde auf Eiswasser gegossen und mit Ether extrahiert. Die organischen Phasen wurden mit Wasser gewaschen, über Magnesiumsulfat getrocknet und das Lösungsmittel abdestilliert. Das erhaltene Gel wurde an der 30-fachen Menge Kieselgel mit Methylenchlorid/Essigester (1:4) chromatographiert. Es kristallisierte beim Versetzen mit tert-Butylmethylether/Hexan. Erhalten wurden 0.33 g Produkt. Smp. 82-83°.

Beispiel **12**

3-[(1RS,2RS,4SR)-4-Bicyclo[2.2.1]-hept-2-yl-phenyl]-5-methoxymethyl-oxazolidin-2-on (R:S=1:1)

Eine Lösung von 56 mg Bis-(triphenylphosphin)-palladium(II)-diacetat, 0.5 g (RS)-3-(4-Jodphenyl)-5-methoxymethyl-oxazolidin-2-on, 0.16 g Bicyclo-[2.2.1]hept-2-en, 0.5 ml Piperidin, 1 ml Dimethylformamid und 0.15 ml Ameisensäure wurde während 3 Stunden unter Argonatmosphäre gerührt. Das Reaktionsgemisch wurde mit 50 ml Essigester verdünnt und der ausgefallene Niederschlag abgetrennt. Das Filtrat wurde 2 mal mit je 20 ml Wasser extrahiert, die organische Phase über Magnesiumsulfat getrocknet und das Lösungsmittel abdestilliert. Das erhaltene braune Oel wurde an der 30-fachen Menge Kieselgel mit Essigester/Hexan (7:3) chromatographiert, wobei 0.43 g einheitliches Produkt nach Kristallisieren in Essigester/Hexan in der Kälte entstanden. Das racemische (1:1)-Diastereomerengemisch schmolz bei 83-84°.

Beispiel **13**

3-[(1RS,2SR,4RS)-4-Bicyclo[2.2.1]hept-5-en-2-yl-phenyl]-5-methoxymethyl-oxazolidin-2-on (R:S=1:1)

1.0 g (RS)-3-(4-Jodphenyl)-5-methoxymethyl-oxazolidin-2-on wurde wie in Beispiel 17 beschrieben umgesetzt und aufgearbeitet. Es resultierten 0.37 g racemisches (1:1)-Diastereomerengemisch in Form eines kristallinen Produkts. Smp. 46-48°.

Beispiel **14**

Gemisch aus (RS)-und (SR)-5-Methoxymethyl-3-[4-[-(RS)-3-oxo-cyclopentyl]-phenyl]-oxazolidin-2-on

Eine Suspension von 0.44 g (RS)-3-(4-Iod-phenyl)-5-methoxymethyl-2-oxa-zolidinon und 15 mg Tetrakis(triphenylphosphin)palladium in 1.1 ml 2-Cyclopentenon und 1.8 ml Triethylamin wurde unter Argon 24 h bei 80°C gerührt. Das Reaktionsgemisch wurde abgekühlt und nach Zugabe von 100 ml 2N Salzsäure mit Essigsäureethylester extrahiert. Die organische Phase wurde mit Magnesiumsulfat getrocknet und eingeengt. Der Rückstand (0.6 g braunes Oel) wurde über Kieselgel 60 mit Essigsäureethylester/Hexan-Gemischen (1:3-1:1) chromatographiert und lieferte 0.26 g (RS)- und (SR)-5-Methoxymethyl-3-[4-[(RS)-3-oxo-cyclopentyl]-phenyl]-oxazolidin-2-on als farbloses Oel. $^1$H-NMR($CDCl_3$) ppm: 7.53 (d, 2H), 7.26 (d, 2H), 4.76 (m, 1H), 4.06 (t, 1H), 3.93 (t, 1H), 3.65 (d, 2H), 3.44 (s, 3H), 3.40 (m, 1H), 2.65 (m, 1H), 2.36 (m, 4H), 1.98 (m, 1H).

Beispiel **15**

(RS)-5-Methoxymethyl-3-[4-(4-oxo-piperidin-1-yl)phenyl]-oxazolidin-2-on

Unter Argon wurde ein Gemisch aus 6.0 g 4-(4-Oxo-piperidin-1-yl)-phenylcarbamidsäureethylester, 5.0 g 4-Methoxymethyl-1,3-dioxolan-2-on und 0.6 g Kaliumcarbonat 3 h lang auf 160°C erhitzt. Das Reaktionsgemisch wurde abgekühlt, mit Methylenchlorid und Wasser versetzt und die Phasen getrennt. Die organische Phase wurde mit Magnesiumsulfat getrocknet und eingeengt. Der Rückstand (7.8 g) wurde mit Essigsäureethylester als Laufmittel über Kieselgel 60 chromatographiert. Man erhielt 0.6 g (RS)-5-Methoxymethyl-3-[4-(4-oxo-piperidin-1-yl)-phenyl]-oxazolidin-2-on. $^1$H-NMR($CDCl_3$ ) ppm: 7.46 (d, 2H), 6.98 (d, 2H), 4.76 (m, 1H), 3.99 (t, 1H), 3.88 (t, 1H), 3.64 (d, 2H), 3.57 (t, 4H), 3.44 (s, 3H), 2.56 (t, 4H).

Beispiel **16**

(RS)-3-[4-(4-Hydroxy-piperidin-1-yl)-phenyl]-5-methoxymethyl-2-oxazolidin-2-on

Zu einer Lösung von 0.6 g (RS)-5-Methoxymethyl-3-[4-(4-oxo-piperidin-1-yl)-phenyl]-oxazolidin-2-on in 25 ml Methanol und 2.5 ml Wasser wurden 0.3 g Natriumborhydrid gegeben. Dieses Gemisch wurde 24 h bei Raumtemperatur gerührt und anschliessend eingeengt. Der Rückstand wurde mit Methylenchlorid aufgenommen und mit 10%iger wässriger Ammoniaklösung gewaschen. Die organische Phase wurde mit Magnesiumsulfat getrocknet und eingeengt. Der Rückstand (0.6 g) wurde mit Essigsäureethylester/Hexan-Gemischen (1:1-2:1) über Kieselgel 60 chromatographiert. Kristallisation aus Essigsäureethylester lieferte 0.1 g (RS)-3-[4-(4-Hydroxy-piperidin-1-yl)-phenyl]-5-methoxymethyl-2-oxazolidin-2-on. Smp.: 149-152°.

Beispiel **17**

(RS)-3-[(4-Cycloheptyl)phenyl]-5-methoxymethyl-oxazolidin-2-on

4.18 g (16.0 mmol) 4-Cycloheptylphenylcarbamidsäureethylester wurden mit 1.3 g (16.0 mmol) Pyridin und 18 g (0.136 mol) (RS)-4-Methoxymethyl-1,3-dioxolan-2-on versetzt und bei 160° Badtemperatur während 20 Stunden gerührt. Nach dem Abkühlen wurde das Reaktionsgemisch an 400 g Kieselgel 60 mit Ether chromatographiert. Man erhielt nach Umkristallisieren aus Isopropylether 2.05 g (RS)-3-[(4-Cycloheptyl)phenyl]-5-methoxymethyl-oxazolidin-2-on als weisses Kristallisat. Smp.: 70-72°.

Beispiel **18**

(RS)-3-[(4-Adamantan-1-yl)phenyl]-5-methoxymethyl-oxazolidin-2-on

5.42 g (18.1 mmol) 4-Adamantan-1-yl-phenylcarbamidsäureethylester wurden mit 1.43 g (18.1 mmol) Pyridin und 18.0 g (0.136 mol) (RS)-4-Methoxymethyl-1,3-dioxolan-2-on versetzt und bei 160° Badtemperatur während 20 Stunden gerührt. Nach dem Abkühlen wurde das Reaktionsgemisch an 500 g Kieselgel 60 mit Ether chromatographiert. Man erhielt nach Umkristallisieren aus Methylenchlorid/Ether 1.4 g (RS)-3-[(4-Adamantan-1-yl)phenyl]-5-methoxymethyl-oxazolidin-2-on als weisses Kristallisat. Smp.: 145-147°.

Beispiel **19**

(RS)-3-[trans-4-[4-(5-Methoxymethyl-2-oxo-oxazolidin-3-yl)-phenyl]-cyclohexyloxy]-propionitril

3.05 g (10.0 mmol) (RS)-3-[4-trans-4-Hydroxycyclohexyl-)phenyl]-5-methoxymethyl-oxazolidin-2-on wurden in 150 ml Methylenchlorid gelöst und nacheinander mit 0.64 g (12.0 mmol) Acrylnitril und 1.35 g (12.0 mmol) Kalium-tert-butylat versetzt. Das Reaktionsgemisch wurde 18 Stunden bei Raumtemperatur gerührt. Danach wurde es mit Wasser gewaschen, die organische Phase über Natriumsulfat getrocknet, filtriert und eingedampft. Der Rückstand (4.0 g) wurde an 120 g Kieselgel 60 mit Essigester/n-Hexan (6:4) chromatographiert. Man erhielt nach Umkristallisieren aus Essigester 1.8 g (RS)-3-[trans-4-[4-(5-Methoxymethyl-2-oxo-oxazolidin-3-yl)-phenyl]-cyclohexyloxy]-propionitril. Smp.: 82-84°.

Beispiel **20**

(RS)-3-[trans-4-[4-(5-Methoxymethyl-2-oxo-oxazolidin-3-yl)-phenyl]cyclohexyloxy]propanamin • HCl

545.3 mg (14.4 mmol) Natriumborhydrid, suspendiert in 20 ml absolutem Tetrahydrofüran, wurden mit 1.64 g (14.4 mmol) Trifluoressigsäure während 5 Minuten bei Raumtemperatur versetzt. Dann wurden 1.29 g (3.6 mmol) (RS)-3- [trans- 4-[4-(5-Methoxymethyl- 2-oxo-oxazolidin-3-yl)-phenyl]-cyclohexyloxy]-propionitril, gelöst in 23 ml absolutem Tetrahydrofuran, bei Raumtemperatur innert 15 Minuten dazugetropft. Nach 18 Stunden Rühren wurde das Reaktionsgemisch eingedampft, der Rückstand auf Wasser und Methylenchlorid verteilt, die organische Phase mit Wasser gewaschen, über Natriumsulfat getrocknet, filtriert und eingedampft. Der Rückstand (1.4g) wurde an 39 g Kieselgel 60 mit Methylenchlorid (gesätt. $NH_3$)/Methanol (98:2) chromatographiert. Das Produkt wurde in Methylenchlorid mit Wasser gewaschen, über Natriumsulfat getrocknet, filtriert und das Filtrat mit etherischer Salzsäure sauer gestellt. Nach Umkristallisieren aus Methylenchlorid/Ether erhielt man 1.13 g (RS)-3-[trans-4-[4-(5-Methoxymethyl-2-oxo-oxazolidin-3-yl)-phenyl] cyclohexyloxy]propanamin • HCl. Smp.: 173-175°.

Beispiel **21**

2-(4,5-Dihydro-oxazol-2-yl)-benzoesäure(RS)-trans-4-[4-(5-methoxymethyl-2-oxo-oxazolidin-3-yl)-phenyl]-cyclohexylester

Zu einer Suspension von 690 mg (15.8 mmol) einer 55%igen Natriumhydrid-Dispersion in 20 ml absolutem Dimethylformamid tropfte man unter Argonatmosphäre innert 25 Minuten eine Lösung von 4.58 g (15.0 mmol) (RS)-3[4-trans-4-Hydroxy-cyclohexyl)-phenyl]-5-methoxymethyl-oxazolidin-2-on in 30 ml absolutem Dimethylformamid und rührte 3 Stunden bei Raumtemperatur. Anschliessend tropfte man eine Lösung von 3.81 g (15.0 mmol) N-(2-Bromethyl)phthalimid in 20 ml absolutem Dimethylformamid dazu und rührte bei Raumtemperatur über Nacht. Danach wurde das Reaktionsgemisch mit 0.9 g (15.0 mmol) Eisessig versetzt und eingedampft. Der Rückstand wurde in Essigester gelöst, mit Wasser gewaschen, über Natriumsulfat getrocknet, filtriert und eingedampft. Man erhielt nach Umkristallisieren aus Essigester 4.9 g 2-(4,5-Dihydro-oxazol-2-yl)-benzoesäure(RS)-trans-4-[4-(5-methoxymethyl-2-oxo-oxazolidin-3-yl)-phenyl]-cyclohexylester. Smp.: 182-184°.

Beispiel **22**

Essigsäure (RS)-trans-4-[4-(5-Methoxymethyl-2-oxo-oxazolidin-3-yl)-phenyl]-cyclohexylester

Einer Lösung von 0.5 g (1.639 mmol) (RS)-3-[4-trans-4-Hydroxy-cyclohexyl)-phenyl]-5-methoxymethyl-oxazolidin-2-on in 20 ml Methylenchlorid und 5 ml Pyridin wurde tropfenweise eine Lösung von 0.58 ml Acetylchlorid (8.195 mmol) in 10 ml Methylenchlorid unter Rühren zugegeben und während 3 Stunden bei Raumtemperatur weitergerührt. Das Reaktionsgemisch wurde auf Eiswasser gegossen, mit 3 N Salzsäure sauer gestellt und mit Methylenchlorid extrahiert. Die organische Phase wurde mit Wasser gewaschen, über Magnesiumsulfat getrocknet und das Lösungsmittel abdestilliert. Das Acetat kristallisierte beim Versetzen mit t-Butylmethylether. Erhalten wurden 0.35 g (62%) Essigsäure (RS)-trans-4-[4-(5-methoxymethyl-2-oxo-oxazolidn-3-yl)-phenyl]-cyclohexylester. Smp.: 78-79°.

Beispiel **23**

Benzoesäure (RS)-trans-4-[4-(5-methoxymethyl-2-oxo-oxazolidin-3-yl)-phenyl]-cyclohexylester

Eine Lösung von 0.5 g (1.639 mmol) (RS)-3-[4-(trans-4-Hydroxy-cyclohexyl)-phenyl]-5-methoxymethyl-oxazolidin-2-on in 20 ml Methylenchlorid und 5 ml Pyridin wurde tropfenweise mit einer Lösung von 0.95 ml (8.195 mmol) Benzoylchlorid in 10 ml Methylenchlorid versetzt und während 3 Stunden bei Raumtemperatur weitergerührt. Das Reaktionsgemisch wird auf Eiswasser gegossen, mit 3 N Salzsäure sauer gestellt und mit Methylenchlorid extrahiert. Die organische Phase wird mit Wasser gewaschen, über Magnesiumsulfat getrocknet und das Lösungsmittel abdestilliert. Das Benzoat kristallisierte beim Versetzen mit t-Butylmethylether. Erhalten wurden 0.52 g (77%) Benzoesäure (RS)-trans-4-[4-(5-methoxymethyl-2-oxo-oxazolidin-3-yl)-phenyl]-cyclohexylester. Smp.: 104-105°.

Beispiel **24**

5-Methoxy-3-[4-[5exo-[4-(5-methoxymethyl-2-oxo-oxazolidin-3-yl)-phenyl]-bicyclo[2.2.1]hept-2exo-yl]-phenyl]-oxazolidin-2-on

Bei der Herstellung der Verbindung von Beispiel 18 entsteht als Nebenprodukt die o.g. Verbindung, die chromatographisch (Kieselgel, Essigester/Hexan (1:1) abgetrennt werden konnte. Smp.: 152-154°.

Beispiel **25**

(RS)-4-Chlor-N-[2-[trans-4-(5-methoxymethyl-2-oxo-oxazolidin-3-yl)-phenyl]-cyclohexylamino]ethyl-benzamid hydrochlorid

Eine Lösung aus 1.31 g (4.3 mmol) (RS)-3-[4-(4-oxo-Cyclohexyl)-phenyl]-5-methoxymethyl-oxazolidin-2-on, 2.0 g (10 mmol) N-(2-Aminoethyl)-4-chlorbenzamid und 37 mg (0.2 mmol) p-Toluolsulfonsäuremonohydrat in 20 ml Toluol und 6 ml Dimethylformamid wurde 7 Stunden am Wasserabscheider gekocht. Unter Rühren wurden bei Raumtemperatur 330 mg (8.7 mmol) Natriumborhydrid und nach 30 Minuten 10 ml 1 N Salzsäure zugegeben. Die organische Phase wurde abgetrennt. Die Wasserphase wurde zweimal mit Methylenchlorid extrahiert. Dann wurden die organischen Phasen mit Natriumsulfat getrocknet, eingeengt und über Kieselgel mit Methylenchlorid/Methanol (9:1) chromatographiert. Der erhaltene Feststoff wurde aus Ethanol/Ether umkristallisiert. Man erhielt 200 mg weisse Kristalle. Smp.: 233-235°.

Beispiel **26**

(RS)-5-Methoxymethyl-3-[4-(4-methylen-cyclohexyl)-phenyl]-oxazolidin-2-on

Unter Argon wurden 5.76 g (13,8 mmol) Methyltriphenylphosphoniumbromid/Natriumamid-Gemisch in 170 ml Tetrahydrofuran 1 Stunde lang bei Raumtemperatur gerührt. Dann wurde innerhalb von 5 Minuten eine Lösung von 4.20 g (13.8 mmol) (RS)-3-[4-(4-oxo-Cyclohexyl)-phenyl]-5-methoxymethyl-oxazolidin-2-on zugetropft. Nach Kochen unter Rückfluss über Nacht wurde auf Wasser gegossen und mit Methylenchlorid extrahiert. Nach Trocknen mit Natriumsulfat und Einengen wurde mit Essigester/n-Hexan (1:2) über Kieselgel chromatographiert. Man erhielt 3.47 g (83%) Feststoff. Eine kleine Probe wurde aus Hexan umkristallisiert. Smp. : 63-65°.

Beispiel **27**

(RS)-3-[4-(cis- und trans-4-Hydroxymethyl-cyclohexyl)-phenyl]-5-methoxy-methyl-oxazolidin-2-on

Zu einer Suspension von 1.50 g (5.0 mmol) (RS)-Methoxymethyl-3-[4-(4-methylencyclohexyl)-phenyl]-oxazolidin-2-on in 250 ml Tetrahydrofuran gab man 0.19 g (5.0 mmol) Natriumborhydrid und tropfte 0.46 ml (5.0 mmol) Dimethylsulfat zu.Nach 4 Stunden Rühren bei ca 40° wurde auf 5° abgekühlt und 1.5 ml 2 N Natronlauge zugegeben. Danach wurden 0.77 ml 30%-iges $H_2O_2$ (7.5 mmol) langsam zugetropft. Man goss auf 100 ml gesättigte Kochsalzlösung, trennte die organische Phase ab und extrahierte die Wasserphase mit Methylenchlorid. Die organischen Phasen wurden mit Natriumsulfat getrocknet, eingeengt und über Kieselgel mit Methylenchlorid/Methanol chromatographiert. Es wurden 350 mg farbloses Oel erhalten. MS : m/e (% Basispeak): 319 ($M^+$,73), 246 (16), 233 (16), 170 (30), 158 (16), 144 (86), 118 (60), 95 (19), 91 (25), 77 (24), 71 (60), 45 (100).

Beispiel **28**

(RS)-3-[4-(cis oder trans-4-Hydroxy-4-methyl-cyclohexyl)-phenyl]-5-methoxymethyl-oxazolidin-2-on

Eine Lösung von 525 mg (1.6 mmol) Quecksilberacetat in 10 ml Wasser wurde mit 10 ml Tetrahydrofuran versetzt. Nach 15 Minuten Rühren wurde zu dieser Suspension eine Lösung aus 500 mg (1.7 mmol) (RS)-5-Methoxymethyl-3-[4-(4-methylencyclohexyl)-phenyl]-oxazolidin-2-on in 10 ml Tetrahydrofüran zugetropft. Es wurde 1.5 Stunden bei Raumtemperatur gerührt. Dann wurden 10 ml 2 N Natronlauge zugegeben und nach 10 Minuten Rühren eine Lösung aus 200 ml (5.3 mmol) Natriumborhydrid in 10 ml 2 N Natronlauge. Nach 30 Minuten Rühren wurde mit Methylenchlorid extrahiert, die organische Phase mit gesättigter Kochsalzlösung gewaschen und mit Natriumsulfat getrocknet. Nach Abdestillieren des Lösungsmittels wurde über Kieselgel mit Essigester/Hexan (1:2) chromatographiert. Man erhielt 150 mg weisse Kristalle. Smp.: 118-119°.

Beispiel **29**

(RS)-3-[4-(1,4-Dioxa-spiro[4,5]dec-8-yl)-phenyl]-5-methoxymethyl]-oxazolidin-2-on

Eine Lösung von 0.60 g (2 mmol) (RS)-3-[4-(4-oxo-Cyclohexyl)phenyl]-5-methoxymethyl-oxazolidinon, 0.15 g (2.4 mmol) Ethylenglycol und 30 mg p-Toluolsulfonsäuremonohydrat in 20 ml Toluol wurde 7 Stunden am Wasserabscheider gekocht. Das Toluol wurde abfiltriert, der Rückstand in 10 ml 2 N Natronlauge suspendiert und mit Methylenchlorid extrahiert. Nach Trocknen mit Natriumsulfat wurde das Lösungsmittel abdestilliert. Chromatographieren über Kieselgel mit Ether und Umkristallisieren mit Ether ergab 0.4 g weisse Kristalle. Smp.: 120-123°.

Beispiel **30**

(RS)-3-[4-(cis-4-Fluoro-cyclohexyl)-phenyl]-5-methoxymethyl-oxazolidin-2-on

0.5 g (RS)3-[4-(trans-4-Hydroxy-cyclohexyl)-phenyl]-5-methoxymethyl-oxazolidin-2-on wurden in 10 ml Methylenchlorid gelöst und bei Raumtemperatur binnen 30 Minuten mit einem Gemisch aus 2.2 ml Diethylaminoschwefeltrifluorid und 5 ml Methylenchlorid versetzt. Nach 2 Stunden Rühren bei Raumtemperatur wurde das Gemisch auf 50 ml Eiswasser gegossen und zweimal mit Methylenchlorid extrahiert. Die organische Phase wurde mit Natriumchloridlösung, Natriumhydrogencarbonatlösung und erneut mit einer Natriumchloridlösung gewaschen, mit Magnesiumsulfat getrocknet und eingeengt. Der Rückstand, 0.6 g gelber Honig, wurde an der 30-fachen Menge Kieselgel mit Methylenchlorid-Essigester (19:1) chromatographiert und lieferte 80 mg (RS)-3-[4-(cis-4-Fluoro-cyclohexyl)-phenyl]-5-methoxymethyl-oxazolidin-2-on als farbloses Oel. $^1$H-NMR ($CDCl_3$) ppm: 7.47 (d,2H), 7.24 (d,2H), 4.88 (bd,1H), 4.74 (m,1H), 4.04 (t,1H), 3.91 (m,1H), 3.64 (d,2H), 3.43 (s,3H), 2.53 (m,1H), 2.16 (m,2H), 1.72 (m,6H).

Beispiel **31**

(RS)-3-[4-(trans-4-Amino-cyclohexyl)-phenyl]-5-methoxymethyl-oxazolidin-2-on-hydrochlorid (1:1)

a) Ein Gemisch aus 1.0 g (R,S)-3-[4-(4-Oxo-cyclohexyl)-phenyl]-5-methoxymethyl-oxazolidin-2-on, 0.1 g p-Toluolsulfonsäure und 0.43 ml Benzylamin in 100 ml Toluol wurde 3 Stunden am Wasserabscheider gekocht. Das Lösungsmittel wurde entfernt, der Rückstand in 50 ml Methanol gelöst und mit 150 mg Natriumborhydrid über Nacht bei Raumtemperatur gerührt. Das Lösungsmittel wurde entfernt, der Rückstand mit 1N Salzsäure versetzt und mit Essigester gewaschen. Die wässrige Phase wurde mit Natronlauge basisch gemacht und mit Methylenchlorid extrahiert. Die Methylenchloridphase wurde mit Magnesiumsulfat getrocknet und eingeengt. Kristallisation des Rückstandes aus Essigester/Hexan lieferte 0.6 g beigefarbene Kristalle einer 1:1 Mischung aus (RS)-3-[4-(cis- und (RS)-3-[4-(trans-Benzylamino-cyclohexyl)-phenyl]-5-methoxymethyl-oxazolidin-2-on. Smp.: 93-94°C.

b) 0.5 g des Gemisches aus (RS)-3-[4-(cis- und (RS)-3-[4-(trans-Benzylamino-cyclohexyl)-phenyl]-5-methoxymethyl-oxazolidin-2-on wurden in 50 ml Ethanol unter Zugabe von einem Aequivalent 1N Salzsäure gelöst und mit Palladium/Kohle (10%) als Katalysator bei Raumtemperatur und Normaldruck hydriert. Nach Zugabe von Diethylether fallen 0.3 g (RS)-3-[4-(trans-4-Amino-cyclohexyl)-phenyl]-5-methoxymethyl-oxazolidin-2-on-hydrochlorid (1:1) als farblose Kristalle aus. Smp.: >250°C. $^1$H-NMR (DMSO) ppm: 8.12 (bs,3H), 7.47 (d,2H), 7.25 (d,2H), 4.81 (m,1H), 4.09 (t,1H), 3.77 (m,1H), 3.58 (m,2H), 3.32 (s,3H), 3.03 (m,1H), 2.47 (m,1H), 2.06 (m,2H), 1.80 (m,2H), 1.50 (m,4H).

Beispiel **32**

(R,S)-3-[4-(trans-4-Benzhydryloxy-cyclohexyl)-phenyl]-5-methoxymethyloxazolidin-2-on

0.5 g (RS)-3-[4-(trans-4-Hydroxy-cyclohexyl)-phenyl]-5-methoxymethyl-oxazolidin-2-on wurden in 5 ml Ethyldiisopropylamin mit 0.35 ml Chlordiphenylmethan auf 100°C erhitzt und 7 Stunden bei dieser Temperatur gerührt. Das Reaktionsgemisch wurde abgekühlt, mit 1N Salzsäure angesäuert und mit Essigester extrahiert. Die organische Phase wurde mit Wasser neutral gewaschen, mit Magnesiumsulfat getrocknet und eingeengt. Der Rückstand (0.7 g) wurde an der 30-fachen Menge Kieselgel mit Methylenchlorid/Essigsäureethylester-Gemischen chromatographiert. Man erhielt 0.3 g (RS)-3-[4-(trans-4-Benzhydryloxy-cyclohexyl)-phenyl]-5-methoxymethyl-oxazolidin-2-on als farblose Kristalle. Smp.: 146-148°C.

Beispiel **33**

9:1 Gemisch von (RS)-3-[4-(trans- und cis-4-Benzyloxy-cyclohexyl)-phenyl]-5-methoxymethyl-oxazolidin-2-on

1.0 g (RS)-3-[4-(Hydroxy-cyclohexyl)-phenyl]-5-methoxymethyl-oxazolidin-2-on wurden in 10 ml Dimethylformamid mit 0.21 g Natrium hydrid und 0.78 ml Benzylbromid versetzt. Das Reaktionsgemisch wurde über Nacht bei Raumtemperatur gerührt. Nach Zugabe von 50 ml Essigsäureethylester wurde dreimal mit Wasser gewaschen. Die Wasserphasen wurden mit Essigsäureethylester extrahiert. Die organischen Phasen wurden vereinigt, mit Magnesiumsulfat getrocknet und eingeengt. Die Chromatographie des Rückstandes (1.6 g) an der 30-fachen Menge Kieselgel mit Methylenchlorid/Essigester-Gemischen lieferte 0.8 g gelbes Oel, das aus tert-Butylmethylether kristallisierte. Man erhielt 0.6 g einer 9:1 Mischung aus (RS)-3-[4-(trans- und cis-4-Benzyloxy-cyclohexyl)-phenyl]-5-methoxymethyl-oxazolidin-2-on als farblose Kristalle. Smp.: 82-84°C. $^1$H-NMR ($CDCl_3$) ppm: 7.46 (d,2H), 7.35 (m,5H), 7.23 (d,2H), 4.74 (m,1H), 4.60 (s,2H), 3.99 (t,1H), 3.90 (m,1H), 3.63 (d,2H), 3.43 (s,3H), 3.40 (m,1H), 2.50 (m,1H), 2.22 (m,2H), 1.91 (m,2H), 1.46 (m,4H).

Beispiel **34**

(R)-3-[4-(trans-4-Methoxy-cyclohexyl)-phenyl]-5-methoxymethyl-oxazolidin-2-on

1.4 g (R)-3-[4-(trans-4-Hydroxy-cyclohexyl)-phenyl]-5-methoxymethyl-oxazolidin-2-on wurden in 14 ml Dimethylformamid mit 0.3 g Natrium hydrid und 1.43 ml Methyljodid versetzt. Das Reaktionsgemisch wurde über Nacht bei 40°C gerührt. Nach Zugabe von 100 ml Diethylether wurde dreimal mit Wasser gewaschen. Die Wasserphasen wurden mit Diethylether extrahiert. Die organischen Phasen wurden vereinigt, mit Magnesiumsulfat getrocknet und eingeengt. Die Chromatographie des Rückstandes (1.5 g) an der 30-fachen Menge Kieselgel mit Methylenchlorid/Essigester-Gemischen lieferte 1.3 g farbloses Oel, das aus tert-Butylmethylether kristallisierte. Man erhielt 1.1 g (R)-3- [4-(trans-4-Methoxy-cyclohexyl)-phenyl]-5-methoxymethyl-oxazolidin-2-on als farblose Kristalle. Smp.: 73-74°C.

Beispiel **35**

(RS)-3-(4-Cyclohex-1-enyl-phenyl)-5-methoxymethyl-oxazolidin-2-on

Eine Lösung von 410 mg Bis-(triphenylphosphin)-palladium(II)-diacetat, 2.0 g (RS)-3-(4-Jodphenyl)-5-methoxymethyl-oxazolidin-2-on und 1.8 ml 1-Tributyl-stannyl-1-cyclohexen in 10 ml Dimethylformamid wurde während 18 Stunden bei 80°C gerührt. Das Reaktionsgemisch wurde auf 100 ml Wasser gegossen und dreimal mit Essigsäureethylester extrahiert. Die organische Phase wurde mit gesättigter Kaliumfluoridlösung, gesättigter Natriumchloridlösung und Wasser gewaschen, mit Magnesiumsulfat getrocknet und eingeengt. Die Chromatographie des Rückstandes (2.0 g) an der 30-fachen Menge Kieselgel mit Essigsäureethylester/Hexan (1:2) lieferte 0.7 g Feststoff, der aus Essigsäureethylester/Hexan kristallisierte. Man erhielt 0.3 g (RS)-3-(4-Cyclohex-1-enyl-phenyl)-5-methoxymethyl-oxazolidin-2-on als farblose Kristalle. Smp.: 106-108°C.

Beispiel **36**

Gemisch von (RS)- und (SR)-3-[(RS)-4-Cyclohex-3-enyl-phenyl]-5-methoxymethyl-oxazolidin-2-on

Eine Lösung von 2.0 g (RS)-3-[4-(Hydroxy-cyclohexyl)-phenyl]-5-methoxymethyl-oxazolidin-2-on, 2.1 g Triphenylphosphin und 1.8 g Benzoesäure in 80 ml Tetrahydrofuran wurde bei Raumtemperatur binnen 15 Minuten mit 1.2 ml Azodicarbonsäurediethylester in 20 ml Tetrahydrofuran versetzt und während 22 Stunden bei Raumtemperatur gerührt. Das Reaktionsgemisch wurde eingeengt, der Rückstand in 100 ml Essigester gelöst, zweimal mit 10%iger Natriumcarbonatlösung und zweimal mit gesättigter Natriumchloridlösung gewaschen, mit Magnesiumsulfat getrocknet und eingeengt. Die Chromatographie des Rückstandes (7.0 g) an der 30-fachen Menge Kieselgel mit Methylenchlorid/Hexan-Gemischen lieferte als Nebenprodukt 0.6 g Oel, das aus Essigsäureethylester/Hexan kristallisierte. Man erhielt 0.4 g eines Gemisches von (RS)- und (SR)-3-[(RS)-4-Cyclohex-3-enyl-phenyl]-5-methoxymethyl-oxazolidin-2-on als farblose Kristalle. Smp.: 74-75°C. $^1$H-NMR ($CDCl_3$ ppm: 7.48 (d,2H), 7.23 (d,2H), 5.75 (m,2H), 4.75 (m,1H), 4.04 (t,1H), 3.91 (m,1H), 3.64 (d,2H), 3.43 (s,3H), 2.78 (m,1H), 2.16 (m,4H), 1.88 (m,1H), 1.75 (m,1H).

Beispiel **37**

Mischung aus (RS)- und (SR)-[4-[4-[(RS)-5-Methoxymethyl-2-oxo-oxazolidin-3-yl]-phenyl]-cyclohexyliden]-acetonitril

0.46 g Natrium wurden unter Argon in 50 ml Ethanol gelöst. Danach fügte man bei 20°C 3.5 g (=3.1 ml) Diethylcyanomethylphosphonat in 10 ml Ethanol gelöst zu. Man rührte noch 30 Minuten bei Raumtemperatur nach und fügte dann 2.0 g (RS)-5-Methoxymethyl-3-[4-(4-oxo-cyclohexyl)-phenyl]-oxozolidin-2-on zu. Man rührte danach noch 1 Stunde am Rückfluss, kühlte auf Raumtemperatur, stellte mit ca. 0.7 ml Eisessig auf pH 6 und dampfte das Reaktionsgemisch im Vakuum ein. Das rohe Gemisch (~ 4.2 g) wurde durch 200 g Silicagel chromatographiert. Mit Dichlormethan/Ethylacetat 9:1 eluierte man 1.65 g einer Mischung aus (RS)- und (SR)-[4-[4-[(RS)-5-Methoxymethyl-2-oxo-oxazolidin-3-yl]-phenyl]-cyclohexyliden]-acetonitril als weisse Kristalle. Eine Probe aus Ethylacetat/Hexan umkristallisiert ergab weisse Kristalle vom Smp. 121-125°C.

Beispiel **38**

(RS)-[trans-4-[4-(5-Methoxymethyl-2-oxo-oxazolidin-3-yl)-phenyl]-cyclohexyl]-acetonitril

1.65 g einer Mischung aus a) (R)-[4-[4-[(R)-, b) (R)-[4-[4-[(S)-, c) (S)-[4-[4-[(R)- und d) (S)-[4-[4-[(S)-5-Methoxymethyl-2-oxo-oxazolidin-3-yl]-phenyl]-cyclohexyliden]-acetonitril (Verhältnis a:b=c:d = 1:1) wurden in 175 ml Eisessig und 0.3 g Pd-Kohle 10% bei Normaldruck und Raumtemperatur bis zur theoretischen Aufnahme (~16 Stunden) hydriert. Man nutschte vom Katalysator ab und dampfte im Vakuum ein. Der Rückstand (1.6 g) wurde in Ethylacetat gelöst und mit Natriumhydrogencarbonatlösung 10% sowie Natriumchloridlösung gesättigt gewaschen. Nach dem Trocknen und Eindampfen erhielt man 1.6 g Kristalle, welche zur Reinigung durch 30 g Silicagel chromatographiert wurden. Eluiert wurde mit Dichlormethan/Ethylacetat 9:1. Man erhielt nach dem Zusammenfassen der nach Dünnschichtchromatogramm reinen Fraktionen und Umkristallisieren aus Ethylacetat/Hexan 0.72 g (RS)-[trans-4-[4-(5-Methoxymethyl-2-oxo-oxazolidin-3-yl)-phenyl]-cyclohexyl]-acetonitril als weisse Kristalle vom Smp. 118-120°C.

Beispiel **39**

Mischung aus (RR)- und (SR)-4-[4-(5-Methoxymethyl-2-oxo-oxazolidin-3-yl)-phenyl]-cyclohexyl]-acetonitril

1.1 g (R)-[4-[4-(5-Methoxymethyl-2-oxo-oxazolidin-3-yl]-phenyl]-cyclohexyliden]-acetonitril wurden in 50 ml Methanol gelöst, mit 0.2 g Pd-Kohle 10% versetzt und bei Raumtemperatur und Normaldruck bis zur theoretischen Wasserstoffaufnahme hydriert. Man nutschte vom Katalysator und dampfte im Vakuum ein. Der ölige Rückstand wurde über 15 g Silicagel chromatographiert. Eluiert wurde mit Dichlormethan/Ethylacetat 9:1. Die nach Dünnschichtchromatographie reinen Fraktionen wurden vereinigt und aus Ethylacetat/Hexan kristallisiert. Man erhielt 0.64 g (R)-[trans-[4-[4-(5-Methoxymethyl-2-oxo-oxazolidin-3-yl)-phenyl]-cyclohexyl]-acetonitril als weisse Kristalle vom Smp. 90-91 °C.

$$\left.\begin{array}{l}[\alpha]^{20°}_{589\,D} = -39{,}6° \\ [\alpha]^{546}_{Hg} = -47{,}6°\end{array}\right\} c = 1\% \text{ in Dichlormethan}$$

Beispiel **40**

1:1 Mischung aus cis- und trans-(RS)-3-[4-[4-(2-Amino-ethyl)-cyclohexy]-phenyl]-5-methoxymethyl-oxazolidin-2-on-hydrochlorid (1:1)

2.1 g einer Mischung aus a) (R)-[4-[4-[(R)-, b) (R)-[4-[4-[(S)-, c) (S)-[4-[4-[(R)- und d) (S)-[4-[4-[(S)-5-Methoxymethyl-2-oxo-oxazolidin-3-yl]-phenyl]-cyclohexyliden]-acetonitril (Verhältnis a:b=c:d = 1:1) wurden in 200 ml methanolischem Ammoniak 20% (G/V) und 3.0 g Raney-Nickel während ca. 40 Stunden bei Normaldruck und Raumtemperatur bis zur theoretischen Wasserstoffaufnahme hydriert. Man nutschte vom Katalysator ab und dampfte im Vakuum ein. Der rohe Rückstand (~1.8 g) wurde durch 15 g Silicagel chromatographiert. Man eluierte mit Dichlormethan/Methanol/Ammoniak

25% (250:5:1) sowie mit Dichlormethan gesättigt mit Ammoniak 25% + 5% Methanol. Die nach Dünnschichtchromatographie reinen Fraktionen wurden vereinigt und eingedampft. Man erhielt 1.4 g Base als trübes Oel. Davon wurden 0.7 g in das Hydrochlorid überführt (ethanolische Salzsäure/Ether). Man erhielt 0.5 g (1:1) einer Mischung aus cis- und trans-(RS)-3-[4[4-(2-Amino-ethyl)-cyclohexyl]-phenyl]-5-methoxymethyl-oxazolidin-2-on-hydrochlorid (1/1) als weisse Kristalle vom Smp. 203-207°C.

Beispiel **41**

(RS)-N-[2-[4-[4-(5-Methoxymethyl-2-oxo-oxazolidin-3-yl)-phenyl]-cyclohexyl]-ethyl]-acetamid (Mischung von trans:cis = 6:1)

0.57 g einer 1:1 Mischung von cis- und trans-(RS)-3-[4-[4-(2-Aminoethyl)-cyclohexyl]-phenyl]-5-methoxymethyl-oxazolidin-2-on wurden in 2 ml Pyridin gelöst und mit 0.21 g Essigsäureanhydrid versetzt. Man rührte 3,5 Stunden bei Raumtemperatur und löste danach das Reaktionsgemisch in Dichlormethan. Die organische Phase wurde mit 1N Salzsäure, Wasser, Natriumhydrogencarbonat sowie gesättigter Kochsalzlösung gewaschen, anschliessend getrocknet und eingedampft. Als Rückstand verblieben 0.75 g trübes Oel, welches aus Ethylacetat/Methyl-tert.butylether kristallisiert wurde. Man erhielt 0.25 g (RS)-N-[2-[4-[4-(5-Methoxymethyl-2-oxo-oxazolidin-3-yl)-phenyl]-cyclohexyl]ethyl]-acetamid (Mischung von trans:cis = 6:1), als weisse Kristalle vom Smp. 120-122°C.

Beispiel **42**

(RS)-4-[4-[5-Methoxymethyl-2-oxo-oxazolidin-3-yl)-phenyl]-piperidin-1-carbonsäure-t-butylester

5.5 g (15.0 mmol) 4-(4-Ethoxycarbonylamino-phenyl)-piperidin-1-carbonsäure-t-butylester und 20 g (0.15 mol) (RS)-4-(Methoxymethyl)-1,3-dioxolan-2-on wurden in Gegenwart von 2.37 g (30.0 mmol) Pyriden während 18 Stunden bei einer Oelbadtemperatur von 160°C intensiv gerührt. Nach dem Abkühlen des Reaktionsgemisches wurde es an 840 g Kieselgel 60 mit Ether chromatographiert. Man erhielt 3.4 g (RS)-4-[4(5-Methoxymethyl-2-oxo-oxazolidin-3-yl)-phenyl]-piperidin-1-carbonsäure-t-butylester als ein gelbliches Oel. $^{1}$H-NMR ($CDCl_3$) ppm: 7.48 (d,2H), 7.22 (d,2H), 4.76 (m,1H), 4.25 (bd,2H), 4.05 (t,1H), 3.92 (t,1H), 3.64 (d,2H), 3.43 (s,3H), 2.80 (t,2H), 2.64 (m,1H), 1.78 (bd,2H), 1.61 (m,2H), 1.49 (s,9H).

Beispiel **43**

(RS)-4-[4-(5-Methoxymethyl-2-oxo-oxazolidin-3-yl)-phenyl]-piperidin • HCl

2.51 g (6.4 mmol) (RS)-4-[4-(5-Methoxymethyl-2-oxo-oxazolidin-3-yl)-phenyl]-piperidin-1-carbonsäure-t-butylester wurden in 40 ml Essigester gelöst und unter Rühren bei Zimmertemperatur mit 40 ml einer 2.2 molaren, mit HCl-gesättigten Essigester-Lösung versetzt. Nach 90 Minuten wurde die Suspension eingedampft und mit Toluol abdestilliert. Man erhielt 1.47 g (RS)-4-[4-(5-Methoxymethyl-2-oxo-oxazolidin-3-yl)-phenyl]-piperidin • HCl. Smp.: 161-163°C. $^{1}$H-NMR (DMSO) ppm: 8.98 (bs,2H), 7.52 (d,2H), 7.24 (d,2H), 4.81 (m,1H), 4.10 (t,1H), 3.79 (t,1H), 3.58 (m,2H), 3.35 (m,2H), 3.32 (s,3H), 2.97 (bm,2H), 2.80 (m,1H), 1.87 (bm,4H).

Beispiel **44**

(RS)-1-Benzyl-4-[4-(5-methoxymethyl-2-oxo-oxazolidin-3-yl)-phenyl]-piperidin • HCl

290.4 mg (1.0 mmol) (RS)-4-[4-(5-Methoxymethyl-2-oxo-oxazolidin-3-yl)-phenyl]-piperidin wurden in 5 ml abs. Dimethylformamid gelöst und in Gegenwart von 404.8 mg (4.0 mmol) Triethylamin mit 171.0 mg (1.0 mmol) Benzylbromid versetzt. Das Reaktionsgemisch wurde während 90 Minuten bei 60°C gerührt. Danach wurde es eingedampft, der Rückstand auf Essigester und 3N Ammoniak-Lösung verteilt, die organische Phase mit Wasser gewaschen, über Natriumsulfat getrocknet, filtriert und eingedampft. Die Base wurde in Ethanol gelöst, mit etherischer Salzsäure sauer gestellt und wiederholt mit Toluol abdestilliert. Man erhielt nach Umkristallisieren aus Ethanol/Ether 291.2 mg (RS)-1-Benzyl-4-[4-(5-methoxymethyl-2-oxo-oxazolidin-3-yl)-phenyl]-piperidin • HCl. Smp.: 175-177°C.

Beispiel **45**

(RS)-1-(4-Fluorbutyrophenon-ω-yl)-4-[4-(5-methoxymethyl-2-oxo-oxazolidin-3-yl)-phenyl]-piperidin • HCl

253.0 mg (0.87 mmol) (RS)-4-[4-(5-Methoxymethyl-2-oxo-oxazolidin-3-yl)-phenyl]-piperidin wurden in 15 ml abs. Dimethylformamid gelöst und in Gegenwart von 352.7 mg (3.485 mmol) ,Triethylamin mit 174.8 mg (0.87 mmol) ω-Chlor-4-fluorbutyrophenon versetzt. Das Reaktionsgemisch wurde während 5 Stunden bei 100°C gerührt. Danach wurde es eingedampft, der Rückstand auf Essigester und 3N Ammoniak-Lösung verteilt, die organische Phase mit Wasser gewaschen, über Natriumsulfat getrocknet, filtriert und eingedampft. Der Rückstand wurde in Ethanol gelöst und mit etherischer Salzsäure sauer gestellt. Man erhielt nach Umkristallisieren aus Alkohol/Ether 135.9 mg (RS)-1-(4-Fluorbutyrophenon-ω-yl)-4-[4-(5-methoxymethyl-2-oxo-oxazolidin-3-yl)-phenyl]-piperidin • HCl. Smp.: 182-184°C.

Beispiel **46**

(RS)-4-[4-(5-Methoxymethyl-2-oxo-oxazolidin-3-yl)-phenyl]-1-(4-trifluormethyl-benzoyl)-piperidin

273.1 mg (0.94 mmol) (RS)-4-[4-(5-Methoxymethyl-2-oxo-oxazolidin-3-yl)-phenyl]-piperidin • HCl wurden in 20 ml Methylenchlorid gelöst und in Gegenwart von 380.7 mg (3.76 mmol) Triethylamin mit 196.2 mg (0.94 mmol) 4-Trifluormethylbenzoylchlorid versetzt. Das Reaktionsgemisch wurde 17 Stunden bei Raumtemperatur gerührt. Danach wurde es mit Essigester versetzt, mit 1N Salzsäure und Wasser gewaschen und über Natriumsulfat getrocknet. Nach dem Umkristallisieren aus Essigester/Ether erhielt man 356.3 mg (RS)-4-[4-(5-Methoxymethyl-2-oxo-oxazolidin-3-yl)-phenyl]-1-(4-trifluormethyl-benzoyl)-piperidin. Smp. 164-166°C.

Beispiel **47**

(RS)-1-Acetyl-4-[4-(5-methoxymethyl-2-oxo-oxazolidin-3-yl)-phenyl]-piperidin

177.1 mg (0.61 mmol) (RS)-4-[4-(5-Methoxymethyl-2-oxo-oxazolidin-3-yl)-phenyl]-piperidin wurden in 20 ml Methylenchlorid gelöst und in Gegenwart von 53.1 mg (0.67 mmol) Pyridin mit 68.5 mg (0.67 mmol) Essigsäure anhydrid versetzt. Nach 18 Stunden Rühren bei Raumtemperatur wurde es mit Wasser gewaschen und die organische Phase über Natriumsulfat getrocknet. Nach dem Filtrieren und Eindampfen des Filtrates wurde das Produkt im Hochvakuum getrocknet. Man erhielt 197.3 mg (RS)-1-Acetyl-4-[4-(5-methoxymethyl-2-oxo-oxazolidin-3-yl)-phenyl]-piperidin als ein gelbliches Oel. $^1$H-NMR ($CDCl_3$) ppm: 7.49 (d,2H), 7.20 (d,2H), 4.80 (m,2H), 4.05 (t,1H), 3.92 (m,2H), 3.64 (d,2H), 3.43 (s,3H), 3.21 (m,1H), 2.68 (m,2H), 2.14 (s,3H), 1.85 (m,2H), 1.60 (m,2H).

Beispiel **48**

(RS)-4-[4-(5-Methoxymethyl-2-oxo-oxazoliden-3-yl)-phenyl]-1-(1-oxo-3-phenyl-2-(E)-propenyl)-piperidin

201.7 mg (0.695 mmol) (RS)-4-[4-(5-Methoxymethyl-2-oxo-oxazolidin-3-yl)-phenyl]-piperidin • HCl wurden in 15 ml Methylenchlorid gelöst und in Gegenwart von 281.2 mg (2.78 mmol) Triethylamin unter Rühren bei Raumtemperatur mit einer Lösung von 115.7 mg (0.695 mmol) Zimtsäurechlorid in 5 ml Methylenchlorid tropfenweise versetzt. Nach 17 Stunden wurde es mit Essigester verdünnt und die organische Phase mit 1N Salzsäure und Wasser gewaschen. Man erhielt nach dem Umkristallisieren aus Essigester/Ether 228.9 mg (RS)-4-[4-(5-Methoxymethyl-2-oxo-oxazolidin-3-yl)-phenyl]-1-(1-oxo-3-phenyl-2-(E)-propenyl)-piperidin. Smp.: 136-138°C.

Beispiel **49**

(RS)-4-[4-(5-Methoxymethyl-2-oxo-oxazolidin-3-yl)-phenyl]-piperidin-1-carbonsäure-ethylester

1.12 g (3.5 mmol) 4-(4-Ethoxycarbonylamino-phenyl)-piperidin-1-carbonsäure-ethylester wurden in Gegenwart von 276.8 mg (3.5 mmol) Pyridin mit 4.0 g (30.28 mmol) (RS)-4-(Methoxymethyl)-1,3-dioxolan-2-on versetzt und während 18 Stunden bei einer Oelbadtemperatur von 160°C intensiv gerührt. Nach dem Abkühlen wurde das Reaktionsgemisch an 125 g Kieselgel 60 mit Ether chromatographiert. Man erhielt nach Umkristallisieren aus Methylenchlorid/Isopropylether 976 mg (RS)-4-[4-(5-Methoxymethyl-2-oxo-oxazolidin-3-yl)-phenyl]-piperidin-1-carbonsäure-ethylester. Smp.: 86-88°C.

Beispiel **50**

(R)-5-Methoxymethyl-3-[4-(trans-4-methylsulfanylmethoxy-cyclohexyl)-phenyl]-oxazolidin-2-on

9.5 g (31.11 mmol) (R)-3-[trans-(4-Hydroxycyclohexyl)-phenyl]-5-methoxymethyl-oxazolidin-2-on wurden in 120 ml Dimethylsulfoxid gelöst und nacheinander mit 2 ml Wasser, 25 ml Eisessig und 80 ml Essigsäureanhydrid versetzt. Das Reaktionsgemisch wurde 22 Stunden bei Raumtemperatur gerührt. Danach wurde es zu einer eisgekühlten Lösung von 130 g Natriumcarbonat in 2 l Wasser portionsweise eingetragen. Nach der Zugabe wurde das Gemisch noch 1 Stunde gerührt. Nachher wurde das Produkt mit Methylenchlorid extrahiert, die organische Phase mit Wasser gewaschen, über Natriumsulfat getrocknet, filtriert und eingedampft. Man erhielt 11.2 g gelbliches Oel, das beim Versehen mit Ether/n-Hexan kristallines (R)-5-Methoxymethyl-3-[4-(trans-4-methylsulfanylmethoxy-cyclohexyl)-phenyl]-oxazolidin-2-on ergab. Smp.: 54-56°C, $[a]_D$ = -30.8° (c=1,0, $CHCl_3$). $^1$H-NMR ($CDCl_3$) ppm: 7.47 (d,2H), 7.21 (d,2H), 4.70 (m,1H), 4.04 (t,1H), 3.93 (t,1H), 3.70 (m,1H), 3.63 (d,2H), 3.43 (s,3H), 2.50 (m,1H), 2.18 (s,3H), 2.12 (d,2H), 1.90 (d,2H), 1.56 (bm,4H).

Beispiel **51**

(R)-3-[4-(trans-4-Methansulfinylmethoxy-cyclohexyl)-phenyl]-5-methoxymethyl-oxazolidin-2-on (S-oxyd R:S=1:1 Diastereomerengemisch)

10.7 g (29.28 mmol) (R)-5-Methoxymethyl-3-[4-(trans-4-methylsulfanylmethoxy-cyclohexyl)-phenyl]-oxazolidin-2-on wurden in 500 ml Methanol gelöst, auf 0°C abgekühlt und mit einer Lösung von 4.7 g (22.0 mmol) Natriumperjodat in 150 ml Wasser während 90 Minuten tropfenweise unter Rühren versetzt. Das Reaktionsgemisch wurde im Kühlraum bei 4-5°C während 19 Stunden gerührt. Danach wurde es mit 1 l Wasser verdünnt und mit Methylenchlorid

extrahiert. Die organische Phase wurde mit Wasser gewaschen, über Natriumsulfat getrocknet und filtriert. Das Filtrat wurde durch 400 g Kieselgel 60 Polster filtriert urd nacheinander separat mit Essigester (800 ml), Methanol (1 l) und Tetrahydrofuran (800 ml) gewaschen. Nach dem Eindampfen des Essigester-Eluates erhielt man 3.16 g Edukt zurück. Die Methanol- und Tetrahydrofüran-Eluate ergaben nach dem Eindampfen 8.11 g (R)-3-[4-(trans-4-Methansulfinylmethoxy-cyclohexyl)-phenyl]-5-methoxymethyl-oxazolidin-2-on (S-oxid R:S=1:1 Diastereomerengemisch), als gelbes Oel, Sdp.: 205°/0.02 mbar, $[a]_D$ = -32.2° (c=1,0%, DMSO).

Beispiel **52**

(R)-[trans-4-[4-(5-Methoxymethyl-2-oxo-oxazolidin-3-yl)-phenyl]-cyclohexyloxy]-acetonitril

7.7 g (20.2 mmol) (R)-3-[4-8trans-4-Methansulfinylmethoxy-cyclohexyl)-phenyl]-5-methoxymethy]-oxazolidin-2-on (S-oxid, RS=1:1 Diastereomerengemisch) wurden in 200 ml abs. Tetrahydrofuran gelöst und in Gegenwart von 420 mg (1.32 mmol) Zinkjodid mit einer Lösung von 17.73 g (0.179 mol) Trimethylsilylchlorid in 40 ml abs. Tetrahydrofuran tropfenweise unter Rühren während 30 Minuten versetzt. Das Reaktionsgemisch wurde 24 Stunden bei Raumtemperatur gerührt. Danach wurde es eingedampft, der Rückstand auf Essigester-Wasser verteilt, die organische Phase mit Wasser gewaschen, über Natriumsulfat getrocknet, filtriert und eingedampft. Der Rückstand (7.75 g) an 320 g Kieselgel 60 mit Essigester/n-Hexan (1:1) chromatographiert. Man erhielt 2.64 g (R)-[trans-4-[4-(5-Methoxymethyl-2-oxo-oxazolidin-3-yl)-phenyl]-cyclohexyloxy]-acetonitril. als farbloses Oel; Sdp.: 210°/0.02 mbar; $[a]_D$ = -32.2° (c=1,0% in $CHCl_3$). $^1$H-NMR ($CDCl_3$) ppm: 7.48 (d,2H), 7.20 (d,2H), 4.75 (m,1H), 4.32 (d,2H), 4.02 (t,1H), 3.93 (t,1H), 3.64 (d,2H), 3.61 (m,1H), 3.43 (s,3H), 2.55 (m,1H), 2.39 (m,2H), 1.95 (m,2H), 1.50 (bm,4H).
Nach Umkristallisieren aus Ether/Hexan erhielt man weisse Kristalle vom Smp. 43-45°C.

Beispiel **53**

(RS)-5-Methoxymethyl-3-[4-(trans-4-methylsulfanylmethoxy-cyclohexyl)-phenyl]-oxazolidin-2-on

305.4 mg (1.0 mmol) (RS)-3-[trans-4-Hydroxycyclohexyl)-phenyl]-5-methoxymethyl-oxazolidin-2-on wurden in 35 ml Cyclohexan vorgelegt und bei Raumtemperatur unter Rühren mit 186.9 mg (1.1 mmol) Silbernitrat, 121.4 mg (1.2 mmol) Triethylamin und 115.9 mg (1,2 mmol) Chlormethylmethylsulfid versetzt. Das Reaktionsgemisch wurde 20 Stunden gerührt. Danach wurde es vom Silberchlorid durch Dicalit filtriert, das Filtrat mit gesättigtem Natriumhydrogencarbonat gewaschen, die organische Phase über Natriumsulfat getrocknet, filtriert und eingedampft. Man erhielt 354.5 mg (RS)-5-Methoxymethyl-3-[4-(trans-4-methylsulfanylmethoxy-cyclohexyl)-phenyl] -oxazolidin-2-on als gelbliches Oel.

Beispiel **54**

(RS)-3-[4-(trans-4-Methansulfinylmethoxy-cyclohexyl)-phenyl]-5-methoxymethyl-oxazolidin-2-on (Diastereomerengemisch)

1.71 g (4.68 mmol) (RS)-5-Methoxymethyl-3-[4-(trans-4-methylsulfanylmethoxy-cyclohexyl)-phenyl]-oxazolidin-2-on wurden in 100 ml Methanol gelöst, auf 0°C abgekühlt und mit einer Lösung von 1.0 g (4.68 mmol) Natriummetaperjodat, gelöst in 40 ml Wasser, unter Rühren während 40 Minuten tropfenweise versetzt. Das Reaktionsgemisch wurde 24 Stunden bei ca. 4°C gehalten. Danach wurde es mit Wasser verdünnt und das Produkt mit Methylenchlorid extrahiert. Die organische Phase wurde mit Wasser gewaschen, über Natriumsulfat getrocknet und filtriert. Das Filtrat wurde durch 50 g Kieselgel 60 filtriert und separat mit Methanol gewaschen. Nach dem Eindampfen des Methanol-Eluates wurde das Diastereomerengemisch (RS)-3-[4-(trans-4-Methansulfinylmethoxy-cyclohexyl)-phenyl]-5-methoxymethyl-oxazolidin-2-on aus Benzol kristallisiert. Smp.: 98-100°C.

Beispiel **55**

(RS)-[trans-4-[4-(5-Methoxymethyl-2-oxo-oxazolidin-3-yl)-phenyl]-cyclohexyloxy]-acetonitril

536.3 mg (1.40 mmol) (RS)-3-[4-(trans-4-Methansulfinylmethoxy-cyclohexyl)-phenyl]-5-methoxymethyl-oxazolidin-2-on (Diastereomerengemisch) wurden in 25 ml abs. Tetrahydrofuran gelöst und unter Rühren mit 50 mg Zinkjodid und 465 mg (2.81 mmol) Trimethylsilylcyanid versetzt. Nach 21 Stunden Rühren bei Raumtemperatur wurde das Reaktionsgemisch mit 40 ml Methylenchlorid und 40 ml Wasser verdünnt, die organische Phase mit Wasser gewaschen, über Natriumsulfat getrocknet, filtriert und eingedampft. Der Rückstand (517,5 mg) wurde an 25 g Kieselgel 60

mit Essigester/n-Hexan (1:1) chromatographiert. Man erhielt 90 mg (RS)-[trans-4-(5-Methoxymethyl-2-oxo-oxazolidin-3-yl)-phenyl]-cyclohexyloxy]-acetonitril als farbloses Oel. $^{1}$H-NMR ($CDCl_3$) ppm: 7.48 (d,2H), 7.20 (d,2H), 4.75 (m,1H), 4.32 (d,2H), 4.05 (t,1H), 3.92 (t,1H), 3.65 (d,2H), 3.60 (m,1H), 3.43 (s,3H), 2.50 (m,1H), 2.20 (d,2H), 1.95 (d,2H), 1.47 (bm,4H).

Beispiel **56**

(RS)-3-[4-(trans-4-Methansulfonylmethoxy-cyclohexyl)-phenyl]-5-methoxymethyl-oxazolidin-2-on

320.1 mg (0.88 mmol) (RS)-5-Methoxymethyl-3-[4-(trans-4-methylsulfanylmethoxy-cyclohexyl)-phenyl]-oxazolidin-2-on wurden in 15 ml Methylenchlorid gelöst und mit 226.9 mg (0.97 mmol) m-Chlorperbenzoesäure versetzt. Nach 3 Stunden bei Raumtemperatur wurde das Reaktionsgemisch mit Natriumhydrogencarbonat-Lösung und Wasser gewaschen, die organische Phase über Natriumsulfat getrocknet, filtriert und eingedampft. Der Rückstand (343.2 mg) wurde an 20 g Kieselgel 60 mit Essigester chromatographiert. Man erhielt nach Umkristallisieren aus Methylenchlorid/Ether 136.7 mg (RS)-3-[4-(trans-4-Methansulfonylmethoxy-cyclohexyl)-phenyl]-5-methoxymethyl-oxazolidin-2-on als weisses Kristallisat vom Smp. 145-147°C.

Beispiel **57**

(RS)-3-[trans-4-[4-(5-Methoxymethyl-2-oxo-oxazolidin-3-yl)-phenyl]-cyclohexyloxyl-ethanamin • HCl

189.7 mg (0.5508 mmol) (RS)-[trans-4-[4-(5-Methoxymethyl-2-oxo-oxazolidin-3-yl)-phenyl]-cyclohexyloxy]-acetonitril wurden in 15 ml mit trockenem Ammoniakgas gesättigten Methanol gelöst und in Gegenwart von 300 mg Raney-Nickel (Typ B 113W Degussa) hydriert. Nach 30 Minuten wurde es vom Katalysator abfiltriert und das Filtrat eingedampft. Der Rückstand wurde in Methylenchlorid gelöst, mit Wasser gewaschen, über Natriumsulfat getrocknet, filtriert und eingedampft. Die Base wurde in Alkohol gelöst und mit etherischer Salzsäure sauer gestellt. Nach Umkristallisieren aus Alkohol-Ether erhielt man 186.0 mg (RS)-3-[trans-4-[4-(5-Methoxymethyl-2-oxo-oxazolidin-3-yl)-phenyl]-cyclohexyloxy]-ethanamin • HCl. Smp.: 204-206°C.

Beispiel **58**

(RS)-3-[trans-4-[4-(5-Methoxymethyl-2-oxo-oxazolidin-3-yl)-phenyl]-cyclohexyloxy]-propylacetamid

199.5 mg (0.5 mmol) (RS)-3-[trans-4-[4-(5-Methoxymethyl-2-oxo-oxazolidin-3-yl)-phenyl]-cyclohexyloxy]-propanamin • HCl wurden in 5 ml Methylenchlorid gelöst und in Gegenwart von 87.01 mg (1.1 mmol) Pyridin mit 43.2 mg (0.55 mmol) Acetylchlorid versetzt. Nach 3 Stunden wurde es mit Wasser gewaschen, die organische Phase über Natriumsulfat getrocknet, filtriert und eingedampft. Nach Umkristallisieren aus Essigester/Ether erhielt man 175.8 mg (RS)-3-[trans-4-[4-(5-Methoxymethyl-2-oxo-oxazolidin-3-yl)-phenyl]-cyclohexyloxy]-propylacetamid. Smp.: 72-74°C.

Beispiel **59**

(R)-3-[trans-4-[4-(5-Methoxymethyl-2-oxo-oxazolidin-3-yl)-phenyl]-cyclohexyloxy]-propionitril

4.58 g (15.0 mmol) (R)-3-[trans-(4-Hydroxycyclohexyl)-phenyl]-5-methoxymethyl-oxazolidin-2-on wurden in 400 ml Methylenchlorid gelöst und nacheinander mit 0.96 g (18.0 mmol) Acrylnitril und 2.02 g (18.0 mmol) Kalium-tert-butylat versetzt. Das Reaktionsgemisch wurde 16 Stunden bei Raumtemperatur gerührt. Danach wurde es mit Wasser versetzt, vom Unlöslichen durch Dicalit filtriert, die organische Phase mit Wasser gewaschen, über Natriumsulfat getrocknet, filtriert und eingedampft. Der Rückstand (5.1 g) wurde an 204 g Kieselgel 60 mit Essigester/n-Hexan (6:4) chromatographiert. Man erhielt nach dem Umkristallisieren aus tert-Butyl-methylether 1.6 g (R)-3-[trans-4-[4-(5-Methoxymethyl-2-oxo-oxazolidin-3-yl)-phenyl]-cyclohexyloxy]-propionitril. Smp.: 57-59°C. $[a]_D$ = -30.0° (c=0.7 in $CHCl_3$).

Beispiel **60**

(R)-3-[trans-4-(3-Amino-propoxy)-cyclohexyl]-phenyl]-5-methoxymethyl-oxazolidin-2-on-hydrochlorid

932 mg (2.6 mmol) (R)-3-[trans-4-[4-(5-Methoxymethyl-2-oxo-oxazolidin-3-yl)-phenyl]-cyclohexyloxy]-propionitril wurden in 60 ml mit trockenem Ammoniakgas gesättigten Methanol gelöst und in Gegenwart von 0.9 g Raney-Nickel (Typ B 113W Degussa) hydriert. Nach 3 Stunden wurde es vom Katalyt genutscht und das Filtrat eingedampft. Der Rückstand wurde in Methylenchlorid gelöst, mit Wasser gewaschen, über Natriumsulfat getrocknet, filtriert und eingedampft. Die Base wurde in Alkohol gelöst und mit etherischer Salzsäure sauer gestellt. Nach Umkristallisieren aus Alkohol-Ether erhielt man 825.2 mg (R)-3-[trans-4-(3-Amino-propoxy)-cyclohexyl]-phenyl]-5-methoxymethyl-oxazolidin-2-on • HCl. Smp.: 178-180°C. $[a]_D$ =-29.78° (c=1%, in $CHCl_3$).

Beispiel **59**

(R)-3-[trans-4-[4-(5-Methoxymethyl-2-oxo-oxazolidin-3-yl)-phenyl]-cyclohexyloxy]-propionitril

4.58 g (15.0 mmol) (R)-3-[trans-(4-Hydroxycyclohexyl)-phenyl]-5-methoxymethyl-oxazolidin-2-on wurden in 400 ml Methylenchlorid gelöst und nacheinander mit 0.96 g (18.0 mmol) Acrylnitril und 2.02 g (18.0 mmol) Kalium-tert-butylat versetzt. Das Reaktionsgemisch wurde 16 Stunden bei Raumtemperatur gerührt. Danach wurde es mit Wasser versetzt, vom Unlöslichen durch Dicalit filtriert, die organische Phase mit Wasser gewaschen, über Natriumsulfat getrocknet, filtriert und eingedampft. Der Rückstand (5.1 g) wurde an 204 g Kieselgel 60 mit Essigester/n-Hexan (6:4) chromatographiert. Man erhielt nach dem Umkristallisieren aus tert-Butyl-methylether 1.6 g (R)-3-[trans-4-[4-(5-Methoxymethyl-2-oxo-oxazolidin-3-yl)-phenyl]-cyclohexyloxy]-propionitril. Smp.: 57-59°C. $[a]_D$ =-30.0° (c=0.7 in $CHCl_3$).

Beispiel **60**

(R)-3-[trans-4-(3-Amino-propoxy)-cyclohexyl]-phenyl]-5-methoxymethyl-oxazolidin-2-on-hydrochlorid

932 mg (2.6 mmol) (R)-3-[trans-4-[4-(5-Methoxymethyl-2-oxo-oxazolidin-3-yl)-phenyl]-cyclohexyloxy]-propionitril wurden in 60 ml mit trockenem Ammoniakgas gesättigten Methanol gelöst und in Gegenwart von 0.9 g Raney-Nickel (Typ B 113W Degussa) hydriert. Nach 3 Stunden wurde es vom Katalyt genutscht und das Filtrat eingedampft. Der Rückstand wurde in Methylenchlorid gelöst, mit Wasser gewaschen, über Natriumsulfat getrocknet, filtriert und eingedampft. Die Base wurde in Alkohol gelöst und mit etherischer Salzsäure sauer gestellt. Nach Umkristallisieren aus Alkohol-Ether erhielt man 825.2 mg (R)-3-[trans-4-(3-Amino-propoxy)-cyclohexyl]-phenyl]-5-methoxymethyl-oxazolidin-2-on • HCl. Smp.: 178-180°C. $[a]_D$ =-29.78° (c=1%, in $CHCl_3$).

Beispiel **61**

(R)-5-Methoxymethyl-3-[4-(4-methylencyclohexyl)-phenyl]-oxazolidin-2-on

2.0 g (7.7 mmol) [4-(4-Methylencyclohexyl)-phenyl]-ethoxycarbaminester wurden mit 1.02 g (7.7 mmol) (S)-4-Methoxymethyl-1,3-dioxolan-2-on und 170 mg Kaliumcarbonat über Nacht bei 160°C gerührt. Der Rückstand wurde in 50 ml Dichlormethan aufgenommen und mit 50 ml Wasser extrahiert. Die organische Phase wurde mit Natriumsulfat getrocknet und eingeengt. Nach Chromatographieren über Kieselgel mit Essigester/Hexan 1:99 und Umkristallisieren aus Hexan erhielt man 1.04 g (45%) (R)-5-Methoxymethyl-3-[4-(4-methylencyclohexyl)-phenyl]-oxazolidin-2-on, weisse Kristalle mit Smp. 64°C.

Beispiel **62**

(R)-3-[4-(cis oder trans-4-Hydroxy-4-methyl-cyclohexyl)-phenyl]-5-methoxymethyl-oxazolidin-2-on

Eine Lösung von 945 mg (3 mmol) Quecksilberacetat in 18 ml Wasser wurde mit 18 ml Tetrahydrofuran versetzt. Nach 30 Minuten Rühren bei Raumtemperatur wurde zu dieser Suspension eine Lösung aus 900 mg (3 mmol) (R)-5-Methoxymethyl-3-[4-(4-methylencyclohexyl)-phenyl]-oxazolidin-2-on in 18 ml Tetrahydrofuran zugetropft. Nach mehrtägigem Stehen im Kühlschrank wurden 18 ml 2N Natronlauge und nach 30 Minuten Rühren eine Lösung aus 360 mg (9.5 mmol) Natriumborhydrid in 12 ml 2N Natronlauge zugegeben. Nach 30 Minuten Rühren wurde mit Methylenchlorid extrahiert, die organische Phase mit Wasser gewaschen und mit Natriumsulfat getrocknet. Nach Abdestillieren des Lösungsmittels wurde über Kieselgel mit Essigester/Dichlormethan (1:1) chromatographiert. Man erhielt 200 mg (21%) (R)-3-[4-(cis-4-Hydroxy-4-methyl-cyclohexyl)-phenyl]-5-methoxymethyl-oxazolidin-2-on als weisse Kristalle mit Smp. 122-123°C und 500 mg cis- und trans-Mischung.

Beispiel **63**

(R)-trans-5-Methoxymethyl-3-[4-(4-cyano-cyclohexyl)-phenyl]-oxazolidin-2-on

Eine Suspension aus 500 mg (1.84 mmol) trans-[4-(4-Cyano-cyclohexyl)-phenyl]-ethoxycarbaminsäure, 365 mg (2.8 mmol) (S)-4-Methoxymethyl-1,3-dioxolan-2-on und 185 mg Kaliumcarbonat wurde 16 Stunden bei 160°C gerührt. Das Reaktionsgemisch wurde über Kieselgel mit Essigester/Hexan 1:1 chromatographiert, die erhaltenen gelben Kristalle aus Essigester umkristallisiert. Man erhielt 165 mg (29%) als weisse Kristalle mit Smp. 174°C.

Beispiel **64**

(RS)-5-Methoxymethyl-3-[4-(4-dimethylmethylen-cyclohexyl)-phenyl]-oxazolidin-2-on

1.60 g (3.3 mmol) Isopropyl-triphenylphosphoniumbromid-Natriumamidgemisch in 30 ml Tetrahydrofuran wurden 1 Stunde bei Raumtemperatur gerührt. Dann wurde eine Lösung von 1.0 g (3.3 mmol) (RS)-3-[4-(4-Oxocyclohexyl)-phenyl]-5-methoxymethyl-oxazolidin-2-on in 25 ml Tetrahydrofuran zugetropft. Nach Kochem am Rückfluss über das Wochenende wurde in 100 ml Wasser geleert und dreimal mit je 50 ml Dichlormethan extrahiert. Nach Chromatographieren über Kieselgel mit Essigester/Hexan 1:2 und Umkristallisieren aus n-Hexan erhielt man 120 mg (11%) als weisse Kristalle mit Smp. 89-91°C.

Beispiel **65**

Mischung aus (RS)- und (SR)-3-[4-[(RS)-4-Benzyliden-cyclohexyl]-phenyl]-5-methoxymethyl-oxazolidin-2-on

5.0 g (10 mmol) Benzyl-triphenylphosphoniumbromid/Natriumamidgemisch in 100 ml Tetrahydrofüran wurden 1 Stunde bei Raumtemperatur gerührt. Dann wurde eine Lösung aus 304 g (10 mmol) (RS)-3-[4-(4-Oxocyclohexyl)-phenyl]-5-methoxymethyl-oxazolidin-2-on in 150 ml Tetrahydrofuran zugetropft und 20 Stunden unter Rückfluss gekocht. Nach Abdestillieren des Lösungsmittels wurde mit 150 ml Wasser aufgenommen und viermal mit je 80 ml Dichlormethan extrahiert. Nach Chromatographieren über Kieselgel mit Essigester/Hexan 1:2 und Umkristallisieren aus Essigester/Hexan erhielt man 250 mg (7%) als weisse Kristalle mit Smp. 65-69°C.

Beispiel **66**

(RS)-3-[4-(cis- und trans-4-Benzyl-cyclohexyl)-phenyl]-5-methoxymethyl-oxazolidin-2-on

290 mg der Mutterlauge (der vorherigen Vorschrift) in 40 ml Ethanol wurden mit 100 mg 10% Palladium/Kohle versetzt und 20 Stunden bei Raumtemperatur hydriert. Nach Chromatographieren über Kieselgel mit Essigester/Hexan 1:2 erhielt man 110 mg Harz. MS: m/e (% Basispeak): 379 ($M^+$, 100), 246 (23), 144 (31), 118 (26), 91 (55), 71 (26), 45 (28).

Beispiel **67**

Mischung aus (RS)- und (SR)-[4-[4-[(RS)-5-Methoxymethyl-2-oxo-oxazolidin-3-yl]-phenyl]-cyclohexyliden]-essigsäuremethylester

10.0 g (33 mmol) (RS)-3-[4-(4-Oxocyclohexyl)-phenyl]-5-methoxymethyl-oxazolidin-2-on und 11.0 g (33 mmol) Methoxycarbonylmethylen-triphenylphosphoran in 500 ml Benzol wurden während 24 Stunden unter Rückfluss gekocht. Nach Abdestillieren des Lösungsmittels im Vakuum chromatographierte man über Kieselgel mit Essigester/Hexan 1:1. Man erhielt 3.6 g (43%) weisse Kristalle mit Smp. 62-64°C.

Beispiel **68**

(RS)-3-(4-Cyclohexyl-3-nitro-phenyl)-5-methoxymethyl-oxazolidin-2-on

2.44 g (8.4 mmol) (4-Cyclohexyl-3-nitrophenyl)-ethoxycarbaminester und 1.10 g (8.4 mmol) (RS)-4-Methoxymethyl-1,3-dioxolan-2-on und 200 mg Kaliumcarbonat wurden 1 Tag lang bei 160°C gerührt. Nach Chromatographieren über Kieselgel mit Essigester/Hexan 2:3 und Umkristallisieren aus Essigester/Ether erhielt man 0.94 g (34%) beige Kristalle mit Smp. 105-106°C.

Beispiel **69**

(RS)-3-(3-Amino-4-cyclohexyl-phenyl)-5-methoxymethyl-oxazolidin-2-on

0.5 g (1.5 mmol) (RS)-3-(4-Cyclohexyl-3-nitrophenyl)-5-methoxymethyl-oxazolidin-2-on (aus der Mutterlauge der vorherigen Reaktion) in 75 ml Ethanol wurden mit 100 mg (10 proz. Palladium auf Kohle versetzt und während 5 Stunden bei Raumtemperatur hydriert. Filtrieren, Einengen und Umkristallisieren aus Essigester ergab 150 mg weisse Kristalle mit Smp. 130-132°C.

Beispiel **70**

(RS)-3-(4-Cyclohexyl-3-jod-phenyl)-5-methoxymethyl-oxazolidin-2-on

180 mg (0.6 mmol) (RS)-3-(3-Amino-4-cyclohexyl-phenyl)-5-methoxymethyl-oxazolidin-2-on (aus der Mutterlauge der vorherigen Reaktion) wurden in einer Mischung aus 0.32 ml Wasser, 0.32 g 37 proz. Salzsäure und 0.64 g Eis suspendiert und bei 0-5°C mit einer Lösung aus 41.4 mg (0.6 mmol) Natriumnitrit in 0.3 ml Wasser versetzt. Nach 30 Minuten Rühren bei dieser Temperatur wurde eine Lösung aus 0.1 g (0.6 mmol) Kaliumjodid in 0.3 ml Wasser zugegeben. Man rührte über Nacht bei Raumtemperatur, extrahierte mit Essigester und chromatographierte über Kieselgel mit Essigester/Hexan 3:1 und erhielt 100 mg Oel. MS: m/e (% Basispeak): 415 ($M^+$, 100), 372 (5), 346 (9), 289 (9) 245 (12), 143 (15), 71 (22).

Beispiel **71**

(RS)-3-(4-Cyclohexyl-3-hydroxy-phenyl)-5-methoxymethyl-oxazolidin-2-on

230 mg (0.7 mmol) (RS)-3-(3-Amino-4-cyclohexyl-phenyl)-5-methoxymethyl-oxazolidin-2-on wurden in einer Mischung aus 0.86 ml Wasser, 0.72 g Eis und 0.235 ml konz. Schwefelsäure suspendiert und bei 0-5°C mit einer Lösung aus 45 mg (0.66 mmol) Natriumnitrit in 0.4 ml Wasser versetzt. Nach 3 Stunden Kochen unter Rückfluss extrahierte man mit Dichlormethan, trocknete mit Natriumsulfat und destillierte das Lösungsmittel ab. Chromatographieren über Kieselgel mit Essigester/Hexan 1:2 ergab 120 mg (60%) weisse Kristalle mit Smp. 207-208°C.

Beispiel **72**

(RS)-3-(3-Chlor-4-cyclohexyl-phenyl)-5-methoxymethyl-oxazolidin-2-on

0.45 g (1.6 mmol) 3-Chlor-4-cyclohexylphenyl)-ethoxycarbaminester und 0.84 g (6.4 mmol) (RS)-4-Methoxymethyl-1,3-dioxolan-2-on und 200 mg Kaliumcarbonat wurden 24 Stunden bei 160°C gerührt. Chromatographieren über Kieselgel mit Dichlormethan lieferte 0.57 g Oel, das noch Dioxolanon enthielt. Dies wurde durch Kurzwegdestillation bei 100°C/0.2 mbar entfernt. Es verblieben 0.38 g (73%) Harz. MS: m/e (% Basispeak) 323 ($M^+$, 100), 280 (13), 254 (13), 178 (25), 152 (18), 71 (36).

Herstellung der Zwischenprodukte

Beispiel **73**

(RS)-3-(4-Cyclohexyl-phenylamino)-propan-1,2-diol

8.05 g (44.6 mmol) 4-Cyclohexylanilin, 12.0 g (57 mmol) Methansulfonsäure(RS)-2,2-dimethyl-1,3-dioxolan-4-yl-methylester und 11.2 ml (80 mmol) Triethylamin wurden während 4h im Bombenrohr bei 140° gehalten. Das Lösungsmittel wurde im Wasserstrahlvakuum abdestilliert, der braune ölige Rückstand mit 50 ml 3 N Salzsäure versetzt und während einer Stunde bei 50° gerührt. Nach dem Abkühlen wurde 2 mal mit je 250 ml Ether extrahiert, die wässrige Phase mit 3 N Natronlauge alkalisch gestellt und das Produkt in Essigester aufgenommen. Die organische Phase wurde mit Wasser gewaschen, über Magnesiumsulfat getrocknet und das Lösungsmittel abdestilliert.. Der ölige Rückstand kristallisierte beim Versetzen mit Essigester/Hexan. Erhalten wurden 2.2 g kristallines (RS)-3-(4-Cyclohexyl-phenylamino)-propan-1,2-diol. Smp.: 121-122° (Zers).
Aus der Mutterlauge wurden weitere 0.65 g kristallines Produkt erhalten.

Beispiel **74**

a) (S)-3-(4-Cyclohexyl-phenylamino-methyl)-4-(2,2-dimethyl-1,3-dioxolan)

5.0 g (28,5 mmol) 4-Cyclohexylanilin, 10.0 g (34,9 mmol) (R)-2,2-Dimethyl-1,3-dioxolan-4-methanol-toluol-4-sulfonat und 10 ml Triethylamin wurden während 3 Stunden bei Oelbadtemperatur von 140° gerührt. Das nach Abdestillieren der leichtflüchtigen Anteile im Wasserstrahlvakuum erhaltene braune Oel wurde mit gesättigter Natriumkarbonatlösung versetzt und mit Essigester extrahiert. Die organische Phase wurde mit Kochsalzlösung gewaschen, über Magnesiumsulfat getrocknet und das Lösungsmittel abdestilliert. Es resultierten 9.5 g eines braunen Oels, das an der 30-fachen Menge Kieselgel chromatographiert wurde. Die nach Elution mit Essigester-Hexan (1:3) im DC (Essigester-Hexan 1:2) einheitlichen Fraktionen wurden zusammengefasst und das Lösungsmittel abdestilliert. Erhalten wurden 9.4 g eines gelbliches Oels. Sdp.: 140°/0,01 mbar. $[a]_D$ =+1.43 (c=1, $CHCl_3$).

b) (S)-3-(4-Cyclohexyl-phenylamino)-propan-1,2-diol

4.5 g (S)-3-(4-Cyclohexyl-phenylamino-methyl)-4-(2,2-dimethyl-1,3-dioxolan) wurden mit 50 ml 3 N Salzsäure versetzt und während einer Stunde bei 50° gerührt. Nach dem Abkühlen wurde mit 3 N Natronlauge unter Eiskühlung alkalisch gestellt, das ausgefallene Oel in Essigester aufgenommen, mit Kochsalzlösung gewaschen, über Magnesiumsulfat getrocknet und das Lösungsmittel abdestilliert. Das erhaltene hellbraune Oel (4.1 g) wurde an der 30-fachen Menge Kieselgel chromatographiert. Die Methylenfraktionen wurden verworfen und die Essigester-Methylenchlorid-(1:1)-Fraktionen zusammengefasst. Es resultierten 1.5 g Produkt in Form beiger Kristalle. Smp.: 116°. $[a]_D$ =-12.5°, (c=0.6,$CHCl_3$).

Beispiel **75**

a) (R)-3-(4-Cyclohexyl-phenylamino-methyl)-4-(2,2-dimethyl-1,3-dioxolan)

10.0 g (57 mmol) 4-Cyclohexylanilin wurden mit 20.0 g (70 mmol) (S)-2,2-Dimethyl-1,3-dioxolan-4-methanol-toluol-4-sulfonat wie in Beispiel 42 a) angegeben umgesetzt und aufgearbeitet. Erhalten wurden 11.7 g (71%) gelbliches Oel. $[a]_D$ =-2.4 (c=1.0,$CHCl_3$).

b) (R)-3-(4-Cyclohexyl-phenylamino)-propan-1,2-diol

11.5 g (R)-3-(4-Cyclohexyl-phenylamino-methyl)-4-(2,2-dimethyl-1,3-dioxolan) wurden mit 100 ml 3 N Salzsäure wie in Beispiel 42 b) angegeben umgesetzt und aufgearbeitet. Es resultierten 6.25 g Produkt in Form beiger Kristalle. Smp.: 118-120°. $[a]_D$ =-12.75, (c=0.4, $CHCl_3$).

Beispiel **76**

[4-(4-oxo-Cyclohexyl)-phenyl]-ethoxycarbaminester

10.0 g (52.8 mmol) 4-(4-Aminophenyl)-cyclohexanon wurden in 150 ml Tetrahydrofuran gelöst. Nach Zugabe von 10 ml Wasser und 6.65 g (79.2 mmol) Natriumbicarbonat wurde während 15 Minuten intensiv gerührt und anschliessend eine Lösung von 5.5 ml Chlorameisensäureethylester (58.1 mmol) in 25 ml Tetrahydrofuran innert 20 Minuten zugetropft, wobei die Temperatur 20° nicht übersteigen sollte. Nach 2 Stunden wurde der ausgefallene Niederschlag abgesaugt und das Lösungsmittel im Wasserstrahlvakuum abdestilliert. Der ölige Rückstand wurde mit Wasser versetzt und das Reaktionsprodukt mit Essigester extrahiert. Die organische Phase wurde mit Wasser gewaschen, über Magnesiumsulfat getrocknet und das Lösungsmittel abdestilliert. Es resultierten 13.8 g (100%) Carbamat. Smp.: 160-162°.

Beispiel **77**

a) 1-(4-Aminophenyl)-4-piperidinon

5.6 g 1-(4-Nitrophenyl)-4-piperidon wurden unter Argon in 60 ml Dioxan suspendiert, mit 2.6 ml Triethylamin und 0.6 g Palladium auf Kohlenstoff (5%) versetzt und bei Raumtemperatur und Normaldruck hydriert. Filtrieren und Einengen lieferte 5.0 g 1-(4-Aminophenyl)-4-piperidinon.

b) 4-(4-Oxo-piperidin-1-yl)-phenylcarbamidsäureethylester

4.9 g 1-(4-Aminophenyl)-4-piperidinon wurden unter Argon in einem Gemisch aus 30 ml Tetrahydrofüran und 9 ml Wasser suspendiert und mit 3.3 g Natriumhydrogencarbonat versetzt. Dann wurde eine Lösung von 2.7 ml Chlorameisensäureethylester in 20 ml Tetrahydrofuran unter heftigem Rühren bei Raumtemperatur zugetropft und nach beendeter Zugabe noch 1 h gerührt. Nach Zugabe von Wasser und Essigsäureethylester wurden die Phasen getrennt und die wässrige Phase mit Essigsäureethylester extrahiert. Die organischen Phasen wurden vereinigt, mit Magnesiumsulfat getrocknet und eingeengt. Der Rückstand (6.6 g) wurde über Kieselgel 60 mit Essigsäureethylester/Hexan-Gemischen (1:2-1:0) chromatographiert. Kristallisation aus Diethylether lieferte 3.8 g 4-(4-Oxo-piperidin-1-yl)-phenylcarbamidsäureethylester als graue Kristalle. Smp.: 145-146°.

Beispiel **78**

a) (RS)- 3-(5-Brom-pyridin-2-yl)-5-methoxy-methyl-oxazolidin-2-on

Ein Gemisch aus 9.3 g 5-Brom-pyridin-2-yl-carbamidsäureethylester, 7.5 g 4-Methoxymethyl-1,3-dioxolan-2-on, 1.0 g Kaliumcarbonat und 10 ml Decahydronaphthalin wurde 6 h auf 160°C erhitzt. Das Gemisch wurde abgekühlt und mit Essigsäureethylester und Wasser versetzt. Die Phasen wurden getrennt, die wässrige mit Essigsäureethylester extrahiert und die organischen mit gesättigter Natriumchloridlösung gewaschen. Die organischen Phasen wurden vereinigt und mit Magnesiumsulfat getrocknet und dann eingeengt. Chromatographie des Rückstandes (20.2 g) mit Essigsäureethylester/Hexan-Gemischen (1:2-1:1) an Kieselgel lieferte 6.4 g (RS)-3-(5-Brom-pyridin-2-yl)-5-methoxy-methyl-oxazolidin-2-on, das aus Diethylether umkristallisiert wurde. Smp.: 90-92°.

b) (RS)-3-[5-(1,4-Dioxa-spiro[4,5]dec-7-en-8-yl)-pyridin-2-yl]-5-methoxymethyl-oxazolidin-2-on

Unter Argon wurde eine Suspension aus 1.4 g 3-(5-Brom-pyridin-2-yl)-5-methoxymethyl-oxazolidin-2-on, 1.4 g 8-Trimethylstannyl-1,4-dioxaspiro[4,5]dec-7-en und 0.3 Bis(triphenylphosphin)palladium(II)dichlorid in 20 ml Tetrahydrofuran 18 h bei 70°C gerührt. Dann wurde abgekühlt, mit 150 ml Essigsäureethylester und 150 ml gesättigter Kaliumfluoridlösung versetzt und 30 Minuten gerührt. Das Gemisch wurde über ein Celite®-Polster filtriert, die Phasen getrennt und die wässrige mit Essigsäureethylester extrahiert. Die organischen Phasen wurden vereinigt, mit Magnesiumsulfat getrocknet, eingeengt und mit Diethylether als Laufmittel über Kieselgel 60 chromatographiert. Man erhielt 0.6 g (RS)-3-[5-(1,4-Dioxa-spiro[4,5]dec-7-en-8-yl)-pyridin-2-yl]-5-methoxymethyl-oxazolidin-2-on. $^1$H-NMR($CDCl_3$) ppm: 8.41 (s, 1H), 8.03 (d, 1H), 7.89 (d, 1H), 6.08 (t, 1H), 4.82 (m, 1H), 4.21 (t, 1H), 3.91 (t, 1H), 3.92 (s, 4H), 3.60 (m, 2H), 3.32 (s, 3H), 2.54 (m, 2H), 2.38 (m, 2H), 1.82 (t, 2H).

Beispiel **79**

a) Trifluormethansulfonsäure-1,4-dioxa-spiro[4,5]dec-7-en-8-yl-ester

Aus 48 ml Diisopropylamin und 210 ml einer 1.6 M Lösung von n-Butyl-lithium in Hexan wurde in 1 l absolutem Tetrahydrofuran unter Argon eine Lithiumdiisopropylamid-Lösung hergestellt. Zu dieser Lösung wurde bei -70°C eine Lösung von 50 g 1,4-Cyclohexandion-monoethylenketal in 200 ml Tetrahydrofuran getropft. Nach Entfernen der Kühlung kam das Gemisch auf -20°C, wurde dann erneut auf -70°C abgekühlt und schnell mit 120 g N-Phenyl-bis(trifluormethansulfonimid) in 500 ml Tetrahydrofüran versetzt. Nach Entfernen der Kühlung kam das Gemisch auf Raumtemperatur und wurde eingeengt. Der Rückstand (215 g) wurde mit Essigsäureethylester/Hexan (1:9) über Kieselgel 60 filtriert. Man erhielt in quantitativer Ausbeute Trifluormethansulfonsäure-1-4-dioxa-spiro[4,5]dec -7-en-8-yl-ester. $^1$H-NMR($CDCl_3$ ) ppm: 5.66 (m, 1H), 3.99 (s, 4H), 2.53 (m, 2H), 2.42 (m, 2H), 1.90 (t, 2H).

b) 8-Tributyl-stannyl-1,4-dioxa-spiro[4,5]dec-7-en

Aus 13 ml Diisopropylamin und 59 ml einer 1.6 M Lösung von n-Butyl-lithium in Hexan wurde in 400 ml absolutem Tetrahydrofuran unter Argon eine Lithiumdiisopropylamidlösung hergestellt. Bei -70°C wurden zu dieser Lösung 21 ml Tributylzinnhydrid getropft und 2 h gerührt. Nach Zugabe von 3.5 g Kupfer(I)cyanid liess man das Gemisch auf -50°C aufwärmen und tropfte schnell eine Lösung von 10.4 g Trifluormethansulfonsäure-1,4-dioxaspiro[4,5]dec-7-en-8-yl-ester in 80 ml Tetrahydrofuran zu. Die Reaktionslösung färbte sich während 2 h Rühren bei -30°C langsam rot. Die Kühlung wurde entfernt und das Gemisch mit gesättigter Ammoniumchloridlösung und Hexan versetzt. Nach Filtration über ein Celite® -Polster wurde die wässrige Phase abgetrennt und die organische nacheinander mit Wasser und gesättigter Natriumchloridlösung gewaschen. Die organische Phase wurde mit Magnesiumsulfat getrocknet und eingeengt. Der Rückstand wurde in 300 ml Essigsäureethylester aufgenommen, mit 18 g Silberacetat versetzt und an der Luft gerührt. Das Gemisch wurde über ein Celite® -Polster filtriert und das Filtrat mit Wasser und gesättigter Natriumchloridlösung gewaschen. Die organische Phase wurde mit Magnesiumsulfat getrocknet, eingeengt und über Kieselgel 60 (desaktiviert mit Triethylamin) chromatographiert. Man erhielt 8.9 g 8-Tributyl-stannyl-1,4-dioxa-spiro[4,5]dec-7-en. $^1$H-NMR($CDCl_3$) ppm: 5.70 (m, 1H), 3.98 (s, 4H), 2.36 (bm, 4H), 1.74 (t, 2H), 1.45 (m, 6H), 1.30 (m, 6H), 0.88 (m, 9H).

Beispiel **80**

a) (RS) -5-Methoxymethyl-3-pyridin-3-yl-oxazolidin-2-on

Ein Gemisch aus 16.8 g Pyridin-3-yl- carbamidsäureethylester, 20 g 4-Methoxymethyl-1,3-dioxolan-2-on und 2.8 g Kaliumcarbonat wurde 3 h auf 160°C erhitzt. Das Gemisch wurde abgekühlt und mit Essigsäureethylester und Wasser versetzt. Die Phasen wurden getrennt, die wässrige mit Essigsäureethylester extrahiert und die organischen mit gesättigter Natriumchloridlösung gewaschen. Die organischen Phasen wurden vereinigt, mit Magnesiumsulfat getrocknet und eingeengt. Chromatographie des Rückstandes (21 g) mit Essigsäureethylester/Methanol-Gemischen (1:0-20:1) an Kieselgel lieferte 17,6 g (RS)-5-Methoxymethyl-3-pyridin-3-yl-oxazolidin-2-on, das aus Diethylether umkristallisiert wurde. Smp.: 65-66°.

b) (RS)-5-Methoxymethyl-3-(1-oxy-pyridin-3-yl)-oxazolidin-2-on

Unter Argon wurden zu einer Lösung von 16.5 g (RS)-5-Methoxymethyl-3-pyridin-3-yl-oxazolidin-2-on in 250 ml Methylenchlorid 33.4 g 3-Chlorperbenzoesäure gegeben und das Gemisch 4 h bei Raumtemperatur gerührt. Anschliessend wurde das Gemisch mit Wasser gewaschen und die wässrige Phase mit Methylenchlorid extrahiert. Die organischen Phasen wurden vereinigt, mit Magnesiumsulfat getrocknet, eingeengt und mit Methylenchlorid/Methanol-Gemischen (50:1-20:1) über Kieselgel chromatographiert. Das Produkt, 15.9 g (RS)-5-Methoxymethyl-3-(1-oxy-pyridin-3-yl)-oxazolidin-2-on, wurde zur weiteren Reinigung mit Diethylether aufgekocht. Smp.: 82-83°.

c) (RS)-3-(6-Brom-1-oxy-pyridin-3-yl)-5-methoxymethyl-oxazolidin-2-on

Bei Raumtemperatur wurden zu einer Lösung von 25.5 g (RS)-5-Methoxymethyl-3-(1-oxy-pyridin-3-yl)-oxazolidin-2-on in 300 ml Methylenchlorid unter Argon 5.9 ml Brom, gelöst in 35 ml Methylenchlorid, getropft. Das Gemisch wurde noch 3 h gerührt, dann mit 350 ml Natriumhydrogencarbonat versetzt und schliesslich die Phasen getrennt. Die wässrige Phase wurde noch mehrmals mit Methylenchlorid extrahiert. Die organischen Phasen wurden vereinigt, mit Magnesiumsulfat getrocknet, eingeengt und mit Methylenchlorid/Methanol- Gemischen (50:1-20:1) über Kieselgel 60 chromatographiert. Die Chromatographie lieferte 22.5 g (RS)-3-(6-Brom-1-oxy-pyridin-3-yl)-5-methoxymethyl-oxazolidin-2-on. $^1$H-NMR($CDCl_3$) ppm: 8.54 (s, 1H), 7.74 (d,1H), 7.59 (d, 1H), 4.82 (m, 1H), 4.00 (t, 1H), 3.90 (t, 1H), 3.66 (m, 2H), 3.43 (s, 3H).

d) (RS) -3-(6-Brom-pyridin-3-yl)-5-methoxymethyl-oxazolidin-2-on

Zu einer siedenden Lösung von 22.5 g (RS)-3-(6-Brom-1-oxy-pyridin-3-yl)-5-methoxymethyl-oxazolidin-2-on in Methylenchlorid unter Argon wurden 10.5 ml Phosphortribromid getropft und das Gemisch über Nacht gekocht. Anschliessend wurde abgekühlt und unter heftigem Rühren auf 300 ml 2 N Natronlauge gegossen. Die wässrige Phase wurde mehrfach mit Methylenchlorid extrahiert, die organischen Phasen vereinigt, mit Magnesiumsulfat getrocknet und eingeengt. Der Rückstand (11.3 g) wurde mit Methylenchlorid als Laufmittel über Kieselgel 60 chromatographiert. Die Chromatographie lieferte 9.9 g (RS)-3-(6-Brom-pyridin-3-yl)-5-methoxymethyl-oxazolidin-2-on, das zur weiteren Reinigung mit Diethylether aufgekocht wurde. Smp.: 108-110°.

Beispiel **81**

(RS)-3-[6-(1,4-Dioxa-spiro[4,5]dec-7-en-8-yl)pyridin-3-yl]-5-methoxymethyl-oxazolidin-2-on

Unter Argon wurde eine Suspension aus 8.9 g (RS)-3-(6-Brom-pyridin-3-yl)-5-methoxymethyl-oxazolidin-2-on, 6,5 g 8-Tributyl-stannyl-1,4-dioxaspiro[4,5]dec-7-en und 1.4 g Bis(triphenylphosphin)palladium(II)dichlorid in 40 ml Dimethylformamid 22 h bei 85°C gerührt. Dann wurde abgekühlt, das Gemisch über ein Celite®- Polster filtriert, der Filterkuchen mit viel Essigsäureethylester gewaschen und die organische Phase mehrfach mit halbgesättigter Natriumchloridlösung gewaschen. Die wässrigen Phasen wurden mit Essigsäureethylester extrahiert, die organischen vereinigt, auf ein Volumen von 250 ml eingeengt, mit 250 ml gesättigter Kaliumfluoridlösung versetzt und 36 Minuten

gerührt. Das Gemisch wurde erneut über ein Celite®-Polster filtriert, die Phasen getrennt und die wässrige Phase mit Essigsäureethylester extrahiert. Die organischen Phasen wurden vereinigt, mit Magnesiumsulfat getrocknet, eingeengt und mit Diethylether als Laufmittel über Kieselgel 66 chromatographiert. Man erhielt 1.2 g (RS)-3-[6-(1,4-Dioxa-spiro[4,5]dec-7-en-8-yl)-pyridin-3-yl]-5-methoxymethyl-oxazolidin-2-on. $^1H$-NMR($CDCl_3$) ppm: 8.48 (s, 1H), 8.19 (d, 1H), 7.56 (d,1H), 6.50 (m, 1H), 4.79 (m, 1H), 4.12 (t, 1H), 4.02 (s, 4H), 3.98 (t, 1H), 3.64 (m, 2H), 3.44 (s, 3H), 2.80 (m, 2H), 2.52 (m, 2H), 1.96 (t, 2H).

Beispiel **82**

4-Cycloheptylphenyl-carbamidsäureethylester

6.81 g (36.0 mmol) 4-Aminophenyl-cycloheptan wurden in 60 ml Methylenchlorid gelöst und mit 3.13 g (39.6 mmol) Pyridin versetzt. Das Reaktionsgemisch wurde während 10 Minuten mit einer Lösung von 4.3 g (39.6 mmol) Chlorameisensäureethylester in 15 ml Methylenchlorid tropfenweise unter Rühren bei Raumtemperatur versetzt. Nach 2 Stunden wurde mit Wasser gewaschen, die organische Phase über Natriumsulfat getrocknet, filtriert und eingedampft. Der 4-Cycloheptylphenyl-carbamidsäureethylester destillierte bei 165-170°C und 0.3 mbar.

Beispiel **83**

4-(Adamantan-1-yl)-phenyl-carbamidsäureethylester

5.28 g (20 mmol) 4-Adamantan-1-yl-phenylamin Hydrochlorid wurden in 100 ml Methylenchlorid vorgelegt und mit 3.22 g (42.0 mmol) Pyridin versetzt. Zu dieser Suspension tropfte man unter Rühren bei Zimmertemperatur während 15 Minuten eine Lösung von 2.39 g (22.0 mmol) Chlorameisensäureethylester in 15 ml Methylenchlorid. Das Reaktionsgemisch wurde 2 Stunden bei Raumtemperatur gerührt. Anschliessend wurde mit Wasser gewaschen, die organische Phase über Natriumsulfat getrocknet, filtriert und eingedampft. Der Rückstand wurde aus Methylenchlorid/n-Hexan umkristallisiert. Man erhielt 5.6 g 4-Adamantan-1-yl-phenyl-carbamidsäureethylester als weisses Kristallisat. Smp.: 132-134°.

Beispiel **84**

Mischung aus (R)- und (S)-[4-[4-[(R)-5-Methoxymethyl-2-oxo-oxazolidin-3-yl]-phenyl]-cyclohexyliden]-acetonitril

0.66 g Natrium wurden unter Argon in 70 ml Ethanol gelöst. Danach fügte man bei 20°C 5.08 g (= 4.51 ml) Diethylcyanomethylphosphonat in 20 ml Ethanol gelöst zu. Man rührte 36 Minuten bei Raumtemperatur nach und fügte dann 2.9 g (R)-5-Methoxymethyl-3-[4-(4-oxo-cyclohexyl)-phenyl]-oxazolidin-2-on zu. Man rührte danach noch 1 Stunde am Rückfluss, kühlte auf Raumtemperatur, stellte mit ca. 1 ml Eisessig auf pH 6 und dampfte das Reaktionsgemisch im Vakuum ein. Der Rückstand wurde in Dichlormethan gelöst und dreimal mit Wasser gewaschen, anschliessend getrocknet und eingedampft. Der überschüssige Phosphorester, welcher im Rückstand noch vorhanden war, wurde bei einer Badtemperatur von ~100°C und 0.03 mm Vakuum abdestilliert ($Kp_{0.03}$ 34°C). Der verbleibende Rückstand (3.2 g) wurde über 250 g Silicagel Mitteldruck chromatographiert. Eluiert wurde mit Dichlormethan, Dichlormethan/Ethylacetat 20:1. Die nach Dünnschichtchromatogramm reinen Fraktionen wurden vereinigt und im Vakuum eingeengt. Man erhielt 2.6 g einer Mischung aus (R)- und (S)-[4-[4-[(R)5-Methoxymethyl-2-oxo-oxazolidin-3-yl]-phenyl]-cyclohexyliden]-acetonitril (~1:2 E/Z oder Z/E) als leicht trübes Oel. Dieses wurde direkt zur Reduktion eingesetzt.

Beispiel **85**

4-(4-Nitrophenyl)-piperidin-1-carbonsäure-t-butylester

7.63 g (37.0 mmol) 4-(4-Nitrophenyl)piperidin und 8.88 g (40.7 mmol) Di-tert-butyldicarbonat wurden in 150 ml Chloroform während 4 Stunden bei Raumtemperatur gerührt. Danach wurde mit Wasser gewaschen, die organische Phase über Natriumsulfat getrocknet, filtriert und eingedampft. Man erhielt nach Umkristallisieren aus n-Hexan 11.2 g 4-(4-Nitrophenyl)-piperidin-1-carbonsäure-t-butylester. Smp.: 60-62°C.

Beispiel **86**

4-(4-Aminophenyl)-piperidin-1-carbonsäure-t-butylester

11.03 g (36.0 mmol) 4-(4-Nitrophenyl)-piperidin-1-carbonsäure-t-butylester wurden in 200 ml Alkohol gelöst und in Gegenwart von 1 g Palladium auf Kohlenstoff (10%) bei Raumtemperatur und Normaldruck hydriert. Nach Abfiltrieren des Katalysators wurde das Filtrat eingeengt und der Rückstand (10.0 g) in Methylenchlorid mit Wasser gewaschen. Man erhielt nach Umkristallisieren aus Ether/n-Hexan 8.0 g 4-(4-Aminophenyl)-piperidin-2-carbonsäure-t-butylester. Smp.: 114-116°C.

Beispiel **87**

4-(4-Ethoxycarbonylamino-phenyl)-piperidin-1-carbonsäure-t-butylester

8.84 g (32.0 mmol) 4-(4-Aminophenyl)-piperidin-1-carbonsäure-t-butylester wurden in 150 ml Methylenchlorid gelöst und in Gegenwart von 2.78 g (35.2 mmol) Pyridin mit 3.82 g (35.2 mmol) Chlorameisensäureethylester versetzt. Das Reaktionsgemisch wurde 16 Stunden bei Raumtemperatur gerührt. Danach wurde es mit Wasser gewaschen, die organische Phase über Natriumsulfat getrocknet, filtriert und eingedampft. Der Rückstand (11.3 g) wurde an 95 g Kieselgel 60 mit Ether/n-Hexan (4:6) chromatographiert. Man erhielt nach Umkristallisieren aus Ether/n-Hexan 6.15 g 4-(4-Ethoxycarbonylamino-phenyl)-piperidin-1-carbonsäure-t-butylester. Smp.: 126-128°C.

Beispiel **88**

4-(4-Ethoxycarbonylamino-phenyl)-piperidin-1-carbonsäureethylester

3.01 g (14.6 mmol) 4-(4-Aminophenyl)piperidin wurden in 100 ml Methylenchlorid gelöst und in Gegenwart von 11.55 g (146 mmol) Pyridin mit 1.74 g (16.06 mmol) Chlorameisensäureethylester unter Rühren versetzt. Nach 3 Stunden wurde es eingedampft, der Rückstand auf Essigester/Wasser verteilt, die organische Phase mit Wasser gewaschen, über Natriumsulfat getrocknet, filtriert und eingedampft. Der Rückstand (2.7 g) wurde an 85 g Kieselgel 60 mit Methylenchlorid/Methanol (98:2) chromatographiert. Man erhielt nach Umkristallisieren aus Ether/n-Hexan 0.9 g 4-(4-Ethoxycarbonylamino-phenyl)-piperidin-1-carbonsäure-ethylester. Smp.: 105-107°C.

Beispiel **89**

4-[4-(Methylen-cyclohexyl)-phenyl]-ethoxycarbaminester

Eine Suspension aus 15.95 g Methyltripheny]phosphoniumbromid/Natriumamidgemisch (38.3 mmol Methyltriphenylphosphoniumbromid) in 300 ml wurde 1 Stunde bei Raumtemperatur gerührt. Dann gab man eine Lösung aus 10.0 g (38.3 mmol) [4-(4-Oxo-cyclohexyl)-phenyl]-ethoxycarbaminester in 120 ml Tetrahydrofuran und kochte über Nacht unter Rückfluss. Das Lösungsmittel wurde abdestilliert und der Rückstand über Kieselgel mit Essigester/Hexan 1:3 chromatographiert. Es ergaben sich 6.1 g (61%) [4-(4-Methylen-cyclohexyl)-phenyl]-ethoxycarbaminester als weisse Kristalle mit Smp. 98°C.

Beispiel **90**

cis- und trans-4-(4-Cyano-cyclohexyl)-anilin

Zu einer Suspension von 278 mg (1.5 mmol) 4-(4-Oxo-cyclohexyl)-anilin und 370 mg (1.9 mmol) Toluol-4-sulfonylmethylisocyanid in 5 ml 1,2-Dimethoxyethan und 0.15 ml Ethanol wurden bei -10°C portionsweise 392 mg (3.5 mmol) Kalium-t-butylat zugegeben. Nach 1 Stunde Rühren bei -5°C und weiteren 70 Stunden bei Raumtemperatur fügte man 25 ml Wasser und 10 ml gesättigte Kochsalzlösung zu und extrahierte dreimal mit je 40 ml Dichlormethan. Die organische Phase wurde mit Natriumsulfat getrocknet und eingeengt. Chromatographieren über Kieselgel mit Essigester/Hexan 1:2 ergab 165 mg (56%) 4-(4-Cyano-cyclohexyl)-anilin mit Smp. 132-140°C.

Beispiel **91**

cis-[4-(4-Cyano-cyclohexyl)-phenyl]ethoxycarbaminester und trans-[4-(4-Cyano-cyclohexyl)-phenyl]ethoxycarbaminester

Zu einer Lösung von 2.75 g (14 mmol) cis- und trans-4-(4-Cyano-cyclohexyl)-anilin in 50 ml Tetrahydrofuran und 4 ml Wasser fügte man 1.74 g (21 mmol) Natriumhydrogencarbonat und nach 15 Minuten 1.46 ml (15.3 mmol) Chlorameisensäureethylester, gelöst in 5 ml Tetrahydrofuran. Nach 4 Tagen Rühren bei Raumtemperatur destillierte man das Lösungsmittel ab, suspendierte den Rückstand in 100 ml Dichlormethan und extrahierte mit Wasser und gesättigter Kochsalzlösung. Die organische Phase wurde mit Natriumsulfat getrocknet und eingeengt. Chromatographieren des Rückstandes über Kieselgel mit Essigester/Hexan 1:2 lieferte 630 mg (17%) trans-[4-(4-Cyano-cyclohexyl)-phenyl]ethoxycarbaminester als weisse Kristalle mit Smp. 145°C und 80 mg (2%) cis-[4-(4-Cyano-cyclohexyl)-phenyl]-ethoxycarbaminester als weisse Kristalle mit Smp. 110°C, sowie 2.5 g (67%) cis-trans-Mischfraktion.

Beispiel **92**

4-Cyclohexyl-3-nitrophenyl-ethoxycarbaminester

Eine Mischung aus 1.97 g (8.9 mmol) 4-Cyclohexyl-3-nitroanilin und 1.13 g (13.4 mmol) Natriumhydrogencarbonat in 25 ml Tetrahydrofüran und 1.7 ml Wasser wurde mit 1.07 g (9.9 mmol) Chlorameisensäureethylester, gelöst in 5 ml Tetrahydrofuran, versetzt. Nach Rühren über Nacht bei Raumtemperatur wurde das Lösungsmittel abdestilliert und der Rückstand über Kieselgel mit Essigester/Hexan chromatographiert. Man erhielt 2.44 g (93%) gelbes Oel. MS: m/e (% Basispeak): 292 ($M^+$, 7), 275 (100), 247 (14), 229 (27), 201 (18), 41 (26).

Beispiel **93**

3-Chlor-4-cyclohexylanilin

Ein Gemisch aus 1.0 g (5 mmol) 4-Cyclohexyl-nitrobenzol und 0.1 g Eisen(III)chlorid wurde auf 60°C erhitzt. Nach Schmelzen des Cyclohexylnitrobenzols leitete man während 15 Minuten Chlorgas über das gut gerührte Gemisch. Dann gab man 20 ml Eiswasser zu und extrahierte mit Ether. Der Ether wurde abdestilliert und der Rückstand in 60 ml Methanol gelöst. Dann versetzte man mit 2.5 g (25 mmol) Kupfer(I)chlorid und portionsweise mit 1.6 g (29 mmol) Kaliumborhydrid. Nach 36 Minuten Rühren wurde in 0.5 l Wasser geleert und mit Ether extrahiert. Trocknen über Natriumsulfat, Abdestillieren des Ethers und Chromatographieren über Kieselgel mit Hexan/Essigester 9:1 ergab 6.68 g Oel. MS: m/e (% Basispeak) = 209 ($M^+$, 75), 166 (100), 140 (78), 131 (62).

Beispiel **94**

(3-Chlor-4-cyclohexylphenyl)-ethoxycarbaminester

Eine Mischung aus 0.59 g (2.8 mmol) 3-Chlor-4-cyclohexyl-anilin und 0.36 g (4.2 mmol) Natriumhydrogencarbonat in 10 ml Tetrahydrofuran und 0.75 ml Wasser wurde mit 0.34 g (3.1 mmol) Chlorameisensäureethylester, gelöst in 1 ml Tetrahydrofuran, versetzt. Nach 5-stündigem Rühren bei Raumtemperatur wurde auf 25 ml Wasser geleert und mit Dichlormethan extrahiert. Chromatographieren über Kieselgel mit Hexan/Dichlormethan 2:1 ergab 0.47 g (59%) Oel. MS: m/e (% Basispeak) = 281 ($M^+$, 100), 238 (18), 225 (12), 212 (34), 192 (23), 166 (19), 140 (20).

Beispiel A

(RS)-3-(4-Cyclohexyl-phenyl)-5-hydroxymethyl-oxazolidin-2-on kann als Wirkstoff nach an sich bekannten Methoden zu pharmazeutischen Präparaten nachfolgender Zusammensetzung formuliert werden:

| 1. Kapseln à 100 mg | |
|---|---|
| Wirkstoff | 100,0 mg |
| Lactose pulv. | 104,7 mg |
| Talk | 12,0 mg |
| Magnesiumstearat | 3,0 mg |
| Maisstärke | 70,0 mg |
| Hydroxypropylmethylcellulose | 10,0 mg |
| Dioctylnatriumsulfosuccinat | 0,3 mg |
| | 300 mg |
| 2. Tabletten à 50 mg | |
| Wirkstoff | 50,0 mg |
| Lactose pulv. | 50,0 mg |
| mikrokristalline Cellulose | 82,0 mg |
| Na-carboxymethylstärke | 15,0 mg |
| Magnesiumstearat | 3,0 mg |
| | 200 mg |
| 3. Suppositorien à 500 mg | |
| Wirkstoff | 500 mg |
| Suppositorienmasse | ad 2000 mg |
| 4. Suppositorien à 500 mg | |
| Wirkstoff | 500 mg |
| Suppositorienmasse | ad 2000 mg |
| 5. Weichgelatinekapseln à 100 mg | |
| Wirkstoff | 100 mg |
| Triglycerid mittelkettig | 300 mg |
| | 400 mg |

**Patentansprüche**

**1.** Verbindungen der allgemeinen Formel

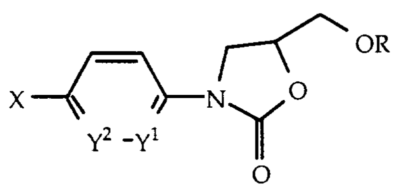

I

worin

R $C_1$-$C_4$-Alkyl;
X $C_3$-$C_7$-Cycloalkenyl; Bicyclo[2.2.1]hept-2-yl, ggf. substituiert durch Phenyl-2-oxo-5-methoxymethyl-oxazolidinyl; Bicyclo[2.2.1]hept-5-en-2-yl; Adamantyl; oder **$C_3$-$C_7$**-Cycloalkyl oder Piperidin, gegebenenfalls einfach oder mehrfach substituiert durch Halogen, Amino, **$C_1$-$C_4$**-Alkyl, Nitril, eine Oxo-Gruppe, Hydroxyimino, Ethylendioxy

oder durch $-OR^1$, worin

$R^1$ $-CH(C_6H_5)_2$, $-(CH_2)_nC_6H_5$, **$C_1$-$C_4$**-Alkyl, Wasserstoff, $-(CH_2)_nNHCOCH_3$, $-(CH_2)_nNH_2$, $-(CH_2)_nSOCH_3$, $-(CH_2)_nCN$, $-(CH_2)_nSCH_3$, $-(CH_2)_nSO_2CH_3$, -CO-**$C_1$-$C_4$**-Alkyl, $-COC_6H_5$, ggf. substituiert durch eine Oxazolidinyl-Gruppe:

oder durch $=CR^2R^3$, worin

$R^2$ Wasserstoff oder **$C_1$-$C_4$**-Alkyl;
$R^3$ Wasserstoff, Nitril, **$C_1$-$C_4$**-Alkyl, Phenyl oder COO-**$C_1$-$C_4$**-Alkyl;

oder durch $-(CH_2)_nR^4$, worin

$R^4$ Nitril, Amino, $-NHCOCH_3$, $-COC_6H_5Hal$, Phenyl oder Hydroxy;

oder durch $-COR^5$, worin

$R^5$ **$C_1$-$C_4$**-Alkyl, $-CH{=}CH-C_6H_5$, $-C_6H_5CF_3$, $-O-C(CH_3)_3$ oder -O-**$C_1$-$C_4$**-Alkyl;

oder durch $-NR^6R^7$, worin

$R^6$ Wasserstoff oder $-COCH_3$;
$R^7$ $-COCH_3$, Benzyl oder $-(CH_2)_nNHCOC_6H_4Hal$;
n 1-3;
$Y^1$ -CH=;
$Y^2$ -CH=, -C(OH)=, $-C(NO_2)=$, $-C(NH_2)=$, **oder** -C(Hal)= bedeuten,

sowie pharmazeutisch annehmbare Salze von basischen Verbindungen der Formel I mit Säuren.

**2.** Verbindungen nach Anspruch 1, worin

R **$C_1$-$C_4$**-Alkyl;
X **$C_3$-$C_7$**-Cycloalkyl oder Piperidin, gegebenenfalls substituiert durch **$C_1$-$C_4$**-Alkyl, Methylen, Ethylendioxy, Halogen, Hydroxy, **$C_1$-$C_4$**-Alkoxy, durch eine Hydroxyimino- oder Oxo-Gruppe, durch $-O-(CH_2)_2-R^8$, $-O-CO-R^9$ oder durch $-NH(CH_2)_nNHCOC_6H_4R^{10}$ oder $-CH_2OH$;
Bicyclo[2.2.1]hept-2-yl, gegebenenfalls substituiert durch Phenyl-2-oxo-5-methoxymethyl-oxazolidinyl;
Bicyclo[2.2.1]hept-5-en-2-yl oder Adamantyl;

$Y^1$ und $Y^2$ beide CH-Gruppen;
$R^8$ -CN oder $-CH_2NH_2$;
$R^9$ **$C_1$-$C_4$**-Alkyl, Phenyl oder durch eine Oxazolidinyl-Gruppe substituiertes Phenyl;
$R^{10}$ Halogen und
n 1-3 bedeuten.

**3.** Verbindungen nach Anspruch 2, worin X Cyclohexyl oder durch eine Oxo-, Hydroxy-, Hydroxyimino-, Methoxy, $-O(CH_2)_2CN$ oder $-O(CH_2)_3NH_2$-Gruppe substituiertes Cyclohexyl, R Methyl und $Y^1$ und $Y^2$ eine CH-Gruppe bedeuten.

**4.** **Die Verbindung** (RS)-3-(4-Cyclohexyl-phenyl)-5-methoxymethyl-oxazolidin-2-on **gemäss Anspruch 1.**

**5.** **Die Verbindung** (R)-3-(4-Cyclohexyl-phenyl)-5-methoxymethyl-oxazolidin-2-on **gemäss Anspruch 1.**

**6.** **Die Verbindung** (R)-3- [4-(4-Oxo-cyclohexyl)-phenyl]-5-methoxymethyl-oxazolidin-2-on **gemäss Anspruch 1.**

7. **Die Verbindung** (RS)-3-[trans-4-[4-(5-Methoxymethyl-2-oxo-oxazolidin-3-yl-phenyl]-cyclohexyloxy]-propionitril **gemäss Anspruch 1.**

8. **Die Verbindung** (RS)-3-[4-(cis- oder trans-4-Hydroxy-4-methylcyclohexyl)-phenyl]-5-methoxymethyl-oxazolidin-2-on **gemäss Anspruch 1.**

9. **Die Verbindung** (RS)-3-[4-(cis- oder trans-4-Hydroxymethyl-cyclohexyl)-phenyl]-5-methoxymethyl-oxazolidin-2-on **gemäss Anspruch 1.**

10. **Die Verbindungen**

    (RS)-3-[4-(4-Oxo-cyclohexyl)-phenyl]-5-methoxymethyl-oxazolidin-2-on;
    (RS)-3-[4-(trans-4-Hydroxy-cyclohexyl)-phenyl]-5-methoxymethyloxazolidin-2-on;
    (RS)-3-[4-(4-Hydroxy-imino-cyclohexyl)-phenyl]-5-methoxymethyl-oxazolidin-2-on;
    (R)-3-[4-(trans-4-Hydroxy-cyclohexyl)-phenyl]-5-methoxymethyl-oxazolidin-2-on;
    (RS)-3-[4-(trans-4-Methoxy-cyclohexyl)-phenyl]-5-methoxymethyl-oxazolidin-2-on;
    Essigsäure (RS)-trans-4-[4-(5-Methoxymethyl-2-oxo-oxazolidin-3-yl)-phenyl]-cyclohexylester **gemäss Anspruch 1.**

11. Verbindungen nach Anspruch 1, worin X Bicyclo[2.2.1]hept-2-yl oder Bicyclo[2.2.1]hept-5-en-2-yl bedeutet.

12. **Die Verbindungen** 3-[(1RS,2RS,4SR)-4-Bicyclo[2.2.1]hept-2-yl-phenyl]-5-methoxymethyl-oxazolidin-2-on und

    3-[(1RS,2SR,4RS)-4-Bicyclo[2.2.1]hept-5-en-2-yl-phenyl]-5-methoxymethyl-oxazolidin-2-on **gemäss Anspruch 1.**

13. Verbindungen nach Anspruch 1, worin X Cyclohexyl oder Piperidin, substituiert durch $-CH_2CN$, $-COCH=CH-C_6H_5$, $-O(CH_2)NH_2$ oder $-OCH_2CN$, R Methyl und $Y^1$ und $Y^2$ eine CH-Gruppe bedeuten.

14. **Die Verbindung** (RS)-[trans-4-[4-(5-Methoxymethyl-2-oxo-oxazolidin-3-yl)-phenyl]-cyclohexyl]-acetonitril **gemäss Anspruch 1.**

15. **Die Verbindung** (R)-[trans-4-[4-(5-Methoxymethyl-2-oxo-oxazolidin-3-yl)-phenyl]-cyclohexyl]-acetonitril **gemäss Anspruch 1.**

16. **Die Verbindung** (RS)-4-[4-(5-Methoxymethy]-2-oxo-oxazolidin-3-yl)-phenyl]-1-(1-oxo-3-phenyl-2-(E)-propenyl]-piperidin **gemäss Anspruch 1.**

17. **Die Verbindung** (RS)-3-[trans-4-[4-(5-Methoxymethyl-2-oxo-oxazolidin-3-yl)-phenyl]-cyclohexyloxy]ethanamin **gemäss Anspruch 1.**

18. **Die Verbindung** (R)-[trans-4-[4-(5-Methoxymethyl-2-oxo-oxazolidin-3-yl)-phenyl]-cyclohexyloxy]-acetonitril **gemäss Anspruch 1.**

19. **Die Verbindung** (RS)-[trans-4-[4-(5-Methoxymethyl-2-oxo-oxazolidin-3-yl)-phenyl[-cyclohexyloxy]-acetonitril **gemäss Anspruch 1.**

20. **Die Verbindung** (R)-3-[4-[trans-4-(3-Amino-propoxy)-cyclohexyl]-phenyl]-5-methoxymethyl-oxazolidin-2-on **gemäss Anspruch 1.**

21. Verbindungen nach einem der Ansprüche 1-20 sowie pharmazeutisch annehmbare Salze davon zur Anwendung als therapeutische Wirkstoffe.

22. Verbindungen nach einem der Ansprüche 1-20 sowie pharmazeutisch annehmbare Salze davon zur **Verwendung bei der** Bekämpfung oder Verhütung von Panik- und Angstzuständen, kognitiven Erkrankungen und neurodegenerativen Erkrankungen, wie der Parkinsonschen Krankheit und der Alzheimerschen Krankheit.

23. Verfahren zur Herstellung von Verbindungen nach einem der Ansprüche 1-20, dadurch gekennzeichnet, dass man

a) eine Verbindung der Formel

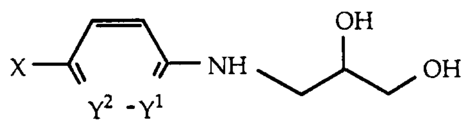

II

worin X, $Y^1$ und $Y^2$ die in Anspruch 1 angegebenen Bedeutungen haben, mit **Diethylcarbonat oder Phosgen** cyclisiert und alkyliert, oder

**b)** eine Verbindung der Formel

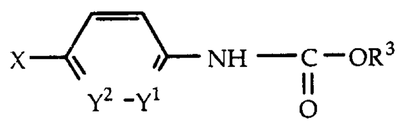

III

worin X, $Y^1$ und $Y^2$ die in Anspruch 1 genannten Bedeutungen haben und $R^3$ niederes Alkyl oder Benzyl bedeutet,
mit einer Verbindung der Formel

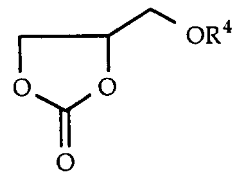

IV

umsetzt,
worin $R^4$ niederes Alkyl oder Benzyl bedeutet,
oder

**c)** eine Verbindung der allgemeinen Formel

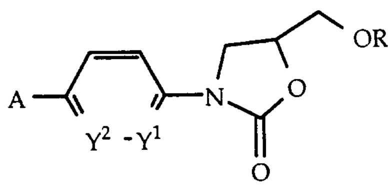

V

worin R, $Y^1$ und $Y^2$ die in Anspruch 1 angegebenen Bedeutungen haben und A eine Abgangsgruppe bedeutet, mit einem reaktiven, den Substituenten X liefernden Mittel behandelt, oder

**d)** eine Verbindung der Formel

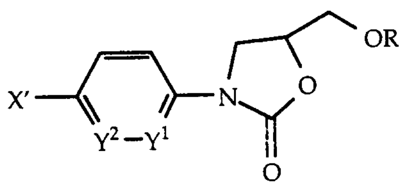

Ia

worin X' ein ($C_5$-$C_7$)-Cycloalkenyl-Rest bedeutet, der wie in Formel I für Cycloalkyl oder Piperidin beschrieben, substituiert sein kann, und $Y^1$, $Y^2$ und R die obigen Bedeutungen haben,
in die entsprechende gesättigte Cycloalkyl-Verbindung katalytisch hydriert, oder

**e)** eine Verbindung der Formel I, worin X ein durch Ethylendioxy substituiertes Cycloalkyl bedeutet, hydrolysiert, oder

**f)** eine Verbindung der Formel I, worin X ein durch -O-$(CH_2)_2$-CN, =CH-CN oder -$CH_2CN$ substituierter Cycloalkyl- oder Piperidin-Rest bedeutet, zur Aminoverbindung hydriert, oder

**g)** eine Verbindung der Formel I, worin X ein durch eine Oxo-Gruppe substituierter Cycloalkyl- oder Piperidin-Rest bedeutet,

- diese in eine Hydroxyimino-Gruppe überführt, oder
- diese zu einer Hydroxy-Gruppe reduziert, oder
- mit entsprechend substituierten Aminen diese in eine - $NH(CH_2)_nNHCOC_6H_4R^3$-Gruppe überführt, worin $R^3$ und n die obigen Bedeutungen haben, oder
- diese in eine Methylen-Gruppe, eine Benzyliden-Gruppe, eine Dimethylmethylen-Gruppe, eine Methylen-Nitril-Gruppe oder in eine Methoxycarbonylmethylen-Gruppe überführt, oder
- diese in eine Ethylendioxy-Gruppe überführt, oder
- diese in eine Aminogruppe überführt, oder
- diese mit einem entsprechenden Phosphonat in eine =CH-CN-Gruppe überführt, oder

**h)** eine Verbindung der Formel I, worin X ein durch eine Hydroxy-Gruppe substituierter Cycloalkyl- oder Piperidin-Rest bedeutet,

- mit Acrylnitril in eine -$O(CH_2)_2CN$-Gruppe überführt, oder
- mit entsprechenden Alkyl-, Aryl- oder Acylhalogeniden alkyliert oder acyliert, oder
- mit geeigneten Halogenierungsmitteln halogeniert, oder
- zu den entsprechenden Sulfanyl-Verbindungen umsetzt und diese gegebenenfalls anschliessend zu den Sulfinyl- oder Sulfonyl-Verbindungen oxydiert, oder

**i)** eine Verbindung der Formel I, worin X ein durch eine Hydroxy-Gruppe substituierter Cycloalkylrest bedeutet,

- diesen zu einem Cycloalkenylrest dehydriert, oder

**j)** eine Verbindung der Formel I, worin X durch eine -$O(CH_2)$n-$SOCH_3$-Gruppe substituierter Cycloalkyl- oder Piperidin-Rest bedeutet,

- diese in eine -$O(CH_2)_nCN$-Gruppe überführt, oder

**k)** eine Verbindung der Formel I, worin X ein Cycloalkyl-Rest, ein Piperidin- oder ein geschützter Piperidin-Rest bedeutet, der nicht über ein Stickstoff verknüpft ist,

- mit entsprechenden Acylierungsmitteln acyliert, oder

**l)** eine Verbindung der Formel I, worin X ein durch eine Methylen-Gruppe substituierter Cycloalkyl- oder Piperidin-Rest bedeutet,

- diese in eine Hydroxymethyl-Gruppe überführt, oder
- diese in eine 4-Hydroxy-4-methyl-Gruppe überführt, und

**m)** erwünschtenfalls eine basische Verbindung der Formel I mittels einer Säure in ein pharmazeutisch anwendbares Salz überführt.

**24.** Arzneimittel, enthaltend eine Verbindung nach einem der Ansprüche 1 bis 20 und ein therapeutisch inertes Excipiens.

**25.** Arzneimittel zur Bekämpfung oder Verhütung von Panik- und Angstzuständen, kognitiven Erkrankungen und neurodegenerativen Erkrankungen, wie der Parkinsonschen Krankheit und der Alzheimerschen Krankheit, enthaltend eine Verbindung nach einem der Ansprüche 1-20 und ein therapeutisch inertes Excipiens.

**26.** Verwendung von Verbindungen nach einem der Ansprüche 1 bis 20 zur Herstellung von Mitteln zur Bekämpfung oder Verhütung von, Panik- und Angstzuständen, kognitiven Erkrankungen und neurodegenerativen Erkrankungen wie der Parkinsonschen Krankheit und der Alzheimerschen Krankheit.

## Claims

**1.** Compounds of the general formula

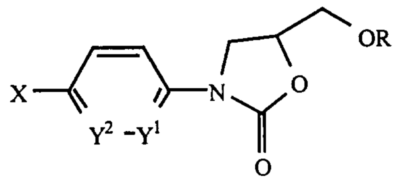

I

wherein

R signifies $C_1$—$C_4$-alkyl;
X signifies $C_3$—$C_7$-cycloalkenyl; bicyclo[2.2.1]hept-2-yl, optionally substituted by phenyl-2-oxo-5-methoxymethyloxazolidinyl; bicyclo[2.2.1]-hept-5-en-2-yl; adamantyl; or $C_3$—$C_7$-cycloalkyl or piperidinyl, optionally mono- or multiply-substituted by halogen, amino, $C_1$—$C_4$-alkyl, nitrilo, an oxo group, hydroxyimino, ethylenedioxy

or by-$OR^1$, in which

$R^1$ signifies $-CH(C_6H_5)_2$, $-(CH_2)_nC_6H_5$, $C_1$—$C_4$-alkyl, hydrogen, $-(CH_2)_nNHCOCH_3$, $-(CH_2)_nNH_2$, $-(CH_2)_nCN$,-$(CH_2)_nSCH_3$, $-(CH_2)_nSO_2CH_3$, -CO-$C_1$—$C_4$-alkyl, $-COC_6H_5$, optionally substituted by an oxazolidinyl group;

or by $=CR^2R^3$, in which

$R^2$ signifies hydrogen or $C_1$—$C_4$-alkyl;
$R^3$ signifies hydrogen, nitrilo, $C_1$—$C_4$-alkyl, phenyl or COO-$C_1$—$C_4$-alkyl;

or by $-(CH_2)_nR^4$, in which

$R^4$ signifies nitrilo, amino, $-NHCOCH_3$, $-COC_6H_5Hal$, phenyl or hydroxy;

or by $-COR^5$, in which

$R^5$ signifies $C_1$—$C_4$-alkyl, $-CH{=}CH-C_6H_5$, $-C_6H_5CF_3$, $-O-C(CH_3)_3$ or $OC_1$—$C_4$-alkyl;

or by $-NR^6R^7$, in which

$R^6$ signifies hydrogen or $-COCH_3$;
$R^7$ signifies $-COCH_3$, benzyl or $(CH_2)_nNHCOC_6H_4Hal$;
n signifies 1-3;
$Y^1$ signifies -CH= and
$Y^2$ signifies -CH=, -C(OH)=, $-C(NO_2)=$, $-C(NH_2)=$ or -C(Hal)=

as well as pharmaceutically usable salts of basic compounds of formula I with acids.

**2.** Compounds according to claim 1, wherein

R signifies $C_1$—$C_4$-alkyl;
X signifies cycloalkyl or piperidinyl, optionally substituted by $C_1$—$C_4$-alkyl, methylene, ethylenedioxy, halogen, hydroxy, $C_1$—$C_4$-alkoxy, by a hydroxyimino or oxo group, by $-O-(CH_2)_2-R^8$, $-O-CO-R^9$ or by $-NH(CH_2)_nNHCOC_6H_4R^{10}$ or $-CH_2OH$;
bicyclo[2.2.1]hept-2-yl, optionally substituted by phenyl-2-oxo-5-methoxymethyl-oxazolidinyl;
bicyclo[2.2.1]hept-5-en-2-yl or adamantyl;
$Y^1$ and $Y^2$ both signify CH groups;

$R^8$ signifies -CN or $-CH_2NH_2$;
$R^9$ signifies $C_1$—$C_4$-alkyl, phenyl or phenyl substituted by an oxazolidinyl group;
$R^{10}$ signifies halogen and
n signifies 1-3.

**3.** Compounds according to claim 2, wherein X signifies cyclohexyl or cyclohexyl substituted by an oxo, hydroxy, hydroyimino, methoxy, $-O(CH_2)_2CN$ or $-O(CH_2)_3NH_2$ group, R signifies methyl and $Y^1$ and $Y^2$ signify a CH group.

**4.** The compound (RS)-3-(4-Cyclohexyl-phenyl)-5-methoxymethyl-oxazolidin-2-one.in accordance with claim. 1

**5.** The compound (R)-3-(4-Cyclohexylphenyl)-5-methoxymethyl-oxazolidin-2-one in accordance with claim 1.

**6.** The compound (R)-3-[4-(4-Oxo-cyclohexyl)-phenyl]-5-methoxymethyl-oxazolidin-2-one in accordance with claim 1.

**7.** The compound (RS)-3-[trans-4-[4-(5-Methoxymethyl-2-oxo-oxazolidin-3-yl-phenyl]-cyclohexyloxy]-propionitrile in accordance with claim 1.

**8.** The compound (RS)-3-[4-(cis- or trans-4-Hydroxy-4-methyl-cyclohexyl)-phenyl]-5-methoxymethyl-oxazolidin-2-one in accordance with claim 1.

**9.** The compound (RS)-3-[4-(cis- or trans-4-Hydroxymethyl-cyclohexyl)-phenyl]-5-methoxymethyl-oxazolidin-2-one in accordance with claim 1.

**10.** The compounds

(RS)-3-[4-(4-oxo-cyclohexyl)-phenyl]-5-methoxymethyl-oxazolidin-2-one;
(RS)-3-[4-(trans-4-hydroxy-cyclohexyl)-phenyl]-5-methoxymethyl-oxazolidin-2-one;
(RS)-3-[4-(4-hydroxy-imino-cyclohexyl)-phenyl]-5-methoxymethyl-oxazolidin-2-one;
(R)-3-[4-(trans-4-hydroxy-cyclohexyl)-phenyl]-5-methoxymethyl-oxazolidin-2-one;
(RS)-3-[4-(trans-4-methoxy-cyclohexyl)-phenyl]-5-methoxymethyl-oxazolidin-2-one;
acetic acid (RS)-trans-4-[4-(5-Methoxymethyl-2-oxo-oxazolidin-3-yl)-phenyl]-cyclohexyl ester in accordance with claim 1.

**11.** Compounds according to claim 1, wherein X signifies bicyclo[2.2.1]hept-2-yl or bicyclo[2.2.1]hept-5-en-2-yl.

**12.** Compounds 3-[(1RS,2RS,4SR)-4-Bicyclo[2.2.1]hept-2-yl-phenyl]-5-methoxymethyl-oxazolidine-2-one and

3-[1RS,2SR,4RS)-4-bicyclo[2.2.1]hept-5-en-2-yl-phenyl]-5-methoxymethyl-oxazolidin-2-one in accordance with claim 1.

**13.** Compounds according to claim 1, wherein X signifies cyclohexyl or piperidinyl substituted by $-CH_2CN$, -COCH=CH-$C_6H_5$, $-O(CH_2)NH_2$ or $-OCH_2CN$, R signifies methyl and $Y^1$ and $Y^2$ signify a CH group.

**14.** Compound (RS)-[trans-4-[4-(5-Methoxymethyl-2-oxo-oxazolidin-3-yl)-phenyl]-cyclohexyl]-acetonitrile in accordance with claim 1.

**15.** Compound (R)-[trans-4-[4-(5-Methoxymethyl-2-oxo-oxazolidin-3-yl)-phenyl]-cyclohexyl]-acetonitrile in accordance with claim 1.

**16.** Compound (RS)-4-[4-(5-Methoxymethyl-2-oxo-oxazolidin-3-yl)-phenyl]-1-(1-oxo-3-phenyl-2-(E)-propenyl]-piperidine in accordance with claim 1.

**17.** Compound (RS)-3-[trans-4-[4-(5-Methoxymethyl-2-oxo-oxazolidin-3-yl)-phenyl]-cyclohexyloxy]ethanamine in accordance with claim 1.

**18.** Compound (R)-[trans-4-[4-(5-Methoxymethyl-2-oxo-oxazolidin-3-yl)-phenyl]-cyclohexyloxy]-acetonitrile in accordance with claim 1.

**19.** Compound (RS)-[trans-4-[4-(5-Methoxymethyl-2-oxo-oxazolidin-3-yl)-phenyl]-cyclohexyloxy]-acetonitrile in accordance with claim 1.

**20.** Compound (R)-3-[4-[trans-4-(3-Amino-propoxy)-cyclohexyl]-phenyl]-5-methoxymethyl-oxazolidin-2-one in accordance with claim 1.

**21.** Compounds according to any one of claims 1-20 as well as pharmaceutically acceptable salts thereof for use as therapeutically active substances.

**22.** Compounds according to any one of claims 1-20 as well as pharmaceutically acceptable salts thereof for use in the control or prevention of panic and anxiety states, cognitive disorders and neurodegenerative diseases such as Parkinson's disease and Alzheimer's disease.

**23.** A process for the manufacture of compounds according to any one of claims 1-20, which process comprises

a) cyclizing a compound of the formula

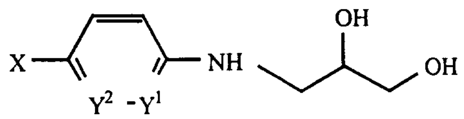

II

wherein X, $Y^1$ and $Y^2$ have the significances given in claim 1,
with diethylcarbonat or phosgen and alkylating, or

b) reacting a compound of the formula

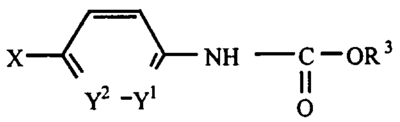

III

wherein X, $Y^1$ and $Y^2$ have the significances given in claim 1 and $R^3$ signifies lower alkyl or benzyl,
with a compound of the formula

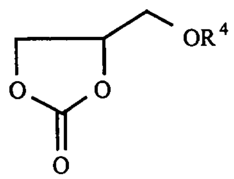

IV

wherein $R^4$ signifies lower alkyl or benzyl,
or

c) treating a compound of the general formula

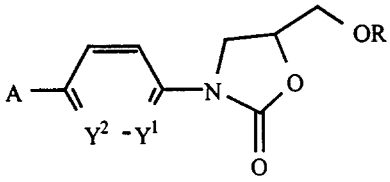

V

wherein R, $Y^1$ and $Y^2$ have the significances given in claim 1 and A signifies a leaving group, with a reactive agent yielding the substituent X, or

d) catalytically hydrogenating a compound of the formula

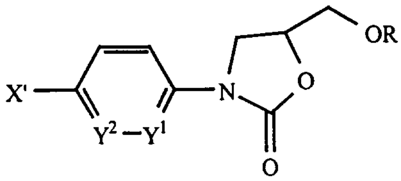

Ia

wherein X' signifies a $(C_5-C_7)$-cycloalkenyl residue, which can be substituted as described in formula I for cycloalkyl or piperidinyl, and $Y^1$, $Y^2$ and R have the significances given in formula I, to the corresponding saturated cycloalkyl compound, or

e) hydrolyzing a compound of formula I in which X signifies cycloalkyl substituted by ethylenedioxy, or

f) hydrogenating a compound of formula I in which X signifies a cycloalkyl or piperidinyl residue substituted by $-O-(CH_2)_2-CN$, =CH-CN or $-CH_2CN$ to the amino compound, or

g) in the case of a compound of formula I in which X signifies a cycloalkyl or piperidinyl residue substituted by an oxo group,

- converting this into a hydroxyimino group, or
- reducing this to a hydroxy group, or
- converting this with appropriately substituted amines into a - $NH(CH_2)_nNHCOC_6H_4R^3$ group, wherein $R^3$ and n have the above significances,
- converting this into a methylene group, a benzylidene group, a dimethylmethylene group, a methylene-nitrilo group or into a methoxycarbonylmethylene group, or
- converting this into an ethylenedioxy group, or
- converting this into an amino group, or
- converting this with a corresponding phosphonate into a =CH-CN group, or

h) in the case of a compound of formula I in which X signifies a cycloalkyl or piperidinyl residue substituted by a hydroxy group,

- converting this with acrylonitrile into a $-O(CH_2)_2CN$ group, or
- alkylating or acylating this with corresponding alkyl, aryl or acyl halides, or
- halogenating this with suitable halogenating agents, or
- reacting this to give the corresponding sulphanyl compounds and, if desired, subsequently oxidizing these to the sulphinyl or sulphonyl compounds, or

i) in the case of a compound of formula I in which X signifies a cycloalkyl residue substituted by a hydroxy group,

- dehydrogenating this to a cycloalkenyl residue, or

j) in the case of a compound of formula I in which X signifies a cycloalkyl or piperidinyl residue substituted by a $-O(CH_2)_n-SOCH_3-$ group,

- converting this into a $-O(CH_2)_nCN-$ group, or

k) in the case of a compound of formula I in which X signifies a cycloalkyl residue, a piperidinyl residue or a protected piperidinyl residue, which is not linked via a nitrogen,

- acylating this with corresponding acylating agents, or

l) in the case of a compound of formula I in which X signifies a cycloalkyl or piperidinyl residue substituted by a methylene group,

- converting this into a hydroxymethyl group, or
- converting this into a 4-hydroxy-4-methyl group, and

m) if desired, converting a basic compound of formula I into a pharmaceutically usable salt by means of an acid.

**24.** A medicament containing a compound according to any one of claims 1 to 20 and a therapeutically inert excipient.

**25.** A medicament for the control or prevention of panic and anxiety states, cognitive disorders and neurodegenerative diseases such as Parkinson's disease and Alzheimer's disease, containing a compound according to any one of claims 1-20 and a therapeutically inert excipient.

**26.** The use of compounds according to any one of claims 1 to 11 for the production of medicaments for the control or prevention of panic and anxiety states, cognitive disorders and neurodegenerative diseases such as Parkinson's disease and Alzheimer's disease.

## Revendications

**1.** Composés de formule générale :

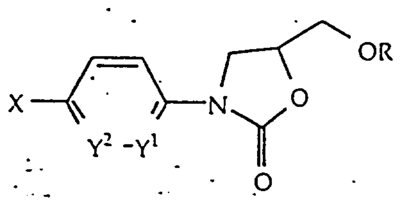

I

dans laquelle :

R est un groupe alkyle en $C_1$-$C_4$ ;

X est un groupe cycloalcényle en $C_3$-$C_7$ ; bicyclo[2.2.1]-hept-2-yle, éventuellement substitué par un groupe phényl-2-oxo-5-méthoxyméthyl-oxazolidinyle ; bicyclo[2.2.1]hept-5-ène-2-yle ; adamantyle ; ou cycloalkyle en $C_3$-$C_7$ ou pipéridine, éventuellement substitué une ou plusieurs fois par des substituants halogéno, amino, alkyle en $C_1$-$C_4$, nitrile, par un groupe oxo, hydroxyimino, éthylènedioxy,

ou par $-OR^1$, où

$R^1$ $-CH(C_6H_5)_2$, $-(CH_2)_nC_6H_5$, un groupe alkyle en $C_1$-$C_4$, un atome d'hydrogène, $-(CH_2)_nNHCOCH_3$, $-(CH_2)_nNH_2$, $-(CH_2)_nSOCH_3$, $-(CH_2)_nCN$, $-(CH_2)_nSCH_3$, $-(CH_2)_nSO_2CH_3$, un groupe -CO(alkyle en $C_1$-$C_4$), $-COC_6H_5$, éventuellement substitué par un groupe oxazolidinyle ;

ou par $=CR^2R^3$, où

$R^2$ est un atome d'hydrogène ou un groupe alkyle en $C_1$-$C_4$ ;

$R^3$ est un atome d'hydrogène, le groupe nitrile, un groupe alkyle en $C_1$-$C_4$, phényle ou COO-(alkyle en $C_1$-$C_4$) ;

ou par $-(CH_2)_nR^4$, où

$R^4$ est le groupe nitrile, amino, $-NHCOCH_3$, $-COC_6H_5Hal$, phényle ou hydroxy ;

ou par $-COR^5$, où

$R^5$ est un groupe alkyle en $C_1$-$C_4$, $-CH=CH-C_6H_5$, $-C_6H_5CF_3$, $-O-C(CH_3)_3$ ou -O-(alkyle en $C_1$-$C_4$) ;

ou par $-NR^6R^7$, où

$R^6$ est un atome d'hydrogène ou $-COCH_3$ ;
$R^7$ est $-COCH_3$, le groupe benzyle ou $-(CH_2)_nNHCOC_6H_4Hal$ ;
n vaut de 1 à 3 ;
$Y^1$ est -CH= ;
$Y^2$ est -CH=, -C(OH)=, $-C(NO_2)=$, $-C(NH_2)=$ ou -C(Hal)=,

ainsi que les sels acceptables d'un point de vue pharmaceutique avec des acides de composés basiques de formule I.

**2.** Composés selon la revendication 1, dans lesquels :

R est un groupe alkyle en $C_1$-$C_4$,
X est un groupe cycloalkyle en $C_3$-$C_7$ ou pipéridine, éventuellement substitué par des substituants alkyle en $C_1$-$C_4$, méthylène, éthylènedioxy, halogéno, hydroxy, alcoxy en $C_1$-$C_4$, par un groupe hydroxyimino ou par un groupe oxo, par $-O-(CH_2)_2-R^8$, $-O-CO-R^9$ ou par $-NH(CH_2)_n-NHCOC_6H_4R^{10}$ ou $-CH_2OH$ ; bicyclo[2.2.1]hept-2-yle éventuellement substitué par un groupe phényl-2-oxo-5-méthoxyméthyl-oxazolidinyle ; bicyclo[2.2.1]hept-5-ène-2-yle ou adamantyle ;
$Y^1$ et $Y^2$ sont tous les deux des groupes CH ;
$R^8$ est -CN ou $-CH_2NH_2$ ;
$R^9$ est un groupe alkyle en $C_1$-$C_4$, phényle, ou phényle substitué par un groupe oxazolidinyle ;
$R^{10}$ est un halogène, et
n vaut de 1 à 3.

**3.** Composés selon la revendication 2, dans lesquels X est le groupe cyclohexyle, ou un groupe cyclohexyle substitué par un groupe oxo, hydroxy, hydroxyimino, méthoxy, $-O(CH_2)_2CN$ ou $-O(CH_2)_3NH_2$, R est le groupe méthyle, et $Y^1$ et $Y^2$ représentent chacun un groupe CH.

**4.** Le composé (RS)-3-(4-cyclohexyl-phényl)-5-méthoxy-méthyloxazolidine-2-one selon la revendication 1.

**5.** Le composé (R)-3-(4-cyclohexyl-phényl)-5-méthoxy-méthyloxazolidine-2-one selon la revendication 1.

**6.** Le composé (R)-3-[4-(4-oxo-cyclohexyl)-phényl]-5-méthoxyméthyloxazolidine-2-one selon la revendication 1.

**7.** Le composé (RS)-3-[trans-4-[4-(5-méthoxyméthyl-2-oxo-oxazolidine-3-yl-phényl]-cyclohexyloxy]-propionitrile selon la revendication 1.

**8.** Le composé (RS)-3-[4-(cis- ou trans-4-hydroxy-4-méthylcyclohexyl)-phényl]-5-méthoxyméthyloxazolidine-2-one selon la revendication 1.

**9.** Le composé (RS)-3-[4-(cis- ou trans-4-hydroxyméthyl-cyclohexyl)-phényl]-5-méthoxyméthyloxazolidine-2-one selon la revendication 1.

**10.** Les composés

(RS)-3-[4-(4-oxo-cyclohexyl)phényl]-5-méthoxyméthyloxazolidine-2-one ;
(RS)-3-[4-(trans-4-hydroxy-cyclohexyl)-phényl]-5-méthoxyméthyl-oxazolidine-2-one ;

(RS)-3-[4-(4-hydroxy-imino-cyclohexyl)-phényl]-5-méthoxyméthyl-oxazolidine-2-one ;
(RS)-3-[4-(trans-4-hydroxy-cyclohexyl)-phényl]-5-méthoxyméthyl-oxazolidine-2-one ;
(RS)-3-[4-(trans-4-méthoxy-cyclohexyl)-phényl]-5-méthoxyméthyl-oxazolidine-2-one ;
ester (RS)-trans-4-[4-5-méthoxyméthyl-2-oxo-oxazolidine-3-yl)-phényl]cyclohexylique de l'acide acétique selon la revendication 1.

**11.** Composés selon la revendication 1, dans laquelle X est le groupe bicyclo[2.2.1]hept-2-yle ou bicyclo[2.2.1]hept-5-ène-2-yle.

**12.** Les composés 3-[(1RS,2RS,4SR)-4-bicyclo[2.2.1]-hept-2-ylphényl]-5-méthoxyméthyl-oxazolidine-2-one et 3-[(1RS,2RS,4RS)-4-bicyclo[2.2.1]hept-5-ène-2-ylphényl]-5-méthoxyméthyl-oxazolidine-2-one selon la revendication 1.

**13.** Composés selon la revendication 1, dans lesquels X est le groupe cyclohexyle ou pipéridine, substitué par $-CH_2CN$, $-COCH=CH-C_6H_5$, $-O(CH_2)NH_2$ ou $-OCH_2CN$, R est le groupe méthyle, et $Y^1$ et $Y^2$ représentent chacun un groupe CH.

**14.** Le composé (RS)-[trans-4-[4-(5-méthoxyméthyl-2-oxo-oxazolidine-3-yl)-phényl]cyclohexyl]-acétonitrile selon la revendication 1.

**15.** Le composé (R)-[trans-4-[4-(5-méthoxyméthyl-2-oxo-oxazolidine-3-yl)-phényl]cyclohexyl]-acétonitrile selon la revendication 1.

**16.** Le composé (RS)-4-[4-(5-méthoxyméthyl-2-oxo-oxazolidine-3-yl)-phényl]-1-(1-oxo-3-phényl-2-(E)-propényl]pipéridine selon la revendication 1.

**17.** Le composé (RS)-3-[trans-4-[4-(5-méthoxyméthyl-2-oxo-oxazolidine-3-yl)-phényl]cyclohexyloxy]-éthanamine selon la revendication 1.

**18.** Le composé (R)-[trans-4-[4-(5-méthoxyméthyl-2-oxo-oxazolidine-3-yl)-phényl]cyclohexyloxy]-acétonitrile selon la revendication 1.

**19.** Le composé (RS)-[trans-4-[4-(5-méthoxyméthyl-2-oxo-oxazolidine-3-yl)-phényl]cyclohexyloxy]-acétonitrile selon la revendication 1.

**20.** Le composé (R)-3-[4-[trans-4-(3-amino-propoxy)-cyclohexyl]-phényl]-5-méthoxyméthyl-oxazolidine-2-one selon la revendication 1.

**21.** Composés selon l'une des revendications 1 à 20, ainsi que leurs sels acceptables d'un point de vue pharmaceutique, pour utilisation en tant que principes actifs thérapeutiques.

**22.** Composés selon l'une des revendications 1 à 20, ainsi que leurs sels acceptables d'un point de vue pharmaceutique, pour utiliser lors de la maîtrise ou de la prévention des états de panique et d'angoisse, des maladies cognitives et des maladies neurodégénératives telles que la maladie de Parkinson et la maladie d'Alzheimer.

**23.** Procédé de préparation de composés selon l'une des revendications 1 à 20, caractérisé en ce que :

a) on cyclise et on alkyle avec du carbonate de diéthyle ou du phosgène un composé de formule :

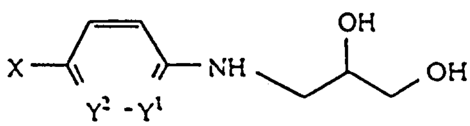

. II

dans laquelle X, $Y^1$ et $Y^2$ ont les significations données dans la revendication 1, ou bien
b) on fait réagir un composé de formule :

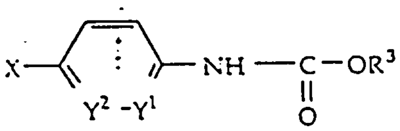

III

dans laquelle, X, $Y^1$ et $Y^2$ ont les significations données dans la revendication 1, et $R^3$ est un groupe alkyle inférieur ou benzyle,
avec un composé de formule :

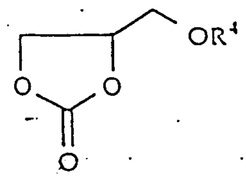

IV

dans laquelle $R^4$ est un groupe alkyle inférieur ou benzyle,
ou bien

c) On traite un composé de formule générale :

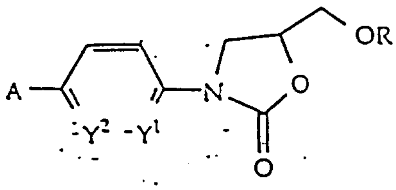

V

dans laquelle R, $Y^1$ et $Y^2$ ont les significations données la revendication 1, et A est un groupe éliminable, avec un composé réactif fournissant des substituants X, ou bien

d) on soumet à une hydrogénation catalytique, pour obtenir le composé cycloalkylé saturé correspondant, un composé de formule :

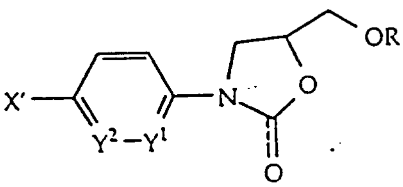

Ia

dans laquelle $X^1$ est un groupe cycloalcényle en $C_5$-$C_7$, qui peut être substitué comme décrit dans la formule I pour les groupes cycloalkyle ou pipéridine, et $Y^1$, $Y^2$ et R ont les significations ci-dessus, ou bien

e) on hydrolyse un composé de formule I dans laquelle X est un groupe cycloalkyle substitué par un groupe éthylènedioxy, ou bien

f) on hydrogène un composé de formule I dans laquelle X est un groupe cycloalkyle ou pipéridine substitué par $-O-(CH_2)_2-CN$, $=CH-CN$ ou $-CH_2CN$, pour obtenir un composé amino, ou bien

g) on fait subir à un composé de formule I dans laquelle X est un radical cycloalkyle ou pipéridine substitué par un groupe oxo les opérations suivantes :

- on le convertit en un groupe hydroxyimino, ou bien
- on le réduit en un groupe hydroxy, ou bien
- on le convertit avec des amines convenablement substituées en un groupe $NH(CH_2)_nNHCOC_6H_4R^3$ dans

lequel $R^3$ et n ont les significations données ci-dessus, ou bien

- on le convertit en un groupe méthylène, un groupe benzylidène, un groupe diméthylméthylène, un groupe méthylènenitrile ou en un groupe méthoxycarbonylméthylène, ou bien
- on le convertit en un groupe éthylènedioxy, ou bien
- on le convertit en un groupe amino, ou bien
- on le convertit avec un phosphonate approprié en un groupe =CH-CN, ou bien

h) on soumet un composé de formule I dans laquelle X est un groupe cycloalkyle ou pipéridine substitué par un groupe hydroxy aux opérations suivantes :

- on le convertit avec de l'acrylonitrile en un groupe $-O(CH_2)_2CN$, ou bien
- on l'alkyle ou on l'acyle avec les halogénures correspondants d'alkyle, d'aryle ou d'acyle, ou bien
- on l'halogène avec des agents d'halogénation appropriés, ou bien
- on le fait réagir pour donner les composés sulfanylés correspondants, puis, éventuellement, on oxyde ces derniers en les composés sulfinylés ou sulfonylés,

i) on déshydrogène pour obtenir un groupe cycloalcényle un composé de formule I dans laquelle X est un groupe cycloalkyle substitué par un groupe hydroxy, ou bien
j) on convertit en un groupe $-O(CH_2)_nCN$ un composé de formule I dans laquelle X est un groupe cycloalkyle ou pipéridine substitué par un groupe $-O(CH_2)n-SOCH_3-$, ou bien
k) on acyle avec des agents d'acylation correspondants un composé de formule I dans laquelle X est un groupe cycloalkyle, un groupe pipéridine ou pipéridine protégé, qui n'est pas relié par l'intermédiaire d'un atome d'azote, ou bien
l) on convertit en un groupe hydroxyméthyle, ou bien on convertit en un groupe 4-hydroxy-4-méthyle,
un composé de formule I dans laquelle X est un groupe cycloalkyle ou pipéridine substitué par un groupe méthylène, et
m) Si on le souhaite, on convertit un composé basique de formule I, à l'aide d'un acide, en un sel utilisable d'un point de vue pharmaceutique.

**24.** Médicament contenant un composé selon l'une des revendications 1 à 20 et un excipient thérapeutiquement inerte.

**25.** Médicament pour maîtriser ou prévenir des états de panique et d'angoisse, des maladies cognitives et des maladies neurodégénératives, telles que la maladie de Parkinson et la maladie d'Alzheimer, contenant un composé selon l'une des revendications 1 à 20 et un excipient thérapeutiquement inerte.

**26.** Utilisation de composés selon l'une des revendications 1 à 20 pour préparer des produits destinés à maîtriser ou prévenir des états de panique et d'angoisse, des maladies cognitives et des maladies neurodégénératives telles que la maladie de Parkinson et la maladie d'Alzheimer.